(19)

**Europäisches Patentamt**
**European Patent Office**
**Office européen des brevets**

(11) **EP 4 192 872 B1**

(12) **EUROPEAN PATENT SPECIFICATION**

(45) Date of publication and mention
of the grant of the patent:
**16.04.2025  Bulletin 2025/16**

(21) Application number: **21762272.9**

(22) Date of filing: **05.08.2021**

(51) International Patent Classification (IPC):
**C07K 16/28** (2006.01)    **A61P 35/00** (2006.01)
**C07K 14/705** (2006.01)

(52) Cooperative Patent Classification (CPC):
**C07K 16/2863; A61P 35/00; C07K 16/283;**
C07K 2317/35; C07K 2317/622; C07K 2317/70;
C07K 2317/73

(86) International application number:
**PCT/US2021/044737**

(87) International publication number:
**WO 2022/031965 (10.02.2022 Gazette 2022/06)**

(54) **PROTEINS BINDING NKG2D, CD16 AND EGFR**

PROTEINE, DIE NKG2D, CD16 UND EGFR BINDEN

PROTÉINES SE LIANT À NKG2D, CD16 ET EGFR

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.08.2020   US 202063061510 P**

(43) Date of publication of application:
**14.06.2023   Bulletin 2023/24**

(60) Divisional application:
**25162701.4**

(73) Proprietor: **Dragonfly Therapeutics, Inc.
Waltham, MA 02451 (US)**

(72) Inventors:
- **CHEUNG, Ann, F.**
**Lincoln, MA 01773 (US)**
- **DRABIC, Stacey, V.**
**Cambridge, MA 02140 (US)**
- **GRINBERG, Asya**
**Lexington, MA 02421 (US)**
- **JUO, Zong, Sean**
**Taipei, 114 (TW)**

- **LIHARSKA, Katia**
**Billerica, MA 01821 (US)**
- **MORGAN, Christopher, Ryan**
**Southborough, MA 01772 (US)**
- **WAGTMANN, Nicolai**
**Concord, MA 01742 (US)**

(74) Representative: **Haseltine Lake Kempner LLP
Cheapside House
138 Cheapside
London EC2V 6BJ (GB)**

(56) References cited:
**WO-A1-2015/184203      WO-A1-2019/035939**

- **FELICES MARTIN ET AL: "Generation of BiKEs
and TriKEs to Improve NK Cell-Mediated
Targeting of Tumor Cells", 14 May 2016,
NATURAL KILLER CELLS : METHODS AND
PROTOCOLS; IN: METHODS IN MOLECULAR
BIOLOGY; ISSN 1064-3745; [METHODS IN
MOLECULAR BIOLOGY; ISSN 1064-3745],
SPRINGER NEW YORK, NEW YORK, NY,
PAGE(S) 333 - 346, ISBN: 978-1-4939-3682-3,
XP009526052**

**(Cont. next page)**

Remarks:
The complete document including Reference Table(s) and the Sequence Listing(s) can be downloaded from the EPO website

Remarks:
The complete document including Reference Table(s) and the Sequence Listing(s) can be downloaded from the EPO website

**Description**

The present application relates to multispecific binding proteins that bind to NKG2D, CD16, and epidermal growth factor receptor (EGFR).

BACKGROUND

Despite substantial research efforts, cancer continues to be a significant clinical and financial burden in countries across the globe. According to the World Health Organization (WHO), it is the second leading cause of death. Surgery, radiation therapy, chemotherapy, biological therapy, immunotherapy, hormone therapy, stem-cell transplantation, and precision medicine are among the existing treatment modalities. Despite extensive research in these areas, a highly effective, curative solution, particularly for the most aggressive cancers, has yet to be identified. Furthermore, many of the existing anticancer treatment modalities have substantial adverse side effects.

Cancer immunotherapies are desirable because they are highly specific and can facilitate destruction of cancer cells using the patient's own immune system. Fusion proteins such as bi-specific T-cell engagers are cancer immunotherapies described in the literature that bind to tumor cells and T-cells to facilitate destruction of tumor cells. Antibodies that bind to certain tumor-associated antigens have been described in the literature. *See, e.g.,* WO 2016/134371 and WO 2015/095412.

Natural killer (NK) cells are a component of the innate immune system and make up approximately 15% of circulating lymphocytes. NK cells infiltrate virtually all tissues and were originally characterized by their ability to kill tumor cells effectively without the need for prior sensitization. Activated NK cells kill target cells by means similar to cytotoxic T cells - *i.e.,* via cytolytic granules that contain perforin and granzymes as well as via death receptor pathways. Activated NK cells also secrete inflammatory cytokines such as IFN-γ and chemokines that promote the recruitment of other leukocytes to the target tissue.

NK cells respond to signals through a variety of activating and inhibitory receptors on their surface. For example, when NK cells encounter healthy self-cells, their activity is inhibited through activation of the killer-cell immunoglobulin-like receptors (KIRs). Alternatively, when NK cells encounter foreign cells or cancer cells, they are activated via their activating receptors (e.g., NKG2D, NCRs, DNAM1). NK cells are also activated by the constant region of some immunoglobulins through CD16 receptors on their surface. The overall sensitivity of NK cells to activation depends on the sum of stimulatory and inhibitory signals. NKG2D is a type-II transmembrane protein that is expressed by essentially all natural killer cells where NKG2D serves as an activating receptor. NKG2D is also found on T cells where it acts as a costimulatory receptor. The ability to modulate NK cell function via NKG2D is useful in various therapeutic contexts including malignancy.

Mutations that lead to epidermal growth factor receptor (EGFR) overexpression or overactivity have been associated with a number of cancers, including non-small cell lung cancer, anal cancers, glioblastoma and epithelial tumors of the head and neck. These somatic mutations involving EGFR lead to its constant activation, which produces uncontrolled cell division. In glioblastoma a more or less specific mutation of EGFR, called EGFRvIII is often observed. Mutations, amplifications or misregulations of EGFR or family members are implicated in other solid tumors, including colorectal cancer, renal cell carcinoma, bladder cancer, cervical cancer, ovarian cancer, pancreatic cancer, and liver cancer.

Anti-EGFR monoclonal antibodies, such as cetuximab, panitumumab, necitumumab, and zalutumumab, have been developed. However, there remains a need for new and useful antibodies and related therapies for use in treatment of cancer that have greater efficacy and reduced adverse effects.

WO 2019/035939 describes multi-specific binding proteins that bind NKG2D receptor, CD16, and a tumour-associated antigen selected from EGFR, HLA-E, CCR4 and PD-L1, as well as pharmaceutical compositions and therapeutic methods useful for the treatment of cancer.

WO 2015/184203 describes tri-specific binding molecules, which are multichain polypeptide molecules that possess three binding domains and are thus capable of mediating coordinated binding to three epitopes. Such epitopes may be epitopes of the same antigen or epitopes of two or three different antigens. For example, one of such epitopes is capable of binding to CD3, the second of such epitopes is capable of binding to CD8, and the third of such epitopes is capable of binding to an epitope of a disease-associated antigen. The document also provides a ROR1-binding antibody, as well as derivatives thereof and uses for such compositions.

SUMMARY

The present invention provides a protein comprising:

(a) an EGFR-binding scFv (VL-VH) sequence in the orientation of heavy chain variable domain (VH) positioned C-terminal to light chain variable domain (VL), linked to an Fc domain polypeptide to form a (VL-VH)-Fc polypeptide, comprising the amino acid sequence of SEQ ID NO: 167;
and
(b) an NKG2D-binding Fab fragment including:

a heavy chain portion comprising a heavy chain variable domain (VH) and a CH1 domain, wherein the CH1 domain is connected to an Fc domain polypeptide to form a VH-CH1-Fc polypeptide comprising the amino acid sequence of SEQ ID NO: 164, and
a light chain portion comprising a light chain variable domain (VL) and a light chain constant (CL) domain to form a VL-CL polypeptide comprising the amino acid sequence of SEQ ID NO: 165.

The invention is defined by the claims and any other aspects, configurations or embodiments set forth herein not falling within the scope of the claims are for information only.

Any references in the description to methods of treatment refer to the compounds, pharmaceutical compositions and medicaments of the present invention for use in a method for treatment of the human (or animal) body by therapy (or for diagnosis).

The present application provides multispecific binding proteins that bind to the NKG2D receptor, CD16 receptor on natural killer cells, and EGFR. Such proteins can engage more than one kind of NK-activating receptor, and may block the binding of natural ligands to NKG2D. In certain embodiments, the proteins can agonize NK cells in humans. In some embodiments, the proteins can agonize NK cells in humans and in other species such as rodents and cynomolgus monkeys. Formulations containing any one of the proteins disclosed herein; cells containing one or more nucleic acids expressing the proteins, and methods of enhancing tumor cell death using the proteins are also provided.

Accordingly, one aspect of the present application provides a protein comprising (a) a first antigen-binding site that binds NKG2D, (b) a second antigen-binding site that binds EGFR, and (c) an antibody Fc domain or a portion thereof sufficient to bind CD16, or a third antigen-binding site that binds CD16, wherein the second antigen-binding site comprises: (i) a heavy chain variable domain (VH) comprising complementarity-determining region 1 (CDR1), complementarity-determining region 2 (CDR2), and complementarity-determining region 3 (CDR3) sequences of SEQ ID NOs: 136, 157, and 138, respectively, and a light chain variable domain (VL) comprising CDR1, CDR2, and CDR3 sequences of SEQ ID NOs: 140, 141, and 151, respectively; or (ii) a VH comprising CDR1, CDR2, and CDR3 sequences of SEQ ID NOs: 136, 146, and 138, respectively, and a VL comprising CDR1, CDR2, and CDR3 sequences of SEQ ID NOs: 140, 141, and 142, respectively.

In some embodiments, the VH comprises CDR1, CDR2, and CDR3 sequences of SEQ ID NOs: 136, 157, and 138, respectively; and the VL comprises CDR1, CDR2, and CDR3 sequences of SEQ ID NOs: 140, 141, and 151, respectively.

In some embodiments, the VH comprises CDR1, CDR2, and CDR3 sequences of SEQ ID NOs: 136, 146, and 138, respectively; and the VL comprises CDR1, CDR2, and CDR3 sequences of SEQ ID NOs: 140, 141, and 151, respectively.

In some embodiments, the VH comprises CDR1, CDR2, and CDR3 sequences of SEQ ID NOs: 136, 137, and 138, respectively; and the VL comprises CDR1, CDR2, and CDR3 sequences of SEQ ID NOs: 140, 141, and 151, respectively.

In some embodiments, the VH comprises CDR1, CDR2, and CDR3 sequences of SEQ ID NOs: 136, 146, and 138, respectively; and the VL comprises CDR1, CDR2, and CDR3 sequences of SEQ ID NOs: 140, 141, and 142, respectively.

In some embodiments, the VH comprises an amino acid sequence at least 90% identical to SEQ ID NO: 145 and the VL comprises an amino acid sequence at least 90% identical to SEQ ID NO: 150. In some embodiments, the VH comprises the amino acid sequence of SEQ ID NO: 145 and the VL comprises the amino acid sequence of SEQ ID NO:150. In some embodiments, the VH comprises the amino acid sequence of SEQ ID NO: 170 and the VL comprises the amino acid sequence of SEQ ID NO:171.

In some embodiments, the VH comprises an amino acid sequence at least 90% identical to SEQ ID NO: 135 and the VL comprises an amino acid sequence at least 90% identical to SEQ ID NO: 150. In some embodiments, the VH comprises the amino acid sequence of SEQ ID NO: 135 and the VL comprises the amino acid sequence of SEQ ID NO:150.

In some embodiments, the VH comprises an amino acid sequence at least 90% identical to SEQ ID NO: 145 and the VL comprises an amino acid sequence at least 90% identical to SEQ ID NO: 147. In some embodiments, the VH comprises the amino acid sequence of SEQ ID NO: 145 and the VL comprises the amino acid sequence of SEQ ID NO:147.

Another aspect of the present application protein comprising (a) a first antigen-binding site that binds NKG2D, (b) a second antigen-binding site that binds EGFR, and (c) an antibody Fc domain or a portion thereof sufficient to bind CD16, or a third antigen-binding site that binds CD16, wherein the second antigen-binding site comprises a VH comprising an amino acid sequence at least 90% identical to SEQ ID NO: 135 and a VL comprising an amino acid sequence at least 90% identical to SEQ ID NO: 139, wherein the VH comprises an S62R substitution relative to SEQ ID NO: 135 and/or the VL comprises a D92R substitution and/or an F87Y substitution relative to SEQ ID NO:139, numbered under the Chothia numbering scheme.

In some embodiments, the VH comprises an S62R substitution relative to SEQ ID NO:135, numbered under the Chothia numbering scheme. In some embodiments, the VL comprises a D92R substitution relative to SEQ ID NO: 139, numbered under the Chothia numbering scheme. In some embodiments, the VH comprises an S62R substitution relative to SEQ ID NO: 135 and the VL comprises a D92R substitution relative to SEQ ID NO: 139, numbered under the Chothia numbering scheme.

In some embodiments of any one of the aspects above, the VL comprises an F87Y substitution relative to SEQ ID NO: 139, numbered under the Chothia numbering scheme.

In some embodiments, the second antigen-binding site comprises a single-chain fragment variable (scFv), and wherein the scFv comprises an amino acid sequence at least 90% identical to, or comprising, a sequence selected from the group consisting of SEQ ID NOs: 152, 154, 148, and 158.

In some embodiments, the protein of the present disclosure comprises a polypeptide comprising an amino acid sequence at least 90% identical to, or comprising, a sequence selected from the group consisting of SEQ ID NO: 167, 168, and 166.

In some embodiments, the second antigen-binding site binds human EGFR with a dissociation constant ($K_D$) smaller than or equal to 5 nM, as measured by surface plasmon resonance (SPR). In some embodiments, the second antigen-binding site binds rhesus macaque EGFR with a dissociation constant ($K_D$) smaller than or equal to 6 nM, as measured by surface plasmon resonance (SPR).

In some embodiments, the first antigen-binding site that binds NKG2D is a Fab fragment, and the second antigen-binding site that binds EGFR is an scFv. In some embodiments, the first antigen-binding site that binds NKG2D is an scFv, and the second antigen-binding site that binds EGFR is a Fab fragment. In some embodiments, the protein further comprises an additional antigen-binding site that binds EGFR. In some embodiments, the first antigen-binding site that binds NKG2D is an scFv, and the second and the additional antigen-binding sites that bind EGFR are each a Fab fragment. In some embodiments, the first antigen-binding site that binds NKG2D is an scFv, and the second and the additional antigen-binding sites that bind EGFR are each an scFv. In some embodiments, the amino acid sequences of the second and the additional antigen-binding sites are identical.

In some embodiments, the scFv that binds NKG2D is linked to an antibody constant domain or a portion thereof sufficient to bind CD16, via a hinge comprising Ala-Ser or Gly-Ser, wherein the scFv comprises a heavy chain variable domain and a light chain variable domain. In some embodiments, each scFv that binds EGFR is linked to an antibody constant domain or a portion thereof sufficient to bind CD16, via a hinge comprising Ala-Ser or Gly-Ser, wherein the scFv comprises a heavy chain variable domain and a light chain variable domain. In some embodiments, the hinge further comprises an amino acid sequence Thr-Lys-Gly.

In some embodiments, within the scFv that binds NKG2D, the heavy chain variable domain of the scFv forms a disulfide bridge with the light chain variable domain of the scFv. In some embodiments, within each scFv that binds EGFR, the heavy chain variable domain of the scFv forms a disulfide bridge with the light chain variable domain of the scFv. In some embodiments, the disulfide bridge is formed between C44 of the heavy chain variable domain and C100 of the light chain variable domain, numbered under the Kabat numbering scheme. In some embodiments, within the scFv that binds NKG2D, the heavy chain variable domain is linked to the light chain variable domain via a flexible linker. In some embodiments, within each scFv that binds EGFR, the heavy chain variable domain is linked to the light chain variable domain via a flexible linker. In some embodiments, the flexible linker comprises $(G_4S)_4$ (SEQ ID NO: 119).

In some embodiments, within the scFv that binds NKG2D, the heavy chain variable domain is positioned at the C-terminus of the light chain variable domain. In some embodiments, within each scFv that binds EGFR, the heavy chain variable domain is positioned at the C-terminus of the light chain variable domain. In some embodiments, within the scFv that binds NKG2D, the heavy chain variable domain is positioned at the N-terminus of the light chain variable domain.

In some embodiments, within each scFv that binds EGFR, the heavy chain variable domain is positioned at the N-terminus of the light chain variable domain. In some embodiments, the Fab fragment that binds NKG2D is not positioned between an antigen-binding site and the Fc or the portion thereof. In some embodiments, no Fab fragment that binds EGFR is positioned between an antigen-binding site and the Fc or the portion thereof.

In some embodiments, the first antigen-binding site that binds NKG2D comprises a VH comprising CDR1, CDR2, and CDR3 comprising the amino acid sequences of SEQ ID NOs: 81, 82, and 112, respectively; and a VL comprising CDR1, CDR2, and CDR3 comprising the amino acid sequences of SEQ ID NOs: 86, 77, and 87, respectively. In some embodiments, the first antigen-binding site that binds NKG2D comprises a VH comprising CDR1, CDR2, and CDR3 comprising the amino acid sequences of SEQ ID NOs: 81, 82, and 97, respectively; and a VL comprising CDR1, CDR2, and CDR3 comprising the amino acid sequences of SEQ ID NOs: 86, 77, and 87, respectively. In some embodiments, the VH of the first antigen-binding site comprises an amino acid sequence at least 90% identical to SEQ ID NO:95, and the VL of the first antigen-binding site comprises an amino acid sequence at least 90% identical to SEQ ID NO:85. In some embodiments, the VH of the first antigen-binding site comprises the amino acid sequence of SEQ ID NO:95, and the VL of the first antigen-binding site comprises the amino acid sequence of SEQ ID NO:85.

In some embodiments, the antibody Fc domain comprises a hinge and a CH2 domain. In some embodiments, the antibody Fc domain is a human IgG1 antibody Fc domain. In some embodiments, the antibody Fc domain or the portion thereof comprises an amino acid sequence at least 90% identical to amino acids 234-332 of a human IgG1 antibody or SEQ ID NO: 118.

In some embodiments, at least one polypeptide chain of the antibody Fc domain comprises one or more mutations, relative to SEQ ID NO:118, at one or more positions selected from Q347, Y349, L351, S354, E356, E357, K360, Q362, S364, T366, L368, K370, N390, K392, T394, D399, S400, D401, F405, Y407, K409, T411, and K439, numbered according to the EU numbering system. In some embodiments, at least one polypeptide chain of the antibody Fc domain comprises one or more mutations, relative to SEQ ID NO:118, selected from Q347E, Q347R, Y349S, Y349K, Y349T, Y349D, Y349E, Y349C, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, D399R, D399K, D399V, S400K, S400R, D401K, F405A, F405T, Y407A, Y407I, Y407V, K409F, K409W, K409D, T411D, T411E, K439D, and K439E, numbered according to the EU numbering system.

In some embodiments, one polypeptide chain of the antibody heavy chain constant region comprises one or more mutations, relative to SEQ ID NO:118, at one or more positions selected from Q347, Y349, L351, S354, E356, E357, K360, Q362, S364, T366, L368, K370, K392, T394, D399, S400, D401, F405, Y407, K409, T411 and K439; and the other polypeptide chain of the antibody heavy chain constant region comprises one or more mutations, relative to SEQ ID NO:118, at one or more positions selected from Q347, Y349, L351, S354, E356, E357, S364, T366, L368, K370, N390, K392, T394, D399, D401, F405, Y407, K409, T411, and K439, numbered according to the EU numbering system. In some embodiments, one polypeptide chain of the antibody heavy chain constant region comprises K360E and K409W substitutions relative to SEQ ID NO:118; and the other polypeptide chain of the antibody heavy chain constant region comprises Q347R, D399V and F405T substitutions relative to SEQ ID NO:118, numbered according to the EU numbering system.

In some embodiments, one polypeptide chain of the antibody heavy chain constant region comprises a Y349C substitution relative to SEQ ID NO:118; and the other polypeptide chain of the antibody heavy chain constant region comprises an S354C substitution relative to SEQ ID NO:118, numbered according to the EU numbering system.

Another aspect of the present application provides a protein comprising (a) a first polypeptide comprising the amino acid sequence of SEQ ID NO:167, (b) a second polypeptide comprising the amino acid sequence of SEQ ID NO:164, and (c) a third polypeptide comprising the amino acid sequence of SEQ ID NO:165.

Another aspect of the present application provides a protein comprising (a) a first polypeptide comprising the amino acid sequence of SEQ ID NO:168, (b) a second polypeptide comprising the amino acid sequence of SEQ ID NO:164, and (c) a third polypeptide comprising the amino acid sequence of SEQ ID NO:165.

Another aspect of the present application provides a protein comprising (a) a first polypeptide comprising the amino acid sequence of SEQ ID NO:166, (b) a second polypeptide comprising the amino acid sequence of SEQ ID NO:164, and (c) a third polypeptide comprising the amino acid sequence of SEQ ID NO:165.

Another aspect of the present application provides a formulation comprising a protein as disclosed herein and a pharmaceutically acceptable carrier.

Another aspect of the present application provides a cell comprising one or more nucleic acids encoding a protein as disclosed herein. In some embodiments, the cell comprising one or more nucleic acids encoding a protein comprising an amino acid sequence of SEQ ID NO: 167 or SEQ ID NO: 169.

Another aspect of the present application provides a purified protein as disclosed herein. In some embodiments, the protein is purified using a method selected from the group consisting of: centrifugation, depth filtration, cell lysis, homogenization, freeze-thawing, affinity purification, gel filtration, ion exchange chromatography, hydrophobic interaction exchange chromatography, and mixed-mode chromatography.

Another aspect of the present application provides a method of enhancing tumor cell death, comprising exposing the tumor cell and a natural killer cell to an effective amount of a protein as disclosed herein or a formulation as disclosed herein, wherein the tumor cell expresses EGFR.

Another aspect of the present application provides a protein as disclosed herein or a formulation as disclosed herein for use in a method of treating cancer, wherein the method comprises administering to a patient in need thereof an effective amount of the protein as disclosed herein or the formulation as disclosed herein. In some embodiments, the cancer is a solid tumor. In some embodiments, the cancer is lung cancer, breast cancer, kidney cancer, colorectal cancer, gastric cancer, brain cancer, glioma, bladder cancer, head and neck cancer, bladder cancer, pancreatic cancer, and liver cancer, cervical cancer, ovarian cancer or prostate cancer. In some embodiments, the cancer expresses EGFR.

These and other aspects and advantages of the TriNKETs described in the present application are illustrated by the following figures, detailed description and claims.

## BRIEF DESCRIPTION OF THE DRAWINGS

FIG. 1 is a representation of a heterodimeric, multispecific binding antibody, e.g., a trispecific binding protein (TriNKET). Each arm can represent either the NKG2D-binding domain, or the binding domain corresponding to a tumor-associated antigen. In some embodiments, the NKG2D binding domain and the tumor-associated antigen binding domains can share a common light chain.

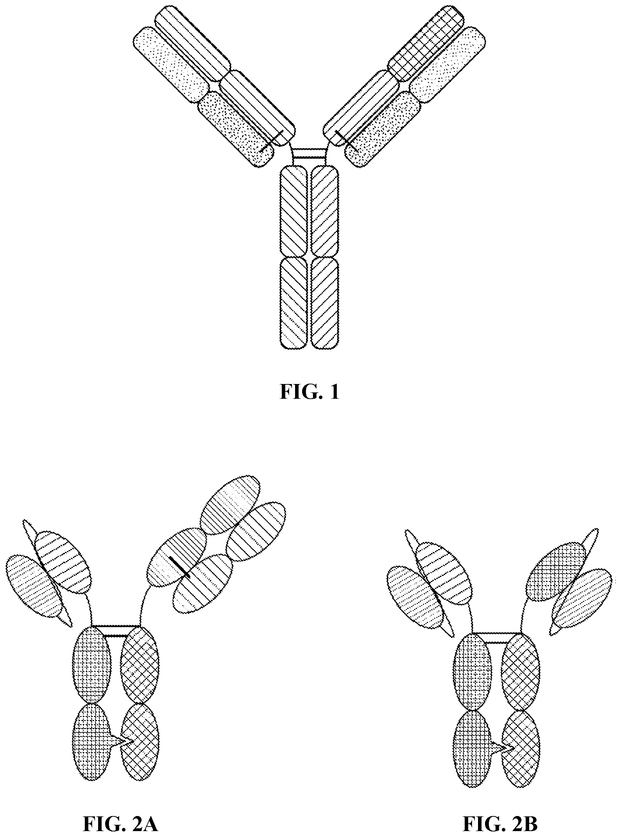

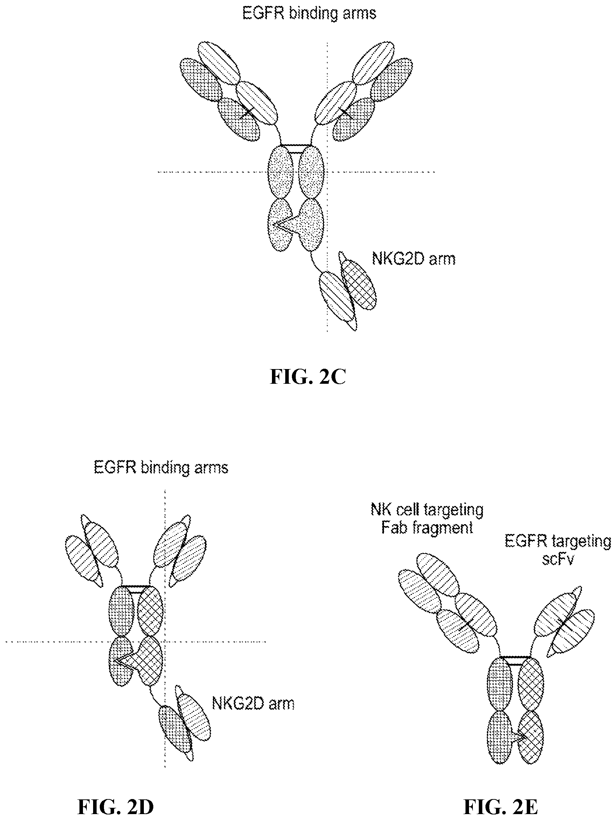

FIGs. 2A-2E illustrate five exemplary formats of a multispecific binding protein, *e.g.,* a trispecific binding protein (TriNKET). As shown in FIG. 2A, either the NKG2D-binding domain or the tumor-associated antigen binding domain can take the scFv format (left arm). An antibody that contains a NKG2D-targeting scFv, a tumor-associated antigen targeting Fab fragment, and a heterodimerized antibody constant region is referred herein as the F3-TriNKET. An antibody that contains a tumor-associated antigen targeting scFv, a NKG2D-targeting Fab fragment, and a heterodimerized antibody constant region/domain that binds CD16 is referred herein as the F3'-TriNKET **(FIG. 2E).** As shown in **FIG. 2B,** both the NKG2D-binding domain and tumor-associated antigen binding domain can take the scFv format. **FIGs. 2C to 2D** are illustrations of an antibody with three antigen-binding sites, including two antigen-binding sites that bind the tumor-associated antigen, and the NKG2D-binding site fused to the heterodimerized antibody constant region. These antibody formats are referred herein as F4-TriNKET. **FIG. 2C** illustrates that the two tumor-associated antigen-binding sites are in the Fab fragment format, and the NKG2D binding site in the scFv format. **FIG. 2D** illustrates that the tumor-associated antigen-binding sites are in the scFv format, and the NKG2D binding site is in the scFv format. **FIG. 2E** represents a trispecific antibody (TriNKET) that contains a tumor-targeting scFv, a NKG2D-targeting Fab fragment, and a heterodimerized antibody constant region/domain ("CD domain") that binds CD16. The antibody format is referred herein as F3'-TriNKET. In certain exemplary multispecific binding proteins, heterodimerization mutations on the antibody constant region include K360E and K409W on one constant domain; and Q347R, D399V and F405T on the opposite constant domain (shown as a triangular lock-and-key shape in the CD domains). The bold bar between the heavy and the light chain variable domains of the Fab fragments represents a disulfide bond.

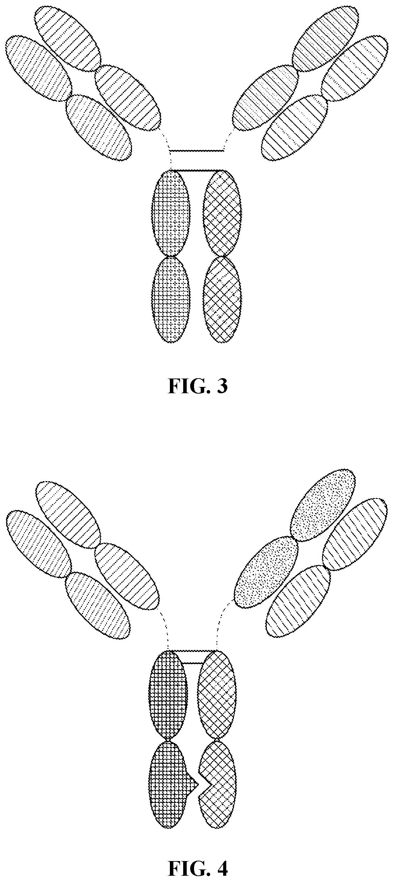

FIG. 3 is a representation of a TriNKET in the Triomab form, which is a trifunctional, bispecific antibody that maintains an IgG-like shape. This chimera consists of two half antibodies, each with one light and one heavy chain, that originate from two parental antibodies. Triomab form may be a heterodimeric construct containing 1/2 of rat antibody and 1/2 of mouse antibody.

FIG. 4 is a representation of a TriNKET in the KiH Common Light Chain form, which involves the knobs-into-holes (KIHs) technology. KiH is a heterodimer containing 2 Fab fragments binding to target 1 and 2, and an Fc stabilized by heterodimerization mutations. TriNKET in the KiH format may be a heterodimeric construct with 2 Fab fragments binding to target 1 and target 2, containing two different heavy chains and a common light chain that pairs with both heavy chains.

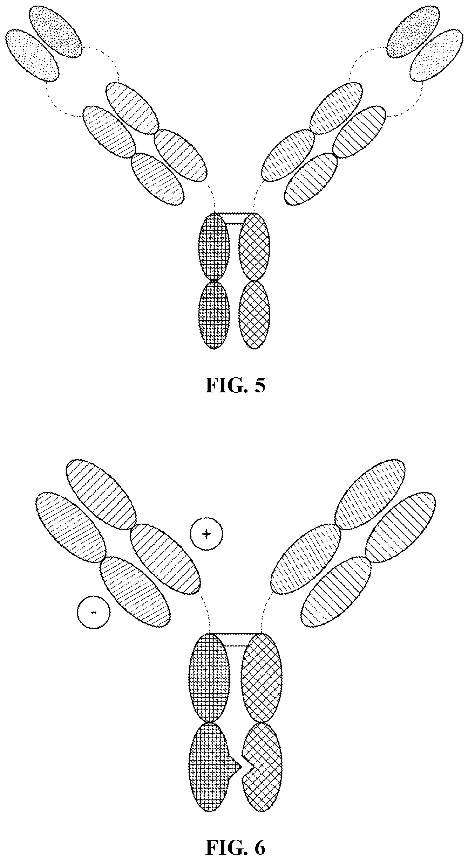

FIG. 5 is a representation of a TriNKET in the dual-variable domain immunoglobulin (DVD-Ig™) form, which combines the target-binding domains of two monoclonal antibodies via flexible naturally occurring linkers, and yields a tetravalent IgG-like molecule. DVD-Ig™ is a homodimeric construct where variable domain targeting antigen 2 is fused to the N-terminus of a variable domain of a Fab fragment targeting antigen 1. DVD-Ig™ form contains normal Fc.

FIG. 6 is a representation of a TriNKET in the Orthogonal Fab fragment interface (Ortho-Fab) form, which is a heterodimeric construct that contains 2 Fab fragments binding to target 1 and target 2 fused to an Fc. Light chain (LC)-heavy chain (HC) pairing is ensured by orthogonal interface. Heterodimerization is ensured by mutations in the Fc.

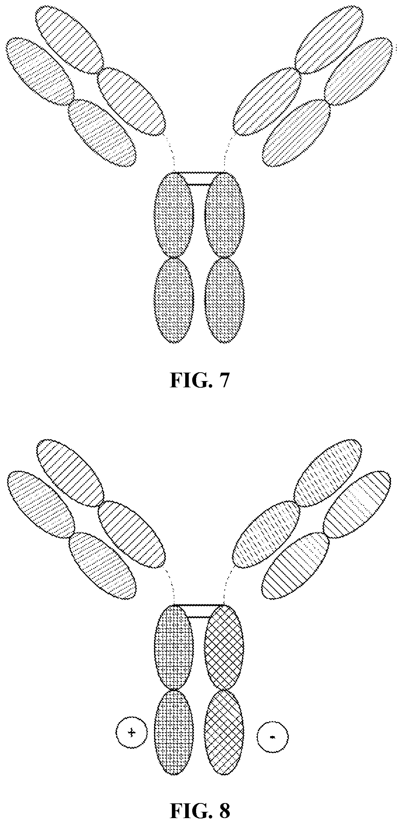

FIG. 7 is a representation of a TriNKET in the 2-in-1 Ig format.

FIG. 8 is a representation of a TriNKET in the ES form, which is a heterodimeric construct containing two different Fab fragments binding to target 1 and target 2 fused to the Fc. Heterodimerization is ensured by electrostatic steering mutations in the Fc.

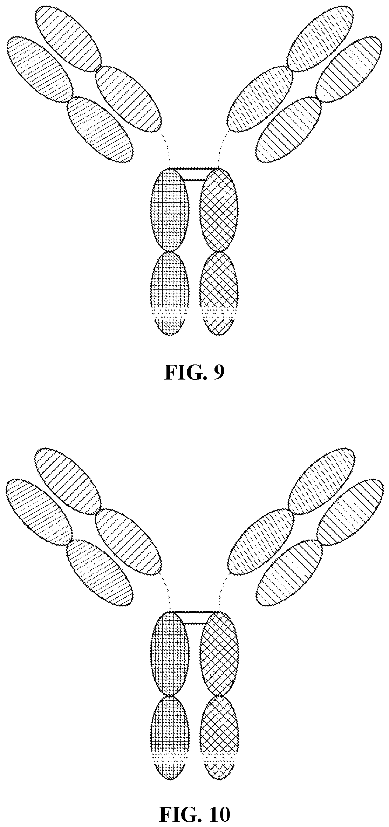

FIG. 9 is a representation of a TriNKET in the Fab Arm Exchange form: antibodies that exchange Fab fragment arms by swapping a heavy chain and attached light chain (half-molecule) with a heavy-light chain pair from another molecule, resulting in bispecific antibodies. Fab Arm Exchange form (cFae) is a heterodimer containing 2 Fab fragments binding to target 1 and 2, and an Fc stabilized by heterodimerization mutations.

FIG. 10 is a representation of a TriNKET in the SEED Body form, which is a heterodimer containing 2 Fab fragments binding to target 1 and 2, and an Fc stabilized by heterodimerization mutations.

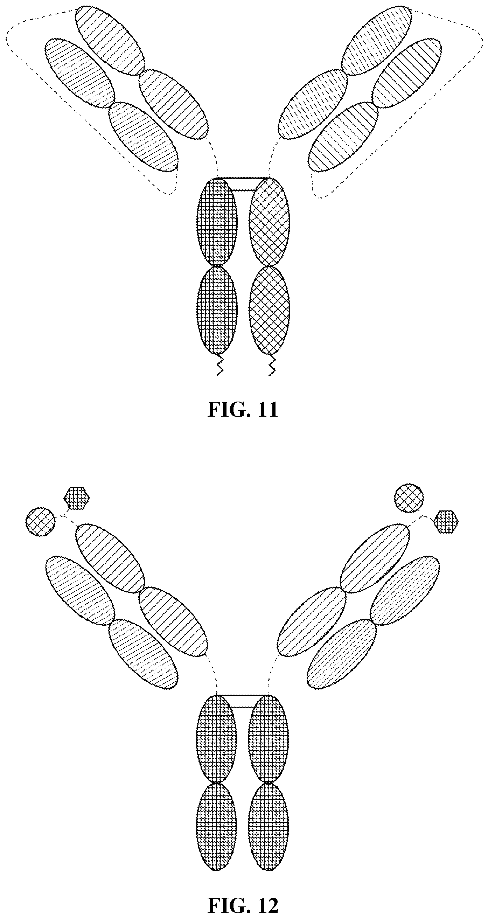

FIG. 11 is a representation of a TriNKET in the LuZ-Y form, in which a leucine zipper is used to induce heterodimerization of two different HCs. The LuZ-Y form is a heterodimer containing two different scFabs binding to target 1 and 2, fused to an Fc. Heterodimerization is ensured through leucine zipper motifs fused to C-terminus of Fc.

FIG. 12 is a representation of a TriNKET in the Cov-X-Body form.

FIGs. 13A-13B are representations of TriNKETs in the $\kappa\lambda$-Body forms, which are heterodimeric constructs with two different Fab fragments fused to an Fc stabilized by heterodimerization mutations: one Fab fragment targeting antigen 1 contains kappa LC, and the second Fab fragment targeting antigen 2 contains lambda LC. **FIG. 13A** is an exemplary representation of one form of a κλ-Body; **FIG. 13B** is an exemplary representation of another κλ-Body.

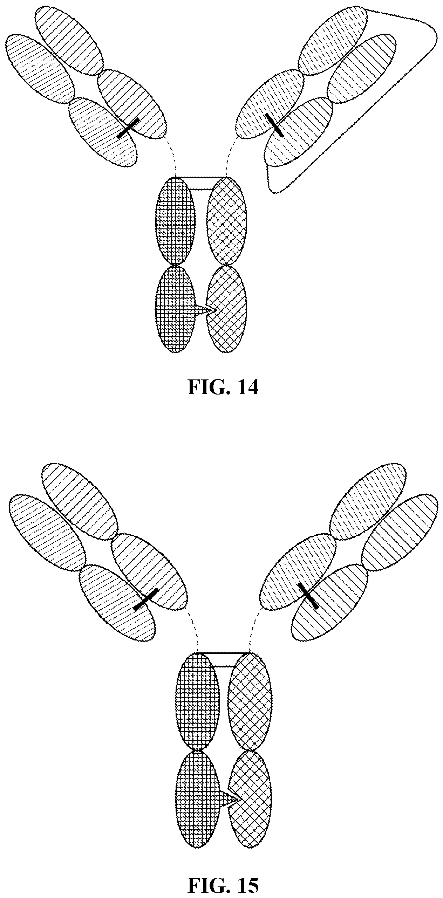

**FIG. 14** is a representation of an Oasc-Fab heterodimeric construct that includes Fab fragment binding to target 1 and scFab binding to target 2, both of which are fused to the Fc domain. Heterodimerization is ensured by mutations in the Fc domain.

**FIG. 15** is a representation of a DuetMab, which is a heterodimeric construct containing two different Fab fragments binding to antigens 1 and 2, and an Fc that is stabilized by heterodimerization mutations. Fab fragments 1 and 2 contain differential S-S bridges that ensure correct light chain and heavy chain pairing.

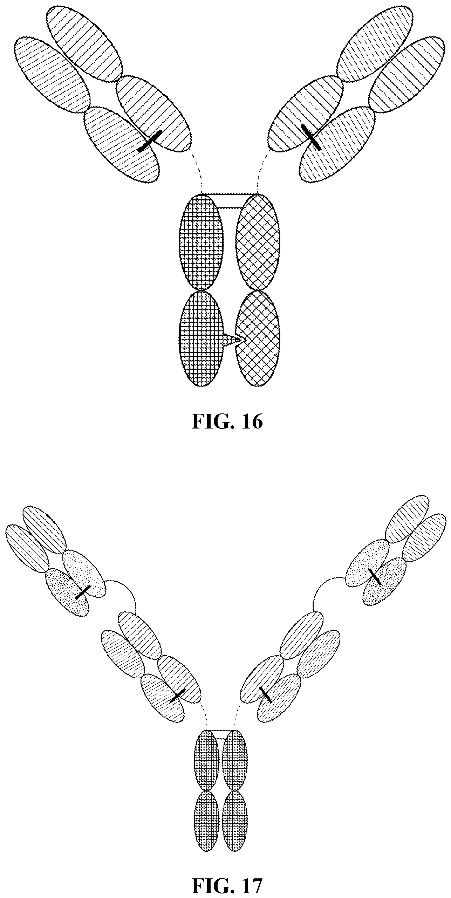

**FIG. 16** is a representation of a CrossmAb, which is a heterodimeric construct with two different Fab fragments binding to targets 1 and 2, and an Fc stabilized by heterodimerization mutations. CL and CH1 domains, and VH and VL domains are switched, *e.g.,* CH1 is fused in-line with VL, and CL is fused in-line with VH.

**FIG. 17** is a representation of a Fit-Ig, which is a homodimeric construct where Fab fragment binding to antigen 2 is fused to the N-terminus of HC of Fab fragment that binds to antigen 1. The construct contains wild-type Fc.

**FIG. 18** is a diagram showing the structural modeling of panitumumab having S62R substitution in the VH (under Chothia numbering scheme), in which the hydrogen bond between this Arg and the Asp at position 1 of the VL may contribute to stabilization of the VH-VL interface.

**FIG. 19** is a diagram showing the structural modeling of panitumumab having F87Y substitution in the VL (under Chothia numbering scheme), in which the hydrogen bond between this Tyr and the Gln at position 39 of the VH may contribute to stabilization of the VH-VL interface.

**FIG. 20** is a diagram showing the structural modeling of panitumumab having D92R substitution in the VL (under Chothia numbering scheme), in which the van der Waals' contact between this Arg (in CDRL3) and the Tyr at position 32 of the VL (in CDRL1) may contribute to stabilization of the paratope.

**FIG. 21** is an SPR sensorgram for a titration of EGFR-TriNKET-1 binding to human EGFR.

**FIG. 22** is an SPR sensorgram for a titration of EGFR-TriNKET-2 binding to human EGFR.

**FIG. 23** is an SPR sensorgram for a titration of EGFR-TriNKET-3 binding to human EGFR.

**FIG. 24** is an SPR sensorgram for a titration of EGFR-TriNKET-4 binding to human EGFR.

**FIGs. 25A, 25B, 25C, and 25D are** plots showing the thermograms for EGFR-TriNKET-1 **(FIG. 25A),** EGFR-TriNKET-2 **(FIG. 25B),** and EGFR-TriNKET-3 in PBS, pH 7.4 **(FIG. 25C),** respectively, as determined by differential scanning calorimetry (DSC) analysis.

**FIGs. 26A and 26B** are plots showing the thermograms for EGFR-TriNKET-3 **(FIG. 26A)** and EGFR-TriNKET-4 **(FIG. 26B),** in 20 mM histidine, 250 mM trehalose, 0.01% PS80, pH 6.0, respectively, as determined by DSC analysis.

**FIG. 27** is a plot showing the binding affinity of a series of concentrations of EGFR-TriNKET-1 ("EGFR1"), EGFR-TriNKET-2 ("EGFR2"), EGFR-TriNKET-3 ("EGFR3"), and panitumumab to EGFR-positive H2172 cancer cells.

**FIG. 28** is a plot showing NK cell-mediated lysis of EGFR-expressing H2172 cancer cells in the presence of a series of concentrations of EGFR-TriNKET-1 ("EGFR1"), EGFR-TriNKET-3 ("EGFR3"), EGFR-TriNKET-4 ("EGFR4"), and cetuximab.

**FIG. 29** is a plot showing CD8[+] T cell-mediated lysis of EGFR positive 786-0 cancer cells in the presence of a series of concentrations of EGFR-TriNKET-3 ("EGFR3"), EGFR-TriNKET-4 ("EGFR4"), and cetuximab.

**FIG. 30** is a plot showing IFN-γ production from NK cells when incubated with EGFR positive BT-474 cancer cells in the presence of a series of concentrations of EGFR-TriNKET-1 ("EGFR1"), EGFR-TriNKET-2 ("EGFR2"), EGFR-TriNKET-3 ("EGFR3"), and cetuximab.

**FIG. 31** is a plot showing IFN-γ production from IL-2 activated NK cells when incubated with EGFR positive BT-474 cancer cells in the presence of a series of concentrations of EGFR-TriNKET-1 ("EGFR1"), EGFR-TriNKET-3 ("EGFR3"), and cetuximab.

**FIG. 32** is a plot showing proliferation of an EGFR-positive cell line for 72 hours in the presence of a series of concentrations of EGFR-TriNKET-1 ("EGFR1"), EGFR-TriNKET-2 ("EGFR2"), EGFR-TriNKET-3 ("EGFR3"), panitumumab, and cetuximab.

**FIG. 33** is a plot showing the percentage of EGFR-expressing target cells engulfed by macrophages as a result of antibody dependent cellular phagocytosis (ADCP) after incubation for 2 hours in the presence of a series of concentrations of EGFR-TriNKET-1 ("EGFR1"), a variant of EGFR-TriNKET-1 having a L234A, L235A, and P329G (LALAPG) mutation in the Fc domain ("EGFR1-CD16si"), EGFR-TriNKET-3 ("EGFR3"), EGFR-TriNKET-4 ("EGFR4"), and cetuximab, as measured by flow cytometry.

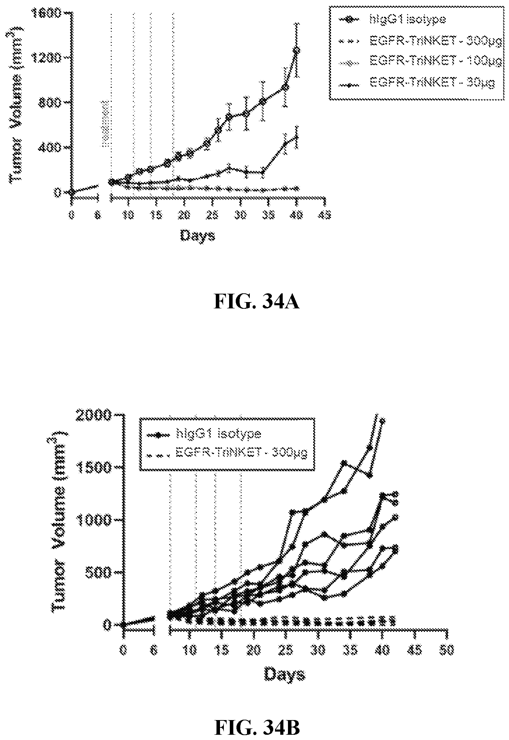

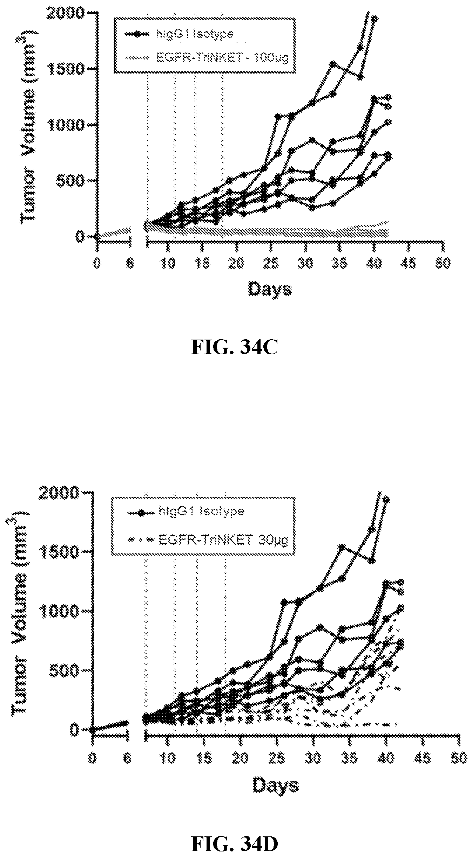

**FIG. 34A-34D** are plots showing tumor volume over time in nude mice xenografted with EGFR-positive NCI-H292 cells treated with isotype control or EGFR-TriNKET as indicated. **FIG. 34A** shows tumor volume in mice treated with 300 μg, 100 μg, or 30 μg of EGFR-TriNKET at indicated timepoints. **FIG. 34B** shows tumor volume in mice treated with 300 μg of isotype control or EGFR-TriNKET at indicated timepoints. **FIG. 34C** shows tumor volume in mice treated with 100 μg of isotype control or EGFR-TriNKET at indicated timepoints. **FIG. 34D** shows tumor volume in mice treated with 30 µg of isotype control or EGFR-TriNKET at indicated timepoints.

**FIG. 35A-35B** are plots showing tumor volume or body weight over time in nude mice xenografted with EGFR-positive NCI-H292 cells treated with isotype control, EGFR-TriNKET, or Cetuximab as indicated. **FIG. 35A** shows tumor volume in mice treated with 100 µg of isotype control, EGFR-TriNKET, or Cetuximab at indicated timepoints. **FIG. 35B** shows body weight in mice treated with 100 µg of isotype control, EGFR-TriNKET, or Cetuximab at indicated timepoints.

DETAILED DESCRIPTION

The present invention provides a protein comprising:

(a) an EGFR-binding scFv (VL-VH) sequence in the orientation of heavy chain variable domain (VH) positioned C-terminal to light chain variable domain (VL), linked to an Fc domain polypeptide to form a (VL-VH)-Fc polypeptide, comprising the amino acid sequence of SEQ ID NO: 167;
and
(b) an NKG2D-binding Fab fragment including:

a heavy chain portion comprising a heavy chain variable domain (VH) and a CH1 domain, wherein the CH1 domain is connected to an Fc domain polypeptide to form a VH-CH1-Fc polypeptide comprising the amino acid sequence of SEQ ID NO: 164, and
a light chain portion comprising a light chain variable domain (VL) and a light chain constant (CL) domain to form a VL-CL polypeptide comprising the amino acid sequence of SEQ ID NO: 165.

The invention is defined by the claims and any other aspects, configurations or embodiments set forth herein not falling within the scope of the claims are for information only.

Any references in the description to methods of treatment refer to the compounds, pharmaceutical compositions and medicaments of the present invention for use in a method for treatment of the human (or animal) body by therapy (or for diagnosis).

The present application provides multispecific binding proteins that bind the NKG2D receptor and CD16 receptor on natural killer cells, and EGFR. In some embodiments, the multispecific binding proteins further include an additional antigen-binding site that binds EGFR. The present application also provides pharmaceutical compositions comprising such multispecific binding proteins, and therapeutic methods using such multispecific binding proteins and pharmaceutical compositions, for purposes such as treating cancer. Various aspects of the multispecific binding proteins described in present application are set forth below in sections; however, aspects of the multispecific binding proteins described in one particular section are not to be limited to any particular section.

To facilitate an understanding of the present application, a number of terms and phrases are defined below.

The terms "a" and "an" as used herein mean "one or more" and include the plural unless the context is inappropriate.

As used herein, the term "antigen-binding site" refers to the part of the immunoglobulin molecule that participates in antigen binding. In human antibodies, the antigen-binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FR." Thus the term "FR" refers to amino acid sequences which are naturally found between and adjacent to hypervariable regions in immunoglobulins. In a human antibody molecule, the three hypervariable regions of a light chain and the three hypervariable regions of a heavy chain are disposed relative to each other in three-dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs." In certain animals, such as camels and cartilaginous fish, the antigen-binding site is formed by a single antibody chain providing a "single domain antibody." Antigen-binding sites can exist in an intact antibody, in an antigen-binding fragment of an antibody that retains the antigen-binding surface, or in a recombinant polypeptide such as an scFv, using a peptide linker to connect the heavy chain variable domain to the light chain variable domain in a single polypeptide.

The term "tumor-associated antigen" as used herein means any antigen including but not limited to a protein, glycoprotein, ganglioside, carbohydrate, lipid that is associated with cancer. Such antigen can be expressed on malignant cells or in the tumor microenvironment such as on tumor-associated blood vessels, extracellular matrix, mesenchymal stroma, or immune infiltrates. In certain embodiments of the present disclosure, the term "tumor-associated antigen" refers to EGFR.

As used herein, the terms "subject" and "patient" refer to an organism to be treated by the methods and compositions described herein. Such organisms preferably include, but are not limited to, mammals (*e.g.*, murines, simians, equines, bovines, porcines, canines, felines, and the like), and more preferably include humans.

As used herein, the term "effective amount" refers to the amount of a compound *(e.g.,* a compound described in the present application) sufficient to effect beneficial or desired results. An effective amount can be administered in one or more administrations, applications or dosages and is not intended to be limited to a particular formulation or administration route. As used herein, the term "treating" includes any effect, e.g., lessening, reducing, modulating, ameliorating or eliminating, that results in the improvement of the condition, disease, disorder, and the like, or ameliorating a symptom thereof.

As used herein, the term "pharmaceutical composition" refers to the combination of an active agent with a carrier, inert or active, making the composition especially suitable for diagnostic or therapeutic use *in vivo* or *ex vivo.*

As used herein, the term "pharmaceutically acceptable carrier" refers to any of the standard pharmaceutical carriers, such as a phosphate buffered saline solution, water, emulsions (e.g., such as an oil/water or water/oil emulsions), and various types of wetting agents. The compositions also can include stabilizers and preservatives. For examples of carriers, stabilizers and adjuvants, *see e.g.,* Martin, Remington's Pharmaceutical Sciences, 15th Ed., Mack Publ. Co., Easton, PA [1975].

As used herein, the term "pharmaceutically acceptable salt" refers to any pharmaceutically acceptable salt (*e.g.,* acid or base) of a compound described in the present application which, upon administration to a subject, is capable of providing a compound described in the present application or an active metabolite or residue thereof. As is known to those of skill in the art, "salts" of the compounds of the present application may be derived from inorganic or organic acids and bases. Exemplary acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the present application and their pharmaceutically acceptable acid addition salts.

Exemplary bases include, but are not limited to, alkali metal (e.g., sodium) hydroxides, alkaline earth metal (e.g., magnesium) hydroxides, ammonia, and compounds of formula $NW_4^+$, wherein W is $C_{1-4}$ alkyl, and the like.

Exemplary salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present application compounded with a suitable cation such as $Na^+$, $NH_4^+$, and $NW_4^+$ (wherein W is a $C_{1-4}$ alkyl group), and the like.

For therapeutic use, salts of the compounds of the present application are contemplated as being pharmaceutically acceptable. However, salts of acids and bases that are non-pharmaceutically acceptable may also find use, for example, in the preparation or purification of a pharmaceutically acceptable compound.

As used herein, EGFR (also known as epidermal growth factor receptor, ErbB-1, or HER1 in humans) refers to the protein of Uniprot Accession No. P00533 (human) and related isoforms and orthologs.

Throughout the description, where compositions are described as having, including, or comprising specific components, or where processes and methods are described as having, including, or comprising specific steps, it is contemplated that, additionally, there are compositions that consist essentially of, or consist of, the recited components, and that there are processes and methods according to the present application that consist essentially of, or consist of, the recited processing steps.

As a general matter, compositions specifying a percentage are by weight unless otherwise specified. Further, if a variable is not accompanied by a definition, then the previous definition of the variable controls.

## I. PROTEINS

The present application provides multispecific binding proteins that bind to the NKG2D receptor and CD16 receptor on natural killer cells, and EGFR. The multispecific binding proteins are useful in the pharmaceutical compositions and therapeutic methods described herein. Binding of the multispecific binding proteins to the NKG2D receptor and CD16 receptor on a natural killer cell enhances the activity of the natural killer cell toward destruction of tumor cells expressing the tumor antigen. Binding of the multispecific binding proteins to tumor antigen expressing tumor cells brings these cells into proximity with the natural killer cell, which facilitates direct and indirect destruction of the tumor cells by the natural killer cell. Multispecific binding proteins that bind NKG2D, CD16, and another target are disclosed in International Application Publication Nos. WO2018148445 and WO2019157366. Further description of some exemplary multispecific binding proteins is provided below.

The first component of the multispecific binding protein is an antigen-binding site that binds to NKG2D receptor-expressing cells, which can include but are not limited to NK cells, $\gamma\delta$ T cells and CD8$^+$ $\alpha\beta$ T cells. Upon NKG2D binding, the multispecific binding proteins may block natural ligands, such as ULBP6 and MICA, from binding to NKG2D and activating NK cells.

The second component of the multispecific binding proteins is an antigen-binding site that binds EGFR. EGFR-expressing cells may be found, for example, in solid tumors, for example, in indications such as lung cancer, breast cancer, kidney cancer, colorectal cancer, gastric cancer, brain cancer, glioma, bladder cancer, head and neck cancer, bladder cancer, pancreatic cancer, and liver cancer, cervical cancer, ovarian cancer or prostate cancer. The antigen-binding site that binds EGFR comprises a heavy chain variable domain (VH) and a light chain variable domain (VL) derived from panitumumab and having mutations in the VH and VL that increase thermostability and retain of affinity to EGFR.

The third component of the multispecific binding proteins is an antibody Fc domain or a portion thereof or an antigen-binding site that binds to cells expressing CD16, an Fc receptor on the surface of leukocytes including natural killer cells, macrophages, neutrophils, eosinophils, mast cells, and follicular dendritic cells.

An additional antigen-binding site of the multispecific binding proteins may bind the same tumor-associated antigen (EGFR). In certain embodiments, the first antigen-binding site that binds NKG2D is an scFv, and the second and the additional antigen-binding sites that bind EGFR are each a Fab fragment. In certain embodiments, the first antigen-binding site that binds NKG2D is an scFv, and the second and the additional antigen-binding sites that bind EGFR are each an scFv. In certain embodiments, the first antigen-binding site that binds NKG2D is a Fab fragment, and the second and the additional antigen-binding sites that bind EGFR are each an scFv. In certain embodiments, the first antigen-binding site that binds NKG2D is a Fab, and the second and the additional antigen-binding sites that bind EGFR are each a Fab fragment.

The antigen-binding sites may each incorporate an antibody heavy chain variable domain and an antibody light chain variable domain (e.g., arranged as in an antibody, or fused together to form an scFv), or one or more of the antigen-binding sites may be a single domain antibody, such as a $V_HH$ antibody like a camelid antibody or a $V_{NAR}$ antibody like those found in cartilaginous fish.

In some embodiments, the second antigen-binding site incorporates a light chain variable domain having an amino acid sequence identical to the amino acid sequence of the light chain variable domain present in the first antigen-binding site.

The multispecific binding proteins described herein can take various formats. For example, one format is a heterodimeric, multispecific antibody including a first immunoglobulin heavy chain, a first immunoglobulin light chain, a second immunoglobulin heavy chain and a second immunoglobulin light chain **(FIG. 1)**. The first immunoglobulin heavy chain includes a first Fc (hinge-CH2-CH3) domain polypeptide, a first heavy chain variable domain and optionally a first CH1 heavy chain domain. The first immunoglobulin light chain includes a first light chain variable domain and optionally a first light chain constant domain. The first immunoglobulin light chain, together with the first immunoglobulin heavy chain, forms an antigen-binding site that binds NKG2D. The second immunoglobulin heavy chain comprises a second Fc (hinge-CH2-CH3) domain polypeptide, a second heavy chain variable domain and optionally a second CH1 heavy chain domain. The second immunoglobulin light chain includes a second light chain variable domain and optionally a second light chain constant domain. The second immunoglobulin light chain, together with the second immunoglobulin heavy chain, forms an antigen-binding site that binds EGFR. In some embodiments, the first Fc domain polypeptide and second Fc domain polypeptide together are able to bind to CD16 **(FIG. 1).** In some embodiments, the first immunoglobulin light chain is identical to the second immunoglobulin light chain.

Another exemplary format involves a heterodimeric, multispecific antibody including a first immunoglobulin heavy chain, a second immunoglobulin heavy chain and an immunoglobulin light chain (e.g., **FIG. 2A).** In some embodiments, the first immunoglobulin heavy chain includes a first Fc (hinge-CH2-CH3) domain polypeptide fused via either a linker or an antibody hinge to a single-chain variable fragment (scFv) composed of a heavy chain variable domain and light chain variable domain, which pair and bind NKG2D or bind EGFR. The second immunoglobulin heavy chain includes a second Fc (hinge-CH2-CH3) domain polypeptide, a second heavy chain variable domain and a CH1 heavy chain domain. The immunoglobulin light chain includes a light chain variable domain and a light chain constant domain. In some embodiments, the second immunoglobulin heavy chain pairs with the immunoglobulin light chain and binds to NKG2D or binds EGFR, with the proviso that when the first Fc domain polypeptide is fused to an scFv that binds NKG2D, the second immunoglobulin heavy chain paired with the immunoglobulin light chain binds EGFR, but not NKG2D, and vice versa. In some embodiments, the scFv in the first immunoglobulin heavy chain binds EGFR; and the heavy chain variable domain in the second immunoglobulin heavy chain and the light chain variable domain in the immunoglobulin light chain, when paired, bind NKG2D (e.g., **FIG. 2E).** In some embodiments, the scFv in the first immunoglobulin heavy chain binds NKG2D; and the heavy chain variable domain in the second immunoglobulin heavy chain and the light chain variable domain in the immunoglobulin light chain, when paired, bind EGFR. In some embodiments, the first Fc domain polypeptide and the second Fc domain polypeptide together are able to bind to CD16 (e.g., **FIG. 2A).**

Another exemplary format involves a heterodimeric, multispecific antibody including a first immunoglobulin heavy chain, and a second immunoglobulin heavy chain (e.g., **FIG. 2B).** In some embodiments, the first immunoglobulin heavy chain includes a first Fc (hinge-CH2-CH3) domain polypeptide fused via either a linker or an antibody hinge to a single-chain variable fragment (scFv) composed of a heavy chain variable domain and light chain variable domain, which pair and bind NKG2D, or bind EGFR. In some embodiments, the second immunoglobulin heavy chain includes a second Fc (hinge-CH2-CH3) domain polypeptide fused via either a linker or an antibody hinge to a single-chain variable fragment (scFv) composed of a heavy chain variable domain and light chain variable domain which pair and bind NKG2D, or EGFR, with the proviso that when the first Fc domain polypeptide is fused to an scFv that binds NKG2D, the second Fc domain polypeptide is fused to an scFv that binds EGFR, but not NKG2D, and vice versa. In some embodiments, the first Fc domain polypeptide and the second Fc domain polypeptide together are able to bind to CD16 (e.g., **FIG. 2B).**

In some embodiments, the single-chain variable fragment (scFv) described above is linked to the antibody constant domain via a hinge sequence. In some embodiments, the hinge comprises amino acids Ala-Ser or Gly-Ser. In some embodiments, the hinge connecting an scFv (e.g., an scFv that binds EGFR or an scFv that binds NKG2D) and the antibody heavy chain constant domain comprises amino acids Ala-Ser. In some embodiments, the hinge connecting an scFv (e.g., an scFv that binds EGFR or an scFv that binds NKG2D) and the antibody heavy chain constant domain comprises amino acids Gly-Ser. In some other embodiments, the hinge comprises amino acids Ala-Ser and Thr-Lys-Gly. The hinge sequence can provide flexibility of binding to the target antigen, and balance between flexibility and optimal geometry.

In some embodiments, the single-chain variable fragment (scFv) described above includes a heavy chain variable domain and a light chain variable domain. In some embodiments, the heavy chain variable domain forms a disulfide bridge with the light chain variable domain to enhance stability of the scFv. For example, a disulfide bridge can be formed between the C44 residue of the heavy chain variable domain and the C100 residue of the light chain variable domain, the amino acid positions numbered under Kabat. In some embodiments, the heavy chain variable domain is linked to the light chain variable domain via a flexible linker. Any suitable linker can be used, for example, the $(G_4S)_4$ linker $((GlyGlyGlyGlySer)_4$ (SEQ ID NO: 119)). In some embodiments of the scFv, the heavy chain variable domain is positioned at the N-terminus of the light chain variable domain. In some embodiments of the scFv, the heavy chain variable domain is positioned at the C terminus of the light chain variable domain.

The multispecific binding proteins described herein can further include one or more additional antigen-binding sites. The additional antigen-binding site(s) may be fused to the N-terminus of the constant region CH2 domain or to the C-terminus of the constant region CH3 domain, optionally via a linker sequence. In certain embodiments, the additional antigen-binding site(s) takes the form of a single-chain variable region (scFv) that is optionally disulfide-stabilized, resulting in a tetravalent or trivalent multispecific binding protein. For example, a multispecific binding protein includes a first antigen-binding site that binds NKG2D, a second antigen-binding site that binds EGFR, an additional antigen-binding site that binds EGFR, and an antibody constant region or a portion thereof sufficient to bind CD16 or a fourth antigen-binding site that binds CD16. Any one of these antigen-binding sites can either take the form of a Fab fragment or an scFv, such as an scFv described above.

In some embodiments, the additional antigen-binding site binds a different epitope of EGFR from the second antigen-binding site. In some embodiments, the additional antigen-binding site binds the same epitope as the second antigen-binding site. In some embodiments, the additional antigen-binding site comprises the same heavy chain and light chain CDR sequences as the second antigen-binding site. In some embodiments, the additional antigen-binding site comprises the same heavy chain and light chain variable domain sequences as the second antigen-binding site. In some embodiments, the additional antigen-binding site has the same amino acid sequence(s) as the second antigen-binding site. In some embodiments, the additional antigen-binding site comprises heavy chain and light chain variable domain sequences that are different from the heavy chain and light chain variable domain sequences of the second antigen-binding site. In some embodiments, the additional antigen-binding site has an amino acid sequence that is different from the sequence of the second antigen-binding site. In some embodiments, the second antigen-binding site and the additional antigen-binding site bind different tumor-associated antigens. In some embodiments, the second antigen-binding site and the additional antigen-binding site binds different antigens. Exemplary formats are shown in **FIG. 2C** and **FIG. 2D.** Accordingly, the multispecific binding proteins can provide bivalent engagement of EGFR. Bivalent engagement of EGFR by the multispecific binding proteins can stabilize EGFR on the tumor cell surface and enhance cytotoxicity of NK cells towards the tumor cells. Bivalent engagement of EGFR by the multispecific binding proteins can confer stronger binding of the multispecific binding proteins to the tumor cells, thereby facilitating stronger cytotoxic response of NK cells towards the tumor cells, especially towards tumor cells expressing a low level of EGFR.

The multispecific binding proteins can take additional formats. In some embodiments, the multispecific binding protein is in the Triomab form, which is a trifunctional, bispecific antibody that maintains an IgG-like shape. This chimera consists of two half antibodies, each with one light and one heavy chain, that originate from two parental antibodies.

In some embodiments, the multispecific binding protein is the KiH form, which involves the knobs-into-holes (KiHs) technology. The KiH involves engineering $C_H3$ domains to create either a "knob" or a "hole" in each heavy chain to promote heterodimerization. The concept behind the "Knobs-into-Holes (KiH)" Fc technology was to introduce a "knob" in one CH3 domain (CH3A) by substitution of a small residue with a bulky one (*e.g.*, T366W$_{CH3A}$ in EU numbering). To accommodate the "knob," a complementary "hole" surface was created on the other CH3 domain (CH3B) by replacing the closest neighboring residues to the knob with smaller ones *(e.g.,* T366S/L368A/Y407V$_{CH3B}$). The "hole" mutation was optimized by structured-guided phage library screening (Atwell S, Ridgway JB, Wells JA, Carter P., Stable heterodimers from remodeling the domain interface of a homodimer using a phage display library, J. Mol. Biol. (1997) 270(1):26-35). X-ray crystal structures of KiH Fc variants (Elliott JM, Ultsch M, Lee J, Tong R, Takeda K, Spiess C, et al., Antiparallel conformation of knob and hole aglycosylated half-antibody homodimers is mediated by a CH2-CH3 hydrophobic interaction. J. Mol. Biol. (2014) 426(9):1947-57; Mimoto F, Kadono S, Katada H, Igawa T, Kamikawa T, Hattori K. Crystal structure of a novel asymmetrically engineered Fc variant with improved affinity for FcγRs. Mol. Immunol. (2014) 58(1):132-8) demonstrated that heterodimerization is thermodynamically favored by hydrophobic interactions driven by steric complementarity at the inter-CH3 domain core interface, whereas the knob-knob and the hole-hole interfaces do not favor homodimerization owing to steric hindrance and disruption of the favorable interactions, respectively.

In some embodiments, the multispecific binding protein is in the dual-variable domain immunoglobulin (DVD-Ig™) form, which combines the target binding domains of two monoclonal antibodies via flexible naturally occurring linkers, and yields a tetravalent IgG-like molecule.

In some embodiments, the multispecific binding protein is in the Orthogonal Fab interface (Ortho-Fab) form. In the ortho-Fab IgG approach (Lewis SM, Wu X, Pustilnik A, Sereno A, Huang F, Rick HL, et al., Generation of bispecific IgG antibodies by structure-based design of an orthogonal Fab interface. Nat. Biotechnol. (2014) 32(2):191-8), structure-based regional design introduces complementary mutations at the LC and HC$_{VH-CH1}$ interface in only one Fab fragment, without any changes being made to the other Fab fragment.

In some embodiments, the multispecific binding protein is in the 2-in-1 Ig format. In some embodiments, the multispecific binding protein is in the ES form, which is a heterodimeric construct containing two different Fab fragments binding to targets 1 and target 2 fused to the Fc. Heterodimerization is ensured by electrostatic steering mutations in the Fc.

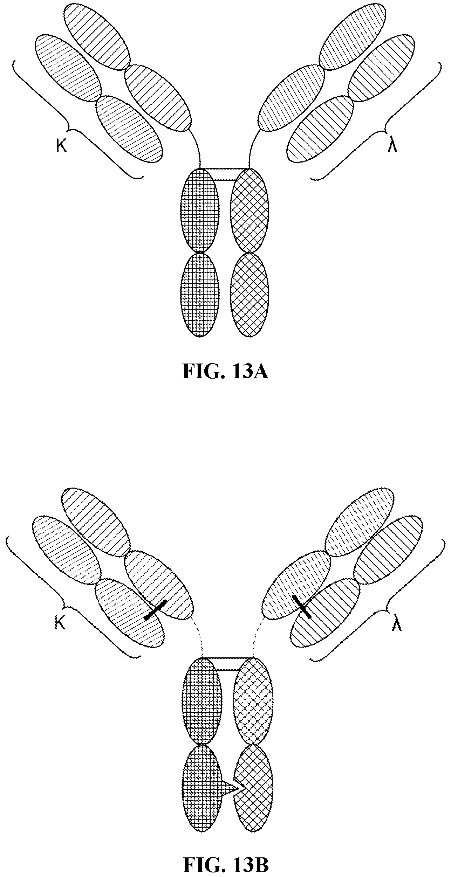

In some embodiments, the multispecific binding protein is in the κλ-Body form, which is a heterodimeric construct with two different Fab fragments fused to an Fc stabilized by heterodimerization mutations: Fab fragment 1 targeting antigen 1 contains kappa LC, while Fab fragment 2 targeting antigen 2 contains lambda LC. **FIG. 13A** is an exemplary representation of one form of a κλ-Body; **FIG. 13B** is an exemplary representation of another κλ-Body.

In some embodiments, the multispecific binding protein is in Fab Arm Exchange form (antibodies that exchange Fab fragment arms by swapping a heavy chain and attached light chain (half-molecule) with a heavy-light chain pair from another molecule, which results in bispecific antibodies).

In some embodiments, the multispecific binding protein is in the SEED Body form. The strand-exchange engineered domain (SEED) platform was designed to generate asymmetric and bispecific antibody-like molecules, a capability that expands therapeutic applications of natural antibodies. This protein engineering platform is based on exchanging structurally related sequences of immunoglobulin within the conserved CH3 domains. The SEED design allows efficient generation of AG/GA heterodimers, while disfavoring homodimerization of AG and GA SEED CH3 domains. (Muda M. et al., Protein Eng. Des. Sel. (2011, 24(5):447-54)).

In some embodiments, the multispecific binding protein is in the LuZ-Y form, in which a leucine zipper is used to induce heterodimerization of two different HCs. (Wranik, BJ. et al., J. Biol. Chem. (2012), 287:43331-9).

In some embodiments, the multispecific binding protein is in the Cov-X-Body form. In bispecific CovX-Bodies, two different peptides are joined together using a branched azetidinone linker and fused to the scaffold antibody under mild conditions in a site-specific manner. Whereas the pharmacophores are responsible for functional activities, the antibody scaffold imparts long half-life and Ig-like distribution. The pharmacophores can be chemically optimized or replaced with other pharmacophores to generate optimized or unique bispecific antibodies. (Doppalapudi VR et al., PNAS (2010), 107(52);22611-22616).

In some embodiments, the multispecific binding protein is in an Oasc-Fab heterodimeric form that includes a Fab fragment binding to target 1, and a scFab binding to target 2 fused to Fc. Heterodimerization is ensured by mutations in the Fc.

In some embodiments, the multispecific binding protein is in a DuetMab form, which is a heterodimeric construct containing two different Fab fragments binding to antigens 1 and 2, and an Fc stabilized by heterodimerization mutations. Fab fragments 1 and 2 contain differential S-S bridges that ensure correct LC and HC pairing.

In some embodiments, the multispecific binding protein is in a CrossMab form, which is a heterodimeric construct with two different Fab fragments binding to targets 1 and 2, fused to an Fc stabilized by heterodimerization. CL and CH1 domains and VH and VL domains are switched, *e.g.,* CH1 is fused in-frame with VL, while CL is fused in-frame with VH.

In some embodiments, the multispecific binding protein is in a Fit-Ig form, which is a homodimeric construct where a Fab fragment binding to antigen 2 is fused to the N terminus of HC of a Fab fragment that binds to antigen 1. The construct contains wild-type Fc.

Individual components of the multispecific binding proteins are described in more detail below.

**NKG2D-binding site**

Upon binding to the NKG2D receptor and CD16 receptor on natural killer cells, and EGFR, the multispecific binding proteins can engage more than one kind of NK-activating receptor, and may block the binding of natural ligands to NKG2D. In certain embodiments, the proteins can agonize NK cells in humans. In some embodiments, the proteins can agonize NK cells in humans and in other species such as rodents and cynomolgus monkeys. In some embodiments, the proteins can agonize NK cells in humans and in other species such as cynomolgus monkeys.

**Table 1** lists peptide sequences of heavy chain variable domains and light chain variable domains that, in combination, can bind to NKG2D. In some embodiments, the heavy chain variable domain and the light chain variable domain are arranged in Fab format. In some embodiments, the heavy chain variable domain and the light chain variable domain are fused together to form an scFv.

The NKG2D binding sites listed in **Table 1** can vary in their binding affinity to NKG2D, nevertheless, they all activate human NK cells.

Unless indicated otherwise, the CDR sequences provided in **Table 1** are determined under Kabat numbering.

| Table 1 | | |
|---|---|---|
| Clones | Heavy chain variable region amino acid sequence | Light chain variable region amino acid sequence |
| ADI-27705 | QVQLQQWGAGLLKPSETLSLTCAVYGG<br>SFSGYYWSWIRQPPGKGLEWIGEIDHSG<br>STNYNPSLKSRVTISVDTSKNQFSLKLSS | DIQMTQSPSTLSASVGDRVTITCRAS<br>QSISSWLAWYQQKPGKAPKLLIYKA<br>SSLESGVPSRFSGSGSGTEFTLTISSL |
| | VTAADTAVYYCARARGPWSFDPWGQG<br>TLVTVSS<br>(SEQ ID NO:1)<br>CDR1 (SEQ ID NO:2) - GSFSGYYWS<br>CDR2 (SEQ ID NO:3) - EIDHSGSTNYNPSLKS<br>CDR3 (SEQ ID NO:4) - ARARGPWSFDP | QPDDFATYYCQQYNSYPITFGGGTK<br>VEIK<br>(SEQ ID NO:5) |
| ADI-27724 | QVQLQQWGAGLLKPSETLSLTCAVYGG<br>SFSGYYWSWIRQPPGKGLEWIGEIDHSG<br>STNYNPSLKSRVTISVDTSKNQFSLKLSS<br>VTAADTAVYYCARARGPWSFDPWGQG<br>TLVTVSS<br>(SEQ ID NO:1) | EIVLTQSPGTLSLSPGERATLSCRASQ<br>SVSSSYLAWYQQKPGQAPRLLIYGA<br>SSRATGIPDRFSGSGSGTDFTLTISRL<br>EPEDFAVYYCQQYGSSPITFGGGTK<br>VEIK<br>(SEQ ID NO:6) |
| ADI-27740<br>(A40) | QVQLQQWGAGLLKPSETLSLTCAVYGG<br>SFSGYYWSWIRQPPGKGLEWIGEIDHSG<br>STNYNPSLKSRVTISVDTSKNQFSLKLSS<br>VTAADTAVYYCARARGPWSFDPWGQG<br>TLVTVSS<br>(SEQ ID NO:1) | DIQMTQSPSTLSASVGDRVTITCRAS<br>QSIGSWLAWYQQKPGKAPKLLIYKA<br>SSLESGVPSRFSGSGSGTEFTLTISSL<br>QPDDFATYYCQQYHSFYTFGGGTK<br>VEIK<br>(SEQ ID NO:7) |

| Table 1 | | |
|---|---|---|
| Clones | Heavy chain variable region amino acid sequence | Light chain variable region amino acid sequence |
| ADI-27741 | QVQLQQWGAGLLKPSETLSLTCAVYGG<br>SFSGYYWSWIRQPPGKGLEWIGEIDHSG<br>STNYNPSLKSRVTISVDTSKNQFSLKLSS<br>VTAADTAVYYCARARGPWSFDPWGQG<br>TLVTVSS<br>(SEQ ID NO:1) | DIQMTQSPSTLSASVGDRVTITCRAS<br>QSIGSWLAWYQQKPGKAPKLLIYKA<br>SSLESGVPSRFSGSGSGTEFTLTISSL<br>QPDDFATYYCQQSNSYYTFGGGTK<br>VEIK<br>(SEQ ID NO:8) |
| ADI-27743 | QVQLQQWGAGLLKPSETLSLTCAVYGG<br>SFSGYYWSWIRQPPGKGLEWIGEIDHSG<br>STNYNPSLKSRVTISVDTSKNQFSLKLSS<br>VTAADTAVYYCARARGPWSFDPWGQG<br>TLVTVSS<br>(SEQ ID NO:1) | DIQMTQSPSTLSASVGDRVTITCRAS<br>QSISSWLAWYQQKPGKAPKLLIYKA<br>SSLESGVPSRFSGSGSGTEFTLTISSL<br>QPDDFATYYCQQYNSYPTFGGGTK<br>VEIK<br>(SEQ ID NO:9) |
| ADI-28153 | QVQLQQWGAGLLKPSETLSLTCAVYGG<br>SFSGYYWSWIRQPPGKGLEWIGEIDHSG<br>STNYNPSLKSRVTISVDTSKNQFSLKLSS<br>VTAADTAVYYCARARGPWGFDPWGQG<br>TLVTVSS<br>(SEQ ID NO:10) | ELQMTQSPSSLSASVGDRVTITCRTS<br>QSISSYLNWYQQKPGQPPKLLIYWA<br>STRESGVPDRFSGSGSGTDFTLTISSL<br>QPEDSATYYCQQSYDIPYTFGQGTK<br>LEIK<br>(SEQ ID NO:11) |
| ADI-28226<br>(C26) | QVQLQQWGAGLLKPSETLSLTCAVYGG<br>SFSGYYWSWIRQPPGKGLEWIGEIDHSG | DIQMTQSPSTLSASVGDRVTITCRAS<br>QSISSWLAWYQQKPGKAPKLLIYKA |

| Clones | Heavy chain variable region amino acid sequence | Light chain variable region amino acid sequence |
|---|---|---|
| | STNYNPSLKSRVTISVDTSKNQFSLKLSS VTAADTAVYYCARARGPWSFDPWGQG TLVTVSS (SEQ ID NO:1) | SSLESGVPSRFSGSGSGTEFTLTISSL QPDDFATYYCQQYGSFPITFGGGTK VEIK (SEQ ID NO:12) |
| ADI-28154 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSISSWLAWYQQKPGKAPKLLIYKA SSLESGVPSRFSGSGSGTDFTLTISSL QPDDFATYYCQQSKEVPWTFGQGT KVEIK (SEQ ID NO:13) |
| ADI-29399 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSISSWLAWYQQKPGKAPKLLIYKA SSLESGVPSRFSGSGSGTEFTLTISSL QPDDFATYYCQQYNSFPTFGGGTKV EIK (SEQ ID NO:14) |
| ADI-29401 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSIGSWLAWYQQKPGKAPKLLIYKA SSLESGVPSRFSGSGSGTEFTLTISSL QPDDFATYYCQQYDIYPTFGGGTKV EIK (SEQ ID NO:15) |

EP 4 192 872 B1

| Table 1 | | |
|---|---|---|
| Clones | Heavy chain variable region amino acid sequence | Light chain variable region amino acid sequence |
| ADI-29403 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSISSWLAWYQQKPGKAPKLLIYKA SSLESGVPSRFSGSGSGTEFTLTISSL QPDDFATYYCQQYDSYPTFGGGTK VEIK (SEQ ID NO:16) |
| ADI-29405 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSISSWLAWYQQKPGKAPKLLIYKA SSLESGVPSRFSGSGSGTEFTLTISSL QPDDFATYYCQQYGSFPTFGGGTKV EIK (SEQ ID NO:17) |
| ADI-29407 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSISSWLAWYQQKPGKAPKLLIYKA SSLESGVPSRFSGSGSGTEFTLTISSL QPDDFATYYCQQYQSFPTFGGGTKV EIK (SEQ ID NO:18) |

(continued)

**Table 1**

| Clones | Heavy chain variable region amino acid sequence | Light chain variable region amino acid sequence |
|---|---|---|
| ADI-29419 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSISSWLAWYQQKPGKAPKLLIYKA SSLESGVPSRFSGSGSGTEFTLTISSL QPDDFATYYCQQYSSFSTFGGGTKV EIK (SEQ ID NO:19) |
| ADI-29421 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSISSWLAWYQQKPGKAPKLLIYKA SSLESGVPSRFSGSGSGTEFTLTISSL QPDDFATYYCQQYESYSTFGGGTKV EIK (SEQ ID NO:20) |
| ADI-29424 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSISSWLAWYQQKPGKAPKLLIYKA SSLESGVPSRFSGSGSGTEFTLTISSL QPDDFATYYCQQYDSFITFGGGTKV EIK (SEQ ID NO:21) |

EP 4 192 872 B1

| Table 1 | | |
|---|---|---|
| Clones | Heavy chain variable region amino acid sequence | Light chain variable region amino acid sequence |
| ADI-29425 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSISSWLAWYQQKPGKAPKLLIYKA SSLESGVPSRFSGSGSGTEFTLTISSL QPDDFATYYCQQYQSYPTFGGGTK VEIK (SEQ ID NO:22) |
| ADI-29426 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSIGSWLAWYQQKPGKAPKLLIYKA SSLESGVPSRFSGSGSGTEFTLTISSL QPDDFATYYCQQYHSFPTFGGGTKV EIK (SEQ ID NO:23) |
| ADI-29429 | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSIGSWLAWYQQKPGKAPKLLIYKA SSLESGVPSRFSGSGSGTEFTLTISSL QPDDFATYYCQQYELYSYTFGGGTK VEIK (SEQ ID NO:24) |
| ADI-29447 (F47) | QVQLQQWGAGLLKPSETLSLTCAVYGG SFSGYYWSWIRQPPGKGLEWIGEIDHSG STNYNPSLKSRVTISVDTSKNQFSLKLSS | DIQMTQSPSTLSASVGDRVTITCRAS QSISSWLAWYQQKPGKAPKLLIYKA SSLESGVPSRFSGSGSGTEFTLTISSL |

(continued)

| Table 1 | | |
| --- | --- | --- |
| Clones | Heavy chain variable region amino acid sequence | Light chain variable region amino acid sequence |
| | VTAADTAVYYCARARGPWSFDPWGQG<br><br>TLVTVSS<br><br>(SEQ ID NO:1) | QPDDFATYYCQQYDTFITFGGGTKV<br><br>EIK<br><br>(SEQ ID NO:25) |
| ADI-27727 | QVQLVQSGAEVKKPGSSVKVSCKASGG<br><br>TFSSYAISWVRQAPGQGLEWMGGIIPIFG<br><br>TANYAQKFQGRVTITADESTSTAYMELS<br><br>SLRSEDTAVYYCARGDSSIRHAYYYYG<br><br>MDVWGQGTTVTVSS<br><br>(SEQ ID NO:26)<br>CDR1- GTFSSYAIS (non-Kabat) (SEQ ID NO:27) or SYAIS (SEQ ID NO:28)<br>CDR2 (SEQ ID NO:29) - GIIPIFGTANYAQKFQG<br>CDR3 - ARGDSSIRHAYYYYGMDV (non-Kabat) (SEQ ID NO:30) or GDSSIRHAYYYYGMDV (SEQ ID NO:31) | DIVMTQSPDSLAVSLGERATINCKSS<br><br>QSVLYSSNNKNYLAWYQQKPGQPP<br><br>KLLIYWASTRESGVPDRFSGSGSGTD<br><br>FTLTISSLQAEDVAVYYCQQYYSTPI<br><br>TFGGGTKVEIK<br><br>(SEQ ID NO:32)<br>CDR1 (SEQ ID NO:33) - KSSQSVLYSSNNKNYLA<br>CDR2 (SEQ ID NO:34) - WASTRES<br>CDR3 (SEQ ID NO:35) - QQYYSTPIT |
| ADI-29443 (F43) | QLQLQESGPGLVKPSETLSLTCTVSGGSI<br><br>SSSSYYWGWIRQPPGKGLEWIGSIYYSGS<br><br>TYYNPSLKSRVTISVDTSKNQFSLKLSSV<br><br>TAADTAVYYCARGSDRFHPYFDYWGQG<br><br>TLVTVSS<br><br>(SEQ ID NO:36)<br>CDR1- GSISSSSYYWG (non-Kabat) (SEQ ID NO:37) or SSSYYWG (SEQ ID NO:38)<br>CDR2 (SEQ ID NO:39) - SIYYSGSTYYNPSLKS<br>CDR3 - ARGSDRFHPYFDY (non-Kabat) (SEQ ID NO:40) or GSDRFHPYFDY (SEQ ID NO:41) | EIVLTQSPATLSLSPGERATLSCRASQ<br><br>SVSRYLAWYQQKPGQAPRLLIYDAS<br><br>NRATGIPARFSGSGSGTDFTLTISSLE<br><br>PEDFAVYYCQQFDTWPPTFGGGTKV<br><br>EIK<br><br>(SEQ ID NO:42)<br>CDR1 (SEQ ID NO:43) - RASQSVSRYLA<br><br>CDR2 (SEQ ID NO:44) - DASNRAT<br>CDR3 (SEQ ID NO:45) - QQFDTWPPT |

(continued)

| Table 1 | | |
|---|---|---|
| **Clones** | **Heavy chain variable region amino acid sequence** | **Light chain variable region amino acid sequence** |
| ADI-29404 (F04) | QVQLQQWGAGLLKPSETLSLTCAVYGG SFSGYYWSWIRQPPGKGLEWIGEIDHSG STNYNPSLKSRVTISVDTSKNQFSLKLSS VTAADTAVYYCARARGPWSFDPWGQG TLVTVSS (SEQ ID NO:1) | DIQMTQSPSTLSASVGDRVTITCRAS QSISSWLAWYQQKPGKAPKLLIYKA SSLESGVPSRFSGSGSGTEFTLTISSL QPDDFATYYCEQYDSYPTFGGGTKV EIK (SEQ ID NO:46) |
| ADI-28200 | QVQLVQSGAEVKKPGSSVKVSCKASGG TFSSYAISWVRQAPGQGLEWMGGIIPIFG TANYAQKFQGRVTITADESTSTAYMELS SLRSEDTAVYYCARRGRKASGSFYYYY GMDVWGQGTTVTVSS (SEQ ID NO:47) CDR1 (SEQ ID NO:27) - GTFSSYAIS CDR2 (SEQ ID NO:29) - GIIPIFGTANYAQKFQG<br><br>CDR3 (SEQ ID NO:48) - ARRGRKASGSFYYYYGMDV | DIVMTQSPDSLAVSLGERATINCESS QSLLNSGNQKNYLTWYQQKPGQPP KPLIYWASTRESGVPDRFSGSGSGTD FTLTISSLQAEDVAVYYCQNDYSYP YTFGQGTKLEIK (SEQ ID NO:49) CDR1 (SEQ ID NO:50) - ESSQSLLNSGNQKNYLT CDR2 (SEQ ID NO:34) - WASTRES<br><br>CDR3 (SEQ ID NO:51) - QNDYSYPYT |
| ADI-29379 (E79) | QVQLVQSGAEVKKPGASVKVSCKASGY TFTSYYMHWVRQAPGQGLEWMGIINPS GGSTSYAQKFQGRVTMTRDTSTSTVYM ELSSLRSEDTAVYYCARGAPNYGDTTHD YYYMDVWGKGTTVTVSS (SEQ ID NO:52) CDR1 (SEQ ID NO:53) - YTFTSYYMH (non-Kabat) or SYYMH (SEQ ID NO:54) CDR2 (SEQ ID NO:55) - IINPSGGSTSYAQKFQG | EIVMTQSPATLSVSPGERATLSCRAS QSVSSNLAWYQQKPGQAPRLLIYGA STRATGIPARFSGSGSGTEFTLTISSL QSEDFAVYYCQQYDDWPFTFGGGT KVEIK (SEQ ID NO:58) CDR1 (SEQ ID NO:59) - RASQSVSSNLA<br><br>CDR2 (SEQ ID NO:60) - GASTRAT CDR3 (SEQ ID NO:61) - QQYDDWPFT |

| Table 1 | | |
|---|---|---|
| **Clones** | **Heavy chain variable region amino acid sequence** | **Light chain variable region amino acid sequence** |
| | CDR3 - ARGAPNYGDTTHDYYYMDV (non-Kabat) (SEQ ID NO:56) or GAPNYGDTTHDYYYMDV (SEQ ID NO:57) | |
| ADI-29463 (F63) | QVQLVQSGAEVKKPGASVKVSCKASGY TFTGYYMHWVRQAPGQGLEWMGWINP NSGGTNYAQKFQGRVTMTRDTSISTAY MELSRLRSDDTAVYYCARDTGEYYDTD DHGMDVWGQGTTVTVSS (SEQ ID NO:62) CDR1 - YTFTGYYMH (non-Kabat) (SEQ ID NO:63) or GYYMH (SEQ ID NO:64) CDR2 (SEQ ID NO:65) - WINPNSGGTNYAQKFQG CDR3 - ARDTGEYYDTDDHGMDV (non-Kabat) (SEQ ID NO:66) or DTGEYYDTDDHGMDV (SEQ ID NO:67) | EIVLTQSPGTLSLSPGERATLSCRASQ SVSSNLAWYQQKPGQAPRLLIYGAS TRATGIPARFSGSGSGTEFTLTISSLQ SEDFAVYYCQQDDYWPPTFGGGTK VEIK (SEQ ID NO:68) CDR1 (SEQ ID NO:59) - RASQSVSSNLA CDR2 (SEQ ID NO:60) - GASTRAT CDR3 (SEQ ID NO:69) - QQDDYWPPT |
| ADI-27744 (A44) | EVQLLESGGGLVQPGGSLRLSCAASGFT FSSYAMSWVRQAPGKGLEWVSAISGSG GSTYYADSVKGRFTISRDNSKNTLYLQM NSLRAEDTAVYYCAKDGGYYDSGAGD YWGQGTLVTVSS (SEQ ID NO:70) CDR1- FTFSSYAMS (non-Kabat) (SEQ ID NO:71) or SYAMS (SEQ ID NO:115) CDR2 (SEQ ID NO:72) - AISGSGGSTYYADSVKG CDR3 - AKDGGYYDSGAGDY (non-Kabat) (SEQ ID NO:73) or DGGYYDSGAGDY (SEQ ID NO:74) | DIQMTQSPSSVSASVGDRVTITCRAS QGIDSWLAWYQQKPGKAPKLLIYA ASSLQSGVPSRFSGSGSGTDFTLTISS LQPEDFATYYCQQGVSYPRTFGGGT KVEIK (SEQ ID NO:75) CDR1 (SEQ ID NO:76) - RASQGIDSWLA CDR2 (SEQ ID NO:77) - AASSLQS CDR3 (SEQ ID NO:78) - QQGVSYPRT |

| | Table 1 | |
|---|---|---|
| **Clones** | **Heavy chain variable region amino acid sequence** | **Light chain variable region amino acid sequence** |
| ADI-27749 (A49) | EVQLVESGGGLVKPGGSLRLSCAASGFT FSSYSMNWVRQAPGKGLEWVSSISSSSS YIYYADSVKGRFTISRDNAKNSLYLQMN SLRAEDTAVYYCARGAPMGAAAGWFD PWGQGTLVTVSS (SEQ ID NO:79) CDR1 - FTFSSYSMN (SEQ ID NO:80) (non-Kabat) or SYSMN (SEQ ID NO:81) CDR2 (SEQ ID NO:82) - SISSSSSYIYYADSVKG CDR3- ARGAPMGAAAGWFDP (SEQ ID NO:83) (non-Kabat) or GAPMGAAAGWFDP (SEQ ID NO:84) | DIQMTQSPSSVSASVGDRVTITCRAS QGISSWLAWYQQKPGKAPKLLIYAA SSLQSGVPSRFSGSGSGTDFTLTISSL QPEDFATYYCQQGVSFPRTFGGGTK VEIK (SEQ ID NO:85) CDR1 (SEQ ID NO:86) - RASQGISSWLA CDR2 (SEQ ID NO:77) - AASSLQS CDR3 (SEQ ID NO:87) - QQGVSFPRT |
| | scFv (VL-VH) with Q44C in VH and G100C in VL, linker italicized: DIQMTQSPSSVSASVGDRVTITCRASQGISSWLAWYQQKPGKAPKLLIYAASSLQS GVPSRFSGSGSGTDFTLTISSLQPEDFATYYCQQGVSFPRTFG<u>C</u>GTKVEIK*GGGGSG GGGSGGGGSGGGGS*EVQLVESGGGLVKPGGSLRLSCAASGFTFSSYSMNWVRQA PGK<u>C</u>LEWVSSISSSSSYIYYADSVKGRFTISRDNAKNSLYLQMNSLRAEDTAVYYC ARGAPMGAAAGWFDPWGQGTLVTVSS (SEQ ID NO:88) | |
| ADI-29378 (E78) | QVQLVQSGAEVKKPGASVKVSCKASGY TFTSYYMHWVRQAPGQGLEWMGIINPS GGSTSYAQKFQGRVTMTRDTSTSTVYM ELSSLRSEDTAVYYCAREGAGFAYGMD YYYMDVWGKGTTVTVSS (SEQ ID NO:89) CDR1 - YTFTSYYMH (SEQ ID NO:53) (non-Kabat) or SYYMH (SEQ ID NO:54) | EIVLTQSPATLSLSPGERATLSCRASQ SVSSYLAWYQQKPGQAPRLLIYDAS NRATGIPARFSGSGSGTDFTLTISSLE PEDFAVYYCQQSDNWPFTFGGGTK VEIK (SEQ ID NO:92) CDR1 (SEQ ID NO:93) - RASQSVSSYLA |

| Table 1 | | |
| --- | --- | --- |
| **Clones** | **Heavy chain variable region amino acid sequence** | **Light chain variable region amino acid sequence** |
| | CDR2 (SEQ ID NO:55) - IINPSGGSTSYAQKFQG<br><br>CDR3 - AREGAGFAYGMDYYYMDV (SEQ ID NO:90) (non-Kabat) or EGAGFAYGMDYYYMDV (SEQ ID NO:91) | CDR2 (SEQ ID NO:44) - DASNRAT<br>CDR3 (SEQ ID NO:94) - QQSDNWPFT |
| A49MI | EVQLVESGGGLVKPGGSLRLSCAASGFT<br>FSSYSMNWVRQAPGKGLEWVSSISSSSS<br>YIYYADSVKGRFTISRDNAKNSLYLQMN<br>SLRAEDTAVYYCARGAP**I**GAAAGWFDP<br>WGQGTLVTVSS (SEQ ID NO:95)<br><br>CDR1: FTFSSYSMN (SEQ ID NO:80) (non-Kabat) or SYSMN (SEQ ID NO:81)<br>CDR2: SISSSSSYIYYADSVKG (SEQ ID NO:82)<br>CDR3: ARGAPIGAAAGWFDP (SEQ ID NO:96) (non-Kabat) or GAPIGAAAGWFDP (SEQ ID NO:97) | DIQMTQSPSSVSASVGDRVTITCRAS<br>QGISSWLAWYQQKPGKAPKLLIYAA<br>SSLQSGVPSRFSGSGSGTDFTLTISSL<br>QPEDFATYYCQQGVSFPRTFGGGTK<br>VEIK<br>(SEQ ID NO:85)<br>CDR1 (SEQ ID NO:86) -<br><br>RASQGISSWLA CDR2 (SEQ ID NO:77) - AASSLQS<br>CDR3 (SEQ ID NO:87) - QQGVSFPRT |
| A49MQ | EVQLVESGGGLVKPGGSLRLSCAASGFT<br>FSSYSMNWVRQAPGKGLEWVSSISSSSS<br>YIYYADSVKGRFTISRDNAKNSLYLQMN<br>SLRAEDTAVYYCARGAP**Q**GAAAGWFDP<br>WGQGTLVTVSS<br>(SEQ ID NO:98)<br>CDR1: FTFSSYSMN (SEQ ID NO:80) (non-Kabat) or SYSMN (SEQ ID NO:81)<br>CDR2: SISSSSSYIYYADSVKG (SEQ ID NO:82)<br><br>CDR3 - ARGAPQGAAAGWFDP (SEQ ID NO:99) (non-Kabat) or GAPQGAAAGWFDP (SEQ ID NO:100) | DIQMTQSPSSVSASVGDRVTITCRAS<br>QGISSWLAWYQQKPGKAPKLLIYAA<br>SSLQSGVPSRFSGSGSGTDFTLTISSL<br>QPEDFATYYCQQGVSFPRTFGGGTK<br>VEIK<br>(SEQ ID NO:85)<br>CDR1 (SEQ ID NO:86) - RASQGISSWLA<br><br>CDR2 (SEQ ID NO:77) - AASSLQS<br>CDR3 (SEQ ID NO:87) - QQGVSFPRT |

EP 4 192 872 B1

| Table 1 | | |
|---|---|---|
| **Clones** | **Heavy chain variable region amino acid sequence** | **Light chain variable region amino acid sequence** |
| A49ML | EVQLVESGGGLVKPGGSLRLSCAASGFT<br><br>FSSYSMNWVRQAPGKGLEWVSSISSSSS<br><br>YIYYADSVKGRFTISRDNAKNSLYLQMN<br><br>SLRAEDTAVYYCARGAP**L**GAAAGWFDP<br><br>WGQGTLVTVSS<br><br>(SEQ ID NO:101)<br><br>CDR1: FTFSSYSMN (SEQ ID NO:80) (non-Kabat) or SYSMN (SEQ ID NO:81)<br>CDR2: SISSSSSYIYYADSVKG (SEQ ID NO:82)<br><br>CDR3 - ARGAPLGAAAGWFDP (SEQ ID NO:102) (non-Kabat) or GAPLGAAAGWFDP (SEQ ID NO:103) | DIQMTQSPSSVSASVGDRVTITCRAS<br><br>QGISSWLAWYQQKPGKAPKLLIYAA<br><br>SSLQSGVPSRFSGSGSGTDFTLTISSL<br><br>QPEDFATYYCQQGVSFPRTFGGGTK<br><br>VEIK<br><br>(SEQ ID NO:85)<br><br>CDR1 (SEQ ID NO:86) - RASQGISSWLA<br><br>CDR2 (SEQ ID NO:77) - AASSLQS<br>CDR3 (SEQ ID NO:87) - QQGVSFPRT |
| A49MF | EVQLVESGGGLVKPGGSLRLSCAASGFT<br><br>FSSYSMNWVRQAPGKGLEWVSSISSSSS<br><br>YIYYADSVKGRFTISRDNAKNSLYLQMN<br><br>SLRAEDTAVYYCARGAP**F**GAAAGWFDP<br><br>WGQGTLVTVSS<br><br>(SEQ ID NO:104)<br><br>CDR1: FTFSSYSMN (SEQ ID NO:80) (non-Kabat) or SYSMN (SEQ ID NO:81)<br>CDR2: SISSSSSYIYYADSVKG (SEQ ID NO:82)<br><br>CDR3 - ARGAPFGAAAGWFDP (SEQ ID NO:105) (non-Kabat) or GAPFGAAAGWFDP (SEQ ID NO:106) | DIQMTQSPSSVSASVGDRVTITCRAS<br><br>QGISSWLAWYQQKPGKAPKLLIYAA<br><br>SSLQSGVPSRFSGSGSGTDFTLTISSL<br><br>QPEDFATYYCQQGVSFPRTFGGGTK<br><br>VEIK<br><br>(SEQ ID NO:85)<br><br>CDR1 (SEQ ID NO:86) - RASQGISSWLA<br><br>CDR2 (SEQ ID NO:77) - AASSLQS<br>CDR3 (SEQ ID NO:87) - QQGVSFPRT |

| Table 1 | | |
| --- | --- | --- |
| **Clones** | **Heavy chain variable region amino acid sequence** | **Light chain variable region amino acid sequence** |
| A49MV | EVQLVESGGGLVKPGGSLRLSCAASGFT<br><br>FSSYSMNWVRQAPGKGLEWVSSISSSSS<br><br>YIYYADSVKGRFTISRDNAKNSLYLQMN<br><br>SLRAEDTAVYYCARGAP**V**GAAAGWFDP<br><br>WGQGTLVTVSS<br><br>(SEQ ID NO:107)<br><br>CDR1: FTFSSYSMN (SEQ ID NO:80) (non-Kabat) or SYSMN (SEQ ID NO:81)<br>CDR2: SISSSSSYIYYADSVKG (SEQ ID NO:82)<br><br>CDR3- ARGAP**V**GAAAGWFDP (SEQ ID NO:108) (non-Kabat) or GAPVGAAAGWFDP (SEQ ID NO:109) | DIQMTQSPSSVSASVGDRVTITCRAS<br><br>QGISSWLAWYQQKPGKAPKLLIYAA<br><br>SSLQSGVPSRFSGSGSGTDFTLTISSL<br><br>QPEDFATYYCQQGVSFPRTFGGGTK<br><br>VEIK<br><br>(SEQ ID NO:85)<br><br>CDR1 (SEQ ID NO:86) - RASQGISSWLA<br><br>CDR2 (SEQ ID NO:77) - AASSLQS<br>CDR3 (SEQ ID NO:87) - QQGVSFPRT |
| A49-consensus | EVQLVESGGGLVKPGGSLRLSCAASGFT<br><br>FSSYSMNWVRQAPGKGLEWVSSISSSSS<br><br>YIYYADSVKGRFTISRDNAKNSLYLQMN<br><br>SLRAEDTAVYYCARGAP**X**GAAAGWFDP<br><br>WGQGTLVTVSS, wherein X is M, L, I, V,<br><br>Q, or F<br><br>(SEQ ID NO:110)<br><br>CDR1: FTFSSYSMN (SEQ ID NO:80) (non-Kabat) or SYSMN (SEQ ID NO:81)<br>CDR2: SISSSSSYIYYADSVKG (SEQ ID NO:82)<br>CDR3- ARGAP**X**GAAAGWFDP (SEQ ID NO:111) (non-Kabat) or GAP**X**-GAAAGWFDP (SEQ ID NO:112), wherein X is M, L, I, V, Q, or F | DIQMTQSPSSVSASVGDRVTITCRAS<br><br>QGISSWLAWYQQKPGKAPKLLIYAA<br><br>SSLQSGVPSRFSGSGSGTDFTLTISSL<br><br>QPEDFATYYCQQGVSFPRTFGGGTK<br><br>VEIK<br><br>(SEQ ID NO:85)<br><br><br>CDR1 (SEQ ID NO:86) - RASQGISSWLA<br>CDR2 (SEQ ID NO:77) - AASSLQS<br>CDR3 (SEQ ID NO:87) - QQGVSFPRT |

(continued)

| Clones | Heavy chain variable region amino acid sequence | Light chain variable region amino acid sequence |
|---|---|---|
| **Table 1** | | |
| NKG2D binder in US 9,273,136 | QVQLVESGGGLVKPGGSLRLSCAASGFT FSSYGMHWVRQAPGKGLEWVAFIRYDG SNKYYADSVKGRFTISRDNSKNTLYLQM NSLRAEDTAVYYCAKDRGLGDGTYFDY WGQGTTVTVSS (SEQ ID NO:113) | QSALTQPASVSGSPGQSITISCSGSSS NIGNNAVNWYQQLPGKAPKLLIYY DDLLPSGVSDRFSGSKSGTSAFLAIS GLQSEDEADYYCAAWDDSLNGPVF GGGTKLTVL (SEQ ID NO:114) |
| NKG2D binder in US 7,879,985 | QVHLQESGPGLVKPSETLSLTCTVSDDSI SSYYWSWIRQPPGKGLEWIGHISYSGSA NYNPSLKSRVTISVDTSKNQFSLKLSSVT AADTAVYYCANWDDAFNIWGQGTMVT VSS (SEQ ID NO:116) | EIVLTQSPGTLSLSPGERATLSCRASQ SVSSSYLAWYQQKPGQAPRLLIYGA SSRATGIPDRFSGSGSGTDFTLTISRL EPEDFAVYYCQQYGSSPWTFGQGTK VEIK (SEQ ID NO:117) |

28

EP 4 192 872 B1

In certain embodiments, the first antigen-binding site that binds NKG2D (*e.g.,* human NKG2D) comprises an antibody heavy chain variable domain (VH) that comprises an amino acid sequence at least 90% (*e.g.,* at least 91%, at least 92%, at least 93%, at least 94%, at least 95%, at least 96%, at least 97%, at least 98%, at least 99%, or 100%) identical to the VH of an antibody disclosed in **Table 1,** and an antibody light chain variable domain (VL) that comprises an amino acid sequence at least 90% (*e.g.,* at least 91%, at least 92%, at least 93%, at least 94%, at least 95%, at least 96%, at least 97%, at least 98%, at least 99%, or 100%) identical to the VL of the same antibody disclosed in **Table** 1. In certain embodiments, the first antigen-binding site comprises the heavy chain CDR1, CDR2, and CDR3 and the light chain CDR1, CDR2, and CDR3, determined under Kabat (*see* Kabat et al., (1991) Sequences of Proteins of Immunological Interest, NIH Publication No. 91-3242, Bethesda), Chothia (see, *e.g.,* Chothia C & Lesk A M, (1987), J. Mol. Biol. 196: 901-917), MacCallum (*see* MacCallum R M et al., (1996) J. Mol. Biol. 262: 732-745), or any other CDR determination method known in the art, of the VH and VL sequences of an antibody discloses in **Table 1.** In certain embodiments, the first antigen-binding site comprises the heavy chain CDR1, CDR2, and CDR3 and the light chain CDR1, CDR2, and CDR3 of an antibody disclosed in **Table 1.**

In certain embodiments, the first antigen-binding site that binds to NKG2D comprises a heavy chain variable domain derived from SEQ ID NO: 1, such as by having an amino acid sequence at least 90% (*e.g.,* at least 90%, at least 91%, at least 92%, at least 93%, at least 94%, at least 95%, at least 96%, at least 97%, at least 98%, at least 99%, or 100%) identical to SEQ ID NO: 1, and/or incorporating amino acid sequences identical to the CDR1 (SEQ ID NO:2), CDR2 (SEQ ID NO:3), and CDR3 (SEQ ID NO:4) sequences of SEQ ID NO: 1. The heavy chain variable domain related to SEQ ID NO: 1 can be coupled with a variety of light chain variable domains to form an NKG2D binding site. For example, the first antigen-binding site that incorporates a heavy chain variable domain related to SEQ ID NO: 1 can further incorporate a light chain variable domain selected from the sequences derived from SEQ ID NOs: 5, 6, 7, 8, 9, 12, 13, 14, 15, 16, 17, 18, 19, 20, 21, 22, 23, 24, 25, and 46. For example, the first antigen-binding site incorporates a heavy chain variable domain with amino acid sequences at least 90% (*e.g.,* at least 90%, at least 91%, at least 92%, at least 93%, at least 94%, at least 95%, at least 96%, at least 97%, at least 98%, at least 99%, or 100%) identical to SEQ ID NO: 1 and a light chain variable domain with amino acid sequences at least 90% (*e.g.,* at least 90%, at least 91%, at least 92%, at least 93%, at least 94%, at least 95%, at least 96%, at least 97%, at least 98%, at least 99%, or 100%) identical to any one of the sequences selected from SEQ ID NOs: 5, 6, 7, 8, 9, 12, 13, 14, 15, 16, 17, 18, 19, 20, 21, 22, 23, 24, 25, and 46.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:26, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:32. In certain embodiments, the VH comprises CDR1, CDR2, and CDR3 comprising the amino acid sequences of SEQ ID NOs: 27 or 28, 29, and 30 or 31, respectively (e.g., SEQ ID NOs: 27, 29, and 30, respectively, or SEQ ID NOs: 28, 29, and 31, respectively). In certain embodiments, the VL comprises CDR1, CDR2, and CDR3 comprising the amino acid sequences of SEQ ID NOs: 33, 34, and 35, respectively. In certain embodiments, the first antigen-binding site comprises (a) a VH that comprises CDR1, CDR2, and CDR3 comprising the amino acid sequences of SEQ ID NOs: 27 or 28, 29, and 30 or 31, respectively (*e.g.,* SEQ ID NOs: 27, 29, and 30, respectively, or SEQ ID NOs: 28, 29, and 31, respectively); and (b) a VL that comprises CDR1, CDR2, and CDR3 comprising the amino acid sequences of SEQ ID NOs: 33, 34, and 35, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:36, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:42. In certain embodiments, the VH comprises CDR1, CDR2, and CDR3 comprising the amino acid sequences of SEQ ID NOs: 37 or 38, 39, and 40 or 41, respectively (*e.g.,* SEQ ID NOs: 37, 39, and 40, respectively, or SEQ ID NOs: 38, 39, and 41, respectively). In certain embodiments, the VL comprises CDR1, CDR2, and CDR3 comprising the amino acid sequences of SEQ ID NOs: 43, 44, and 45, respectively. In certain embodiments, the first antigen-binding site comprises (a) a VH that comprises CDR1, CDR2, and CDR3 comprising the amino acid sequences of SEQ ID NOs: 37 or 38, 39, and 40 or 41, respectively (*e.g.,* SEQ ID NOs: 37, 39, and 40, respectively, or SEQ ID NOs: 38, 39, and 41, respectively); and (b) a VL that comprises CDR1, CDR2, and CDR3 comprising the amino acid sequences of SEQ ID NOs: 43, 44, and 45, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:47, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:49. In certain embodiments, the VH comprises CDR1, CDR2, and CDR3 comprising the amino acid sequences of SEQ ID NOs: 27, 29, and 48, respectively. In certain embodiments, the VL comprises CDR1, CDR2, and CDR3 comprising the amino acid sequences of SEQ ID NOs:

50, 34, and 51, respectively. In certain embodiments, the first antigen-binding site comprises (a) a VH that comprises CDR1, CDR2, and CDR3 comprising the amino acid sequences of SEQ ID NOs: 27, 29, and 48, respectively; and (b) a VL that comprises CDR1, CDR2, and CDR3 comprising the amino acid sequences of SEQ ID NOs: 50, 34, and 51, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:52, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:58. In certain embodiments, the VH comprises CDR1, CDR2, and CDR3 comprising the amino acid sequences of SEQ ID NOs: 53 or 54, 55, and 56 or 57, respectively (*e.g.,* SEQ ID NOs: 53, 55, and 56, respectively, or SEQ ID NOs: 54, 55, and 57, respectively). In certain embodiments, the VL comprises CDR1, CDR2, and CDR3 comprising the amino acid sequences of SEQ ID NOs: 59, 60, and 61, respectively. In certain embodiments, the first antigen-binding site comprises (a) a VH that comprises CDR1, CDR2, and CDR3 comprising the amino acid sequences of SEQ ID NOs: 53 or 54, 55, and 56 or 57, respectively (e.g., SEQ ID NOs: 53, 55, and 56, respectively, or SEQ ID NOs: 54, 55, and 57, respectively); and (b) a VL that comprises CDR1, CDR2, and CDR3 comprising the amino acid sequences of SEQ ID NOs: 59, 60, and 61, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:62, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:68. In certain embodiments, the VH comprises CDR1, CDR2, and CDR3 comprising the amino acid sequences of SEQ ID NOs: 63 or 64, 65, and 66 or 67, respectively (*e.g.,* SEQ ID NOs: 63, 65, and 66, respectively, or SEQ ID NOs: 64, 65, and 67, respectively). In certain embodiments, the VL comprises CDR1, CDR2, and CDR3 comprising the amino acid sequences of SEQ ID NOs: 59, 60, and 69, respectively. In certain embodiments, the first antigen-binding site comprises (a) a VH that comprises CDR1, CDR2, and CDR3 comprising the amino acid sequences of SEQ ID NOs: 63 or 64, 65, and 66 or 67, respectively (e.g., SEQ ID NOs: 63, 65, and 66, respectively, or SEQ ID NOs: 64, 65, and 67, respectively); and (b) a VL that comprises CDR1, CDR2, and CDR3 comprising the amino acid sequences of SEQ ID NOs: 59, 60, and 69, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:89, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:92. In certain embodiments, the VH comprises CDR1, CDR2, and CDR3 comprising the amino acid sequences of SEQ ID NOs: 53 or 54, 55, and 90 or 91, respectively (*e.g.,* SEQ ID NOs: 53, 55, and 90, respectively, or SEQ ID NOs: 54, 55, and 91, respectively). In certain embodiments, the VL comprises CDR1, CDR2, and CDR3 comprising the amino acid sequences of SEQ ID NOs: 93, 44, and 94, respectively. In certain embodiments, the first antigen-binding site comprises (a) a VH that comprises CDR1, CDR2, and CDR3 comprising the amino acid sequences of SEQ ID NOs: 53 or 54, 55, and 90 or 91, respectively (*e.g.,* SEQ ID NOs: 53, 55, and 90, respectively, or SEQ ID NOs: 54, 55, and 91, respectively); and (b) a VL that comprises CDR1, CDR2, and CDR3 comprising the amino acid sequences of SEQ ID NOs: 93, 44, and 94, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:70, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:75. In certain embodiments, the VH comprises CDR1, CDR2, and CDR3 comprising the amino acid sequences of SEQ ID NOs: 71 or 115, 72, and 73 or 74, respectively (*e.g.,* SEQ ID NOs: 71, 72, and 73, respectively, or SEQ ID NOs: 115, 72, and 74, respectively). In certain embodiments, the VL comprises CDR1, CDR2, and CDR3 comprising the amino acid sequences of SEQ ID NOs: 76, 77, and 78, respectively. In certain embodiments, the first antigen-binding site comprises (a) a VH that comprises CDR1, CDR2, and CDR3 comprising the amino acid sequences of SEQ ID NOs: 71 or 115, 72, and 73 or 74, respectively (*e.g.,* SEQ ID NOs: 71, 72, and 73, respectively, or SEQ ID NOs: 115, 72, and 74, respectively); and (b) a VL that comprises CDR1, CDR2, and CDR3 comprising the amino acid sequences of SEQ ID NOs: 76, 77, and 78, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:79, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:85. In certain embodiments, the VH comprises CDR1, CDR2, and CDR3 comprising the amino acid sequences of SEQ ID NOs: 80 or 81, 82, and 83 or 84,

respectively (*e.g.,* SEQ ID NOs: 80, 82, and 83, respectively, or SEQ ID NOs: 81, 82, and 84, respectively). In certain embodiments, the VL comprises CDR1, CDR2, and CDR3 comprising the amino acid sequences of SEQ ID NOs: 86, 77, and 87, respectively. In certain embodiments, the first antigen-binding site comprises (a) a VH that comprises CDR1, CDR2, and CDR3 comprising the amino acid sequences of SEQ ID NOs: 80 or 81, 82, and 83 or 84 respectively (*e.g.,* SEQ ID NOs: 80, 82, and 83, respectively, or SEQ ID NOs: 81, 82, and 84, respectively); and (b) a VL that comprises CDR1, CDR2, and CDR3 comprising the amino acid sequences of SEQ ID NOs: 86, 77, and 87, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:95, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:85. In certain embodiments, the VH comprises CDR1, CDR2, and CDR3 comprising the amino acid sequences of SEQ ID NOs: 80 or 81, 82, and 96 or 97, respectively (*e.g.,* SEQ ID NOs: 80, 82, and 96, respectively, or SEQ ID NOs: 81, 82, and 97, respectively). In certain embodiments, the VL comprises CDR1, CDR2, and CDR3 comprising the amino acid sequences of SEQ ID NOs: 86, 77, and 87, respectively. In certain embodiments, the first antigen-binding site comprises (a) a VH that comprises CDR1, CDR2, and CDR3 comprising the amino acid sequences of SEQ ID NOs: 80 or 81, 82, and 96 or 97, respectively (*e.g.,* SEQ ID NOs: 80, 82, and 96, respectively, or SEQ ID NOs: 81, 82, and 97, respectively); and (b) a VL that comprises CDR1, CDR2, and CDR3 comprising the amino acid sequences of SEQ ID NOs: 86, 77, and 87, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:98, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:85. In certain embodiments, the VH comprises CDR1, CDR2, and CDR3 comprising the amino acid sequences of SEQ ID NOs: 80 or 81, 82, and 99 or 100, respectively (*e.g.,* SEQ ID NOs: 80, 82, and 99, respectively, or SEQ ID NOs: 81, 82, and 100, respectively). In certain embodiments, the VL comprises CDR1, CDR2, and CDR3 comprising the amino acid sequences of SEQ ID NOs: 86, 77, and 87, respectively. In certain embodiments, the first antigen-binding site comprises (a) a VH that comprises CDR1, CDR2, and CDR3 comprising the amino acid sequences of SEQ ID NOs: 80 or 81, 82, and 99 or 100, respectively (*e.g.,* SEQ ID NOs: 80, 82, and 99, respectively, or SEQ ID NOs: 81, 82, and 100, respectively); and (b) a VL that comprises CDR1, CDR2, and CDR3 comprising the amino acid sequences of SEQ ID NOs: 86, 77, and 87, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:101, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:85. In certain embodiments, the VH comprises CDR1, CDR2, and CDR3 comprising the amino acid sequences of SEQ ID NOs: 80 or 81, 82, and 102 or 103, respectively (e.g., SEQ ID NOs: 80, 82, and 102, respectively, or SEQ ID NOs: 81, 82, and 103, respectively). In certain embodiments, the VL comprises CDR1, CDR2, and CDR3 comprising the amino acid sequences of SEQ ID NOs: 86, 77, and 87, respectively. In certain embodiments, the first antigen-binding site comprises (a) a VH that comprises CDR1, CDR2, and CDR3 comprising the amino acid sequences of SEQ ID NOs: 80 or 81, 82, and 102 or 103, respectively (e.g., SEQ ID NOs: 80, 82, and 102, respectively, or SEQ ID NOs: 81, 82, and 103, respectively); and (b) a VL that comprises CDR1, CDR2, and CDR3 comprising the amino acid sequences of SEQ ID NOs: 86, 77, and 87, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:104, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:85. In certain embodiments, the VH comprises CDR1, CDR2, and CDR3 comprising the amino acid sequences of SEQ ID NOs: 80 or 81, 82, and 105 or 106, respectively (*e.g.,* SEQ ID NOs: 80, 82, and 105, respectively, or SEQ ID NOs: 81, 82, and 106, respectively). In certain embodiments, the VL comprises CDR1, CDR2, and CDR3 comprising the amino acid sequences of SEQ ID NOs: 86, 77, and 87, respectively. In certain embodiments, the first antigen-binding site comprises (a) a VH that comprises CDR1, CDR2, and CDR3 comprising the amino acid sequences of SEQ ID NOs: 80 or 81, 82, and 105 or 106, respectively (*e.g.,* SEQ ID NOs: 80, 82, and 105, respectively, or SEQ ID NOs: 81, 82, and 106, respectively); and (b) a VL that comprises CDR1, CDR2, and CDR3 comprising the amino acid sequences of SEQ ID NOs: 86, 77, and 87, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:107, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:85. In certain embodiments, the VH comprises CDR1, CDR2, and CDR3 comprising the amino acid sequences of SEQ ID NOs: 80 or 81, 82, and 108 or 109, respectively (*e.g.,* SEQ ID NOs: 80, 82, and 108, respectively, or SEQ ID NOs: 81, 82, and 109, respectively). In certain embodiments, the VL comprises CDR1, CDR2, and CDR3 comprising the amino acid sequences of SEQ ID NOs: 86, 77, and 87, respectively. In certain embodiments, the first antigen-binding site comprises (a) a VH that comprises CDR1, CDR2, and CDR3 comprising the amino acid sequences of SEQ ID NOs: 80 or 81, 82, and 108 or 109, respectively (*e.g.*, SEQ ID NOs: 80, 82, and 108, respectively, or SEQ ID NOs: 81, 82, and 109, respectively); and (b) a VL that comprises CDR1, CDR2, and CDR3 comprising the amino acid sequences of SEQ ID NOs: 86, 77, and 87, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:110, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:85. In certain embodiments, the VH comprises CDR1, CDR2, and CDR3 comprising the amino acid sequences of SEQ ID NOs: 80 or 81, 82, and 111 or 112, respectively (*e.g.,* SEQ ID NOs: 80, 82, and 111, respectively, or SEQ ID NOs: 81, 82, and 112, respectively). In certain embodiments, the VL comprises CDR1, CDR2, and CDR3 comprising the amino acid sequences of SEQ ID NOs: 86, 77, and 87, respectively. In certain embodiments, the first antigen-binding site comprises (a) a VH that comprises CDR1, CDR2, and CDR3 comprising the amino acid sequences of SEQ ID NOs: 80 or 81, 82, and 111 or 112, respectively (*e.g.,* SEQ ID NOs: 80, 82, and 111, respectively, or SEQ ID NOs: 81, 82, and 112, respectively); and (b) a VL that comprises CDR1, CDR2, and CDR3 comprising the amino acid sequences of SEQ ID NOs: 86, 77, and 87, respectively.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:113, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO: 114.

In certain embodiments, the first antigen-binding site that binds NKG2D comprises a VH that comprises an amino acid sequence at least 90% (*e.g.,* at least 91%, at least 92%, at least 93%, at least 94%, at least 95%, at least 96%, at least 97%, at least 98%, at least 99%, or 100%) identical to the amino acid sequence of SEQ ID NO:116, and a VL that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO: 117.

The multispecific binding proteins can bind to NKG2D-expressing cells, which include but are not limited to NK cells, $\gamma\delta$ T cells and $CD8^+$ $\alpha\beta$ T cells. Upon NKG2D binding, the multispecific binding proteins may block natural ligands, such as ULBP6 and MICA, from binding to NKG2D and activating NK cells.

The multispecific binding proteins binds to cells expressing CD16, an Fc receptor on the surface of leukocytes including natural killer cells, macrophages, neutrophils, eosinophils, mast cells, and follicular dendritic cells. A protein described in the present disclosure binds to NKG2D with an affinity of $K_D$ of 2 nM to 120 nM, *e.g.,* 2 nM to 110 nM, 2 nM to 100 nM, 2 nM to 90 nM, 2 nM to 80 nM, 2 nM to 70 nM, 2 nM to 60 nM, 2 nM to 50 nM, 2 nM to 40 nM, 2 nM to 30 nM, 2 nM to 20 nM, 2 nM to 10 nM, about 15 nM, about 14 nM, about 13 nM, about 12 nM, about 11 nM, about 10 nM, about 9 nM, about 8 nM, about 7 nM, about 6 nM, about 5 nM, about 4.5 nM, about 4 nM, about 3.5 nM, about 3 nM, about 2.5 nM, about 2 nM, about 1.5 nM, about 1 nM, between about 0.5 nM to about 1 nM, about 1 nM to about 2 nM, about 2 nM to 3 nM, about 3 nM to 4 nM, about 4 nM to about 5 nM, about 5 nM to about 6 nM, about 6 nM to about 7 nM, about 7 nM to about 8 nM, about 8 nM to about 9 nM, about 9 nM to about 10 nM, about 1 nM to about 10 nM, about 2 nM to about 10 nM, about 3 nM to about 10 nM, about 4 nM to about 10 nM, about 5 nM to about 10 nM, about 6 nM to about 10 nM, about 7 nM to about 10 nM, or about 8 nM to about 10 nM. In some embodiments, NKG2D-binding sites bind to NKG2D with a $K_D$ of 10 to 62 nM.

### EGFR Binding Site

In one aspect, the present disclosure provides multispecific binding proteins that bind to the NKG2D receptor and CD16 receptor on natural killer cells, and EGFR, wherein the EGFR binding site comprises a heavy chain variable domain (VH) and a light chain variable domain (VL) derived from panitumumab and having mutations in the VH and VL. Mutations that are contemplated, individually or in combination, in the VH and VL sequences described in the present disclosure include S62R in the VH, D92R in the VL, and/or F87Y in the VL, under the Chothia numbering scheme. It has been discovered that these mutations increase thermostability of the antigen-binding site and retain its affinity to EGFR. **Table 2** lists some exemplary sequences of heavy chain variable domains, light chain variable domains, and scFv sequences that can bind to EGFR. CDR sequences are identified under Chothia numbering as indicated. Residues in **bold** indicate mutated residues and *italicized* sequence indicates a polypeptide linker.

**Table 2.** Sequences of Exemplary Antigen-Binding Sites that Bind EGFR

| EGFR binder | Heavy chain variable domain amino acid sequence | Light chain variable domain amino acid sequence |
|---|---|---|
| EGFR-binder-1 (panitu-mumab) | QVQLQESGPGLVKPSETLSLTC TVSGGSVSSGDYYWTWIRQSP GKGLEWIGHIYYSGNTNYNPS LKSRLTISIDTSKTQFSLKLSSV TAADTAIYYCVRDRVTGAFDI WGQGTMVTVSS (SEQ ID NO:135)<br><br>CDR1: GGSVSSGDYYWT (SEQ ID NO:136)<br>CDR2: HIYYSGNTNYNPSLKS (SEQ ID NO:137)<br>CDR3: DRVTGAFDI (SEQ ID NO:138) | DIQMTQSPSSLSASVGDRVTI TCQASQDISNYLNWYQQKPG KAPKLLIYDASNLETGVPSRF SGSGSGTDFTFTISSLQPEDIA TYFCQHFDHLPLAFGGGTKV EIK (SEQ ID NO:139)<br><br>CDR1: QASQDISNYLN (SEQ ID NO:140)<br>CDR2: LLIYDASNLET (SEQ ID NO:141)<br>CDR3: QHFDHLPLA (SEQ ID NO: 142) |
| EGFR-scFv-1 | EGFR-scFv-1 (VL-VH):<br><br>DIQMTQSPSSLSASVGDRVTITCQASQDISNYLNWYQQKPGKAP KLLIYDASNLETGVPSRFSGSGSGTDFTFTISSLQPEDIATYFCQH FDHLPLAFG**C**GTKVEIK*GGGGSGGGGSGGGGSGGGGS*QVQLQ ESGPGLVKPSETLSLTCTVSGGSVSSGDYYWTWIRQSPGK**C**LE WIGHIYYSGNTNYNPSLKSRLTISIDTSKTQFSLKLSSVTAADTAI YYCVRDRVTGAFDIWGQGTMVTVSS (SEQ ID NO:143)<br><br>EGFR-scFv-1 (VH-VL):<br>QVQLQESGPGLVKPSETLSLTCTVSGGSVSSGDYYWTWIRQSP GK**C**LEWIGHIYYSGNTNYNPSLKSRLTISIDTSKTQFSLKLSSVT AADTAIYYCVRDRVTGAFDIWGQGTMVTVSS*GGGGSGGGGSG GGGSGGGGS*DIQMTQSPSSLSASVGDRVTITCQASQDISNYLNW YQQKPGKAPKLLIYDASNLETGVPSRFSGSGSGTDFTFTISSLQP EDIATYFCQHFDHLPLAFG**C**GTKVEIK (SEQ ID NO:144) | |
| EGFR-binder-2 | QVQLQESGPGLVKPSETLSLTC TVSGGSVSSGDYYWTWIRQSP | DIQMTQSPSSLSASVGDRVTI TCQASQDISNYLNWYQQKPG |

33

(continued)

| EGFR binder | Heavy chain variable domain amino acid sequence | Light chain variable domain amino acid sequence |
|---|---|---|
| | **GK**GLEWIGHIYYSGNTNYNP**R**LKSRLTISIDTSKTQFSLKLSSVTAADTAIYYCVRDRVTGAFDIWGQGTMVTVSS (SEQ ID NO:145)<br><br>CDR1: GGSVSSGDYYWT (SEQ ID NO:136)<br><br>CDR2: HIYYSGNTNYNPRLKS (SEQ ID NO:146)<br><br>CDR3: DRVTGAFDI (SEQ ID NO:138) | KAPKLLIYDASNLETGVPSRFSGSGSGTDFTFTISSLQPEDIATY**Y**CQHFDHLPLAFGGGTKVEIK (SEQ ID NO:147)<br><br><br>CDR1: QASQDISNYLN (SEQ ID NO:140)<br>CDR2: LLIYDASNLET (SEQ ID NO:141)<br><br>DR3: QHFDHLPLA (SEQ ID NO: 142) |
| EGFR-scFv-2 | EGFR-scFv-2 (VL-VH):<br><br>DIQMTQSPSSLSASVGDRVTITCQASQDISNYLNWYQQKPGKAPKLLIYDASNLETGVPSRFSGSGSGTDFTFTISSLQPEDIATY**Y**CQHFDHLPLAFG**C**GTKVEIK*GGGGSGGGGSGGGGSGGGGS*QVQLQESGPGLVKPSETLSLTCTVSGGSVSSGDYYWTWIRQSPGK**C**LEWIGHIYYSGNTNYNP**R**LKSRLTISIDTSKTQFSLKLSSVTAADTAIYYCVRDRVTGAFDIWGQGTMVTVSS (SEQ ID NO:148)<br><br>EGFR-scFv-2 (VH-VL):<br><br>QVQLQESGPGLVKPSETLSLTCTVSGGSVSSGDYYWTWIRQSPGK**C**LEWIGHIYYSGNTNYNP**R**LKSRLTISIDTSKTQFSLKLSSVTAADTAIYYCVRDRVTGAFDIWGQGTMVTVSS*GGGGSGGGGSGGGSGGGGS*DIQMTQSPSSLSASVGDRVTITCQASQDISNYLNWYQQKPGKAPKLLIYDASNLETGVPSRFSGSGSGTDFTFTISSLQPEDIATY**Y**CQHFDHLPLAFG**C**GTKVEIK (SEQ ID NO:149) | |

EP 4 192 872 B1

34

(continued)

| EGFR binder | Heavy chain variable domain amino acid sequence | Light chain variable domain amino acid sequence |
|---|---|---|
| EGFR-binder-3 | QVQLQESGPGLVKPSETLSLTC<br>TVSGGSVSSGDYYWTWIRQSP<br>GKGLEWIGHIYYSGNTNYNP**R**<br>LKSRLTISIDTSKTQFSLKLSSV<br>TAADTAIYYCVRDRVTGAFDI<br>WGQGTMVTVSS (SEQ ID<br>NO:145)<br><br>CDR1: GGSVSSGDYYWT (SEQ ID NO:136)<br><br>CDR2: HIYYSGNTNYNPRLKS (SEQ ID NO:146)<br>CDR3: DRVTGAFDI (SEQ ID NO:138) | DIQMTQSPSSLSASVGDRVTI<br>TCQASQDISNYLNWYQQKPG<br>KAPKLLIYDASNLETGVPSRF<br>SGSGSGTDFTFTISSLQPEDIA<br>TY**Y**CQHF**R**HLPLAFGGGTK<br>VEIK (SEQ ID NO:150)<br><br>CDR1: QASQDISNYLN (SEQ ID NO:140)<br>CDR2: LLIYDASNLET (SEQ ID NO:141)<br><br>CDR3: QHFRHLPLA (SEQ ID NO:151) |
| EGFR-binder-3 (scFv mu-tations) | QVQLQESGPGLVKPSETLSLTC<br>TVSGGSVSSGDYYWTWIRQSP<br>GK**C**LEWIGHIYYSGNTNYNP**R**<br>LKSRLTISIDTSKTQFSLKLSSV<br>TAADTAIYYCVRDRVTGAFDI<br><br>WGQGTMVTVSS (SEQ ID<br>NO:170)<br><br>CDR1: GGSVSSGDYYWT (SEQ ID NO:136)<br>CDR2: HIYYSGNTNYNPRLKS (SEQ ID NO:146)<br>CDR3: DRVTGAFDI (SEQ ID NO:138) | DIQMTQSPSSLSASVGDRVTI<br>TCQASQDISNYLNWYQQKPG<br>KAPKLLIYDASNLETGVPSRF<br>SGSGSGTDFTFTISSLQPEDIA<br>TY**Y**CQHF**R**HLPLAFG**C**GTK<br>VEIK (SEQ ID NO:171)<br>CDR1: QASQDISNYLN (SEQ ID NO:140)<br><br><br>CDR2: LLIYDASNLET (SEQ ID NO:141)<br>CDR3: QHFRHLPLA (SEQ ID NO:151) |
| EGFR-scFv-3 | EGFR-scFv-3 (VL-VH): | |

| EGFR binder | Heavy chain variable domain amino acid sequence | Light chain variable domain amino acid sequence |
|---|---|---|
| | DIQMTQSPSSLSASVGDRVTITCQASQDISNYLNWYQQKPGKAP KLLIYDASNLETGVPSRFSGSGSGTDFTFTISSLQPEDIATY**Y**CQ HF**R**HLPLAFG**C**GTKVEIK*GGGGSGGGGSGGGGSGGGGS*QVQL QESGPGLVKPSETLSLTCTVSGGSVSSGDYYWTWIRQSPGK**C**L EWIGHIYYSGNTNYNP**R**LKSRLTISIDTSKTQFSLKLSSVTAADT AIYYCVRDRVTGAFDIWGQGTMVTVSS (SEQ ID NO:152)<br><br>EGFR-scFv-3 (VH-VL):<br>QVQLQESGPGLVKPSETLSLTCTVSGGSVSSGDYYWTWIRQSP GK**C**LEWIGHIYYSGNTNYNP**R**LKSRLTISIDTSKTQFSLKLSSVT AADTAIYYCVRDRVTGAFDIWGQGTMVTVSS*GGGGSGGGGSG GGGSGGGGS*DIQMTQSPSSLSASVGDRVTITCQASQDISNYLNW YQQKPGKAPKLLIYDASNLETGVPSRFSGSGSGTDFTFTISSLQP EDIATY**Y**CQHF**R**HLPLAFG**C**GTKVEIK  (SEQ ID NO:153) | |
| EGFR-binder-4 | QVQLQESGPGLVKPSETLSLTC TVSGGSVSSGDYYWTWIRQSP GKGLEWIGHIYYSGNTNYNPS LKSRLTISIDTSKTQFSLKLSSV TAADTAIYYCVRDRVTGAFDI WGQGTMVTVSS  (SEQ ID NO:135)<br><br>CDR1: GGSVSSGDYYWT (SEQ ID NO:136)<br><br>CDR2: HIYYSGNTNYNPSLKS(SEQ ID NO:137)<br>CDR3: DRVTGAFDI (SEQ ID NO:138) | DIQMTQSPSSLSASVGDRVTI TCQASQDISNYLNWYQQKPG KAPKLLIYDASNLETGVPSRF SGSGSGTDFTFTISSLQPEDIA TY**Y**CQHF**R**HLPLAFGGGTK VEIK (SEQ ID NO:150)<br><br><br>CDR1: QASQDISNYLN (SEQ ID NO:140)<br>CDR2: LLIYDASNLET (SEQ ID NO:141)<br><br>CDR3: QHFRHLPLA (SEQ ID NO:151) |
| EGFR-scFv-4 | EGFR-scFv-4 (VL-VH): | |

EP 4 192 872 B1

| EGFR binder | Heavy chain variable domain amino acid sequence | Light chain variable domain amino acid sequence |
|---|---|---|
| | DIQMTQSPSSLSASVGDRVTITCQASQDISNYLNWYQQKPGKAP KLLIYDASNLETGVPSRFSGSGSGTDFTFTISSLQPEDIATY**Y**CQ HF**R**HLPLAFG**C**GTKVEIK*GGGGSGGGGSGGGGSGGGGS*QVQL QESGPGLVKPSETLSLTCTVSGGSVSSGDYYWTWIRQSPGK**C**L EWIGHIYYSGNTNYNPSLKSRLTISIDTSKTQFSLKLSSVTAADT AIYYCVRDRVTGAFDIWGQGTMVTVSS (SEQ ID NO:154) <br><br> EGFR-scFv-4 (VH-VL): <br> QVQLQESGPGLVKPSETLSLTCTVSGGSVSSGDYYWTWIRQSP GK**C**LEWIGHIYYSGNTNYNPSLKSRLTISIDTSKTQFSLKLSSVT AADTAIYYCVRDRVTGAFDIWGQGTMVTVSS*GGGGSGGGGSG GGGSGGGGS*DIQMTQSPSSLSASVGDRVTITCQASQDISNYLNW YQQKPGKAPKLLIYDASNLETGVPSRFSGSGSGTDFTFTISSLQP EDIATY**Y**CQHF**R**HLPLAFG**C**GTKVEIK (SEQ ID NO:155) | |
| Consensus Sequence (EGFR-binder-3 and EGFR-binder-4) | QVQLQESGPGLVKPSETLSLTC TVSGGSVSSGDYYWTWIRQSP GKGLEWIGHIYYSGNTNYNP**X** LKSRLTISIDTSKTQFSLKLSSV TAADTAIYYCVRDRVTGAFDI WGQGTMVTVSS <br><br> where X is R or S (SEQ ID NO:156) <br> CDR1: GGSVSSGDYYWT (SEQ ID NO:136) <br> CDR2: HIYYSGNTNYNPXLKS where X is R or S (SEQ ID NO:157) <br> CDR3: DRVTGAFDI (SEQ ID NO:138) | DIQMTQSPSSLSASVGDRVTI TCQASQDISNYLNWYQQKPG KAPKLLIYDASNLETGVPSRF SGSGSGTDFTFTISSLQPEDIA TY**Y**CQHF**R**HLPLAFGGGTK VEIK (SEQ ID NO:150) <br><br> CDR1: QASQDISNYLN (SEQ ID NO:140) <br> CDR2: LLIYDASNLET (SEQ ID NO:141) <br> CDR3: QHFRHLPLA (SEQ ID NO:151) |

EP 4 192 872 B1

(continued)

| EGFR binder | Heavy chain variable domain amino acid sequence | Light chain variable domain amino acid sequence |
|---|---|---|
| Consensus Sequence (EGFR-scFv-3 and EGFR-scFv-4) | scFv-309 (VL-VH):<br><br>DIQMTQSPSSLSASVGDRVTITCQASQDISNYLNWYQQKPGKAP KLLIYDASNLETGVPSRFSGSGSGTDFTFTISSLQPEDIATY**Y**CQ HF**R**HLPLAFG**C**GTKVEIK*GGGGSGGGGSGGGGSGGGGS*QVQL QESGPGLVKPSETLSLTCTVSGGSVSSGDYYWTWIRQSPGK**C**LE WIGHIYYSGNTNYNP**X**LKSRLTISIDTSKTQFSLKLSSVTAADTA IYYCVRDRVTGAFDIWGQGTMVTVSS<br><br>where X is R or S (SEQ ID NO:158)<br>scFv-310 (VH-VL):<br><br>QVQLQESGPGLVKPSETLSLTCTVSGGSVSSGDYYWTWIRQSP GK**C**LEWIGHIYYSGNTNYNP**X**LKSRLTISIDTSKTQFSLKLSSVT AADTAIYYCVRDRVTGAFDIWGQGTMVTVSS*GGGGSGGGGSG GGGSGGGGS*DIQMTQSPSSLSASVGDRVTITCQASQDISNYLNW YQQKPGK APKLLIYDASNLETGVPSRFSGSGSGTDFTFTISSLQP EDIATY**Y**CQHF**R**HLPLAFG**C**GTKVEIK<br><br>where X is R or S (SEQ ID NO:159) | |

38

In certain embodiments, the amino acid sequence of the second antigen-binding site comprises an additional arginine (R) residue at the C-terminus of the VL *(e.g.,* SEQ ID NO: 139, 147, or 150). In certain embodiments, the second antigen-binding site comprises a scFv including a VL amino acid sequence that contains an additional arginine (R) residue at the C-terminus of the VL amino acid sequence.

In certain embodiments, the amino acid sequence of the second antigen-binding site comprises one or more mutations relative to the sequence of panitumumab selected from S62R in the VH, D92R in the VL, and F87Y in the VL, under the Chothia numbering scheme. In certain embodiments, the second antigen-binding site that binds EGFR *(e.g.,* human EGFR) comprises an antibody heavy chain variable domain (VH) that comprises an amino acid sequence at least 90% *(e.g.,* at least 91%, at least 92%, at least 93%, at least 94%, at least 95%, at least 96%, at least 97%, at least 98%, or at least 99%) identical to the VH of EGFR-binder-1 as disclosed in **Table 1,** and an antibody light chain variable domain (VL) that comprises an amino acid sequence at least 90% *(e.g.,* at least 91%, at least 92%, at least 93%, at least 94%, at least 95%, at least 96%, at least 97%, at least 98%, or at least 99%) identical to the VL of EGFR-binder-1 as disclosed in **Table 1**. In certain embodiments, the second antigen-binding site that binds EGFR (*e.g.,* human EGFR) comprises an antibody heavy chain variable domain (VH) that comprises an amino acid sequence at least 90% (*e.g.,* at least 91%, at least 92%, at least 93%, at least 94%, at least 95%, at least 96%, at least 97%, at least 98%, at least 99%, or 100%) identical to the VH of EGFR-binder-2, EGFR-binder-3, or EGFR-binder-4 as disclosed in **Table 2,** and an antibody light chain variable domain (VL) that comprises an amino acid sequence at least 90% (*e.g.,* at least 91%, at least 92%, at least 93%, at least 94%, at least 95%, at least 96%, at least 97%, at least 98%, at least 99%, or 100%) identical to the VL of EGFR-binder-2, EGFR-binder-3, or EGFR-binder-4 as disclosed in **Table 2.** In certain embodiments, the second antigen-binding site comprises the heavy chain CDR1, CDR2, and CDR3 and the light chain CDR1, CDR2, and CDR3, determined under Kabat (see Kabat et al., (1991) Sequences of Proteins of Immunological Interest, NIH Publication No. 91-3242, Bethesda), Chothia (see, *e.g.,* Chothia C & Lesk A M, (1987), J. Mol. Biol. 196: 901-917), MacCallum (see MacCallum R M et al., (1996) J. Mol. Biol. 262: 732-745), or any other CDR determination method known in the art, of the VH and VL sequences of EGFR-binder-2, EGFR-binder-3, or EGFR-binder-4 as disclosed in **Table 2**. In certain embodiments, the second antigen-binding site comprises the heavy chain CDR1, CDR2, and CDR3 and the light chain CDR1, CDR2, and CDR3 of EGFR-binder-2, EGFR-binder-3, or EGFR-binder-4 as disclosed in **Table 2.**

In certain embodiments, the amino acid sequence of the second antigen-binding site comprises S62R in the VH and F87Y in the VL mutations relative to the sequence of panitumumab, under the Chothia numbering scheme. In certain embodiments, the second antigen-binding site that binds EGFR comprises a VH that comprises an amino acid sequence at least 90% (*e.g.,* at least 91%, at least 92%, at least 93%, at least 94%, at least 95%, at least 96%, at least 97%, at least 98%, or at least 99%) identical to SEQ ID NO: 135, and a VL that comprises an amino acid sequence at least 90% (*e.g.,* at least 91%, at least 92%, at least 93%, at least 94%, at least 95%, at least 96%, at least 97%, at least 98%, or at least 99%) identical to SEQ ID NO:139. In certain embodiments, the second antigen-binding site that binds EGFR comprises a heavy chain variable domain (VH) that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO: 145, and a light chain variable domain (VL) that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO: 147. In certain embodiments, the second antigen-binding site comprises the heavy chain CDR1, CDR2, and CDR3 and the light chain CDR1, CDR2, and CDR3, determined under Kabat (see Kabat et al., (1991) Sequences of Proteins of Immunological Interest, NIH Publication No. 91-3242, Bethesda), Chothia (see, *e.g.,* Chothia C & Lesk A M, (1987), J. Mol. Biol. 196: 901-917), MacCallum (see MacCallum R M et al., (1996) J. Mol. Biol. 262: 732-745), or any other CDR determination method known in the art, of the VH and VL sequences of EGFR-binder-2 disclosed in **Table 2**. In certain embodiments, the VH comprises CDR1, CDR2, and CDR3 comprising the amino acid sequences of SEQ ID NOs: 136, 146, and 138, respectively. In certain embodiments, the VL comprises CDR1, CDR2, and CDR3 comprising the amino acid sequences of SEQ ID NOs: 140, 141, and 142, respectively. In certain embodiments, the second antigen-binding site comprises (a) a VH that comprises CDR1, CDR2, and CDR3 comprising the amino acid sequences of SEQ ID NOs: 136, 146, and 138, respectively; and (b) a VL that comprises CDR1, CDR2, and CDR3 comprising the amino acid sequences of SEQ ID NOs: 140, 141, and 142, respectively. In certain embodiments, the second antigen-binding site is present as an scFv, wherein the scFv comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO: 148 or 149. In certain embodiments, the second antigen-binding site is present as an scFv, wherein the scFv comprises an amino acid sequence identical to SEQ ID NO: 148 or 149. In certain embodiments, the second antigen-binding site is present as an scFv, wherein the scFv comprises an amino acid sequence identical to SEQ ID NO:148. In certain embodiments, the second antigen-binding site is present as an scFv, wherein the scFv comprises an amino acid sequence identical to SEQ ID NO:149.

In certain embodiments, the amino acid sequence of the second antigen-binding site comprises S62R in the VH, F87Y in the VL, and D92R in the VL mutations relative to the sequence of panitumumab, under the Chothia numbering scheme. In certain embodiments, the second antigen-binding site that binds EGFR comprises a VH that comprises an amino acid sequence at least 90% (*e.g.,* at least 91%, at least 92%, at least 93%, at least 94%, at least 95%, at least 96%, at least 97%, at least 98%, or at least 99%) identical to SEQ ID NO:135, and a VL that comprises an amino acid sequence at least 90% (*e.g.,* at least 91%, at least 92%, at least 93%, at least 94%, at least 95%, at least 96%, at least 97%, at least 98%, or at least 99%) identical to SEQ ID NO:139. In certain embodiments, the second antigen-binding site that binds EGFR comprises a heavy chain variable domain (VH) that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO: 145, and a light chain variable domain (VL) that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO: 150. In certain embodiments, the second antigen-binding site that binds EGFR comprises a heavy chain variable domain (VH) comprising SEQ ID NO: 170, and a light chain variable domain (VL) comprising SEQ ID NO: 171. In certain embodiments, the second antigen-binding site comprises the heavy chain CDR1, CDR2, and CDR3 and the light chain CDR1, CDR2, and CDR3, determined under Kabat (see Kabat et al., (1991) Sequences of Proteins of Immunological Interest, NIH Publication No. 91-3242, Bethesda), Chothia (see, *e.g.,* Chothia C & Lesk A M, (1987), J. Mol. Biol. 196: 901-917), MacCallum (see MacCallum R M et al., (1996) J. Mol. Biol. 262: 732-745), or any other CDR determination method known in the art, of the VH and VL sequences of EGFR-binder-3 disclosed in **Table 2**. In certain embodiments, the VH comprises CDR1, CDR2, and CDR3 comprising the amino acid sequences of SEQ ID NOs: 136, 146, and 138, respectively. In certain embodiments, the VL comprises CDR1, CDR2, and CDR3 comprising the amino acid sequences of SEQ ID NOs: 140, 141, and 151, respectively. In certain embodiments, the second antigen-binding site comprises (a) a VH that comprises CDR1, CDR2, and CDR3 comprising the amino acid sequences of SEQ ID NOs: 136, 146, and 138, respectively; and (b) a VL that comprises CDR1, CDR2, and CDR3 comprising the amino acid sequences of SEQ ID NOs: 140, 141, and 151, respectively. In certain embodiments, the second antigen-binding site is present as an scFv, wherein the scFv comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO: 152 or 153. In certain embodiments, the second antigen-binding site is present as an scFv, wherein the scFv comprises an amino acid sequence identical to SEQ ID NO: 152 or 153. In certain embodiments, the second antigen-binding site is present as an scFv, wherein the scFv comprises an amino acid sequence identical to SEQ ID NO: 152. In certain embodiments, the second antigen-binding site is present as an scFv, wherein the scFv comprises an amino acid sequence identical to SEQ ID NO: 153.

In certain embodiments, the amino acid sequence of the second antigen-binding site comprises F87Y in the VL and D92R in the VL mutations relative to the sequence of panitumumab, under the Chothia numbering scheme. In certain embodiments, the second antigen-binding site that binds EGFR comprises a VH that comprises an amino acid sequence at least 90% (*e.g.,* at least 91%, at least 92%, at least 93%, at least 94%, at least 95%, at least 96%, at least 97%, at least 98%, or at least 99%) identical to SEQ ID NO: 135, and a VL that comprises an amino acid sequence at least 90% (*e.g.,* at least 91%, at least 92%, at least 93%, at least 94%, at least 95%, at least 96%, at least 97%, at least 98%, or at least 99%) identical to SEQ ID NO:139. In certain embodiments, the second antigen-binding site that binds EGFR comprises a heavy chain variable domain (VH) that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO:135, and a light chain variable domain (VL) that comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO: 150. In certain embodiments, the second antigen-binding site comprises the heavy chain CDR1, CDR2, and CDR3 and the light chain CDR1, CDR2, and CDR3, determined under Kabat (see Kabat et al., (1991) Sequences of Proteins of Immunological Interest, NIH Publication No. 91-3242, Bethesda), Chothia (see, *e.g.,* Chothia C & Lesk A M, (1987), J. Mol. Biol. 196: 901-917), MacCallum (see MacCallum R M et al., (1996) J. Mol. Biol. 262: 732-745), or any other CDR determination method known in the art, of the VH and VL sequences of EGFR-binder-4 *d*isclosed in **Table 2.** In certain embodiments, the VH comprises CDR1, CDR2, and CDR3 comprising the amino acid sequences of SEQ ID NOs: 136, 137, and 138, respectively. In certain embodiments, the VL comprises CDR1, CDR2, and CDR3 comprising the amino acid sequences of SEQ ID NOs: 140, 141, and 151, respectively. In certain embodiments, the second antigen-binding site comprises (a) a VH that comprises CDR1, CDR2, and CDR3 comprising the amino acid sequences of SEQ ID NOs: 136, 137, and 138, respectively; and (b) a VL that comprises CDR1, CDR2, and CDR3 comprising the amino acid sequences of SEQ ID NOs: 140, 141, and 151, respectively. In certain embodiments, the second antigen-binding site is present as an scFv, wherein the scFv comprises an amino acid sequence at least 90% (*e.g.,* at least 91%, at least 92%, at least 93%, at least 94%, at least 95%, at least 96%, at least 97%, at least 98%, at least 99%, or 100%) identical to SEQ ID NO: 154 or 155. In certain embodiments, the second antigen-binding site is present as an scFv, wherein the scFv comprises an amino acid sequence identical to SEQ ID NO: 154. In certain embodiments, the second antigen-binding site is present as an scFv, wherein the scFv comprises an amino acid sequence identical to SEQ ID NO: 155.

In certain embodiments, the amino acid sequence of the second antigen-binding site comprises F87Y in the VL and D92R in the VL, and optionally S62R in the VH mutations relative to the sequence of panitumumab, under the Chothia numbering scheme. In certain embodiments, the second antigen-binding site that binds EGFR comprises a VH that comprises an amino acid sequence at least 90% *(e.g.,* at least 91%, at least 92%, at least 93%, at least 94%, at least 95%, at least 96%, at least 97%, at least 98%, or at least 99%) identical to SEQ ID NO:135, and a VL that comprises an amino acid sequence at least 90% *(e.g.,* at least 91%, at least 92%, at least 93%, at least 94%, at least 95%, at least 96%, at least 97%, at least 98%, or at least 99%) identical to SEQ ID NO:139. In certain embodiments, the second antigen-binding site that binds EGFR comprises a heavy chain variable domain (VH) that comprises an amino acid sequence at least 90% *(e.g.,* at least 91%, at least 92%, at least 93%, at least 94%, at least 95%, at least 96%, at least 97%, at least 98%, at least 99%, or 100%) identical to SEQ ID NO: 156, and a light chain variable domain (VL) that comprises an amino acid sequence at least 90% *(e.g.,* at least 91%, at least 92%, at least 93%, at least 94%, at least 95%, at least 96%, at least 97%, at least 98%, at least 99%, or 100%) identical to SEQ ID NO: 150. In certain embodiments, the second antigen-binding site comprises the heavy chain CDR1, CDR2, and CDR3 and the light chain CDR1, CDR2, and CDR3, determined under Kabat (see Kabat et al., (1991) Sequences of Proteins of Immunological Interest, NIH Publication No. 91-3242, Bethesda), Chothia (see, *e.g.,* Chothia C & Lesk A M, (1987), J. Mol. Biol. 196: 901-917), MacCallum (see MacCallum R M et al., (1996) J. Mol. Biol. 262: 732-745), or any other CDR determination method known in the art, of the consensus VH and VL sequences disclosed in **Table 2.** In certain embodiments, the VH comprises CDR1, CDR2, and CDR3 comprising the amino acid sequences of SEQ ID NOs: 136, 157, and 138, respectively. In certain embodiments, the VL comprises CDR1, CDR2, and CDR3 comprising the amino acid sequences of SEQ ID NOs: 140, 141, and 151, respectively. In certain embodiments, the second antigen-binding site comprises (a) a VH that comprises CDR1, CDR2, and CDR3 comprising the amino acid sequences of SEQ ID NOs: 136, 157, and 138, respectively; and (b) a VL that comprises CDR1, CDR2, and CDR3 comprising the amino acid sequences of SEQ ID NOs: 140, 141, and 151, respectively. In certain embodiments, the second antigen-binding site is present as an scFv, wherein the scFv comprises an amino acid sequence at least 90% *(e.g.,* at least 91%, at least 92%, at least 93%, at least 94%, at least 95%, at least 96%, at least 97%, at least 98%, at least 99%, or 100%) identical to SEQ ID NO: 158 or 159. In certain embodiments, the second antigen-binding site is present as an scFv, wherein the scFv comprises an amino acid sequence identical to SEQ ID NO: 158. In certain embodiments, the second antigen-binding site is present as an scFv, wherein the scFv comprises an amino acid sequence identical to SEQ ID NO:159.

Alternatively, novel antigen-binding sites that can bind to EGFR can be identified by screening for binding to the amino acid sequence of EGFR, an isoform thereof, a variant thereof, a mature extracellular fragment thereof or a fragment containing a domain of EGFR.

**Table 3.** Sequences for exemplary EGFR Isoforms

| EGFR Isoform | Amino Acid Sequence |
|---|---|
| 1 (Uniprot ID No.: P00533-1) | MRPSGTAGAALLALLAALCPASRALEEKKVCQGTSNKLTQLGT FEDHFLSLQRMFNNCEVVLGNLEITYVQRNYDLSFLKTIQEVAG YVLIALNTVERIPLENLQIIRGNMYYENSYALAVLSNYDANKTG LKELPMRNLQEILHGAVRFSNNPALCNVESIQWRDIVSSDFLSN MSMDFQNHLGSCQKCDPSCPNGSCWGAGEENCQKLTKIICAQ QCSGRCRGKSPSDCCHNQCAAGCTGPRESDCLVCRKFRDEATC KDTCPPLMLYNPTTYQMDVNPEGKYSFGATCVKKCPRNYVVT DHGSCVRACGADSYEMEEDGVRKCKKCEGPCRKVCNGIGIGE FKDSLSINATNIKHFKNCTSISGDLHILPVAFRGDSFTHTPPLDPQ ELDILKTVKEITGFLLIQAWPENRTDLHAFENLEIIRGRTKQHGQ FSLAVVSLNITSLGLRSLKEISDGDVIISGNKNLCYANTINWKKL FGTSGQKTKIISNRGENSCKATGQVCHALCSPEGCWGPEPRDC VSCRNVSRGRECVDKCNLLEGEPREFVENSECIQCHPECLPQA MNITCTGRGPDNCIQCAHYIDGPHCVKTCPAGVMGENNTLVW KYADAGHVCHLCHPNCTYGCTGPGLEGCPTNGPKIPSIATGMV GALLLLLVVALGIGLFMRRRHIVRKRTLRRLLQERELVEPLTPS GEAPNQALLRILKETEFKKIKVLGSGAFGTVYKGLWIPEGEKVK IPVAIKELREATSPKANKEILDEAYVMASVDNPHVCRLLGICLTS TVQLITQLMPFGCLLDYVREHKDNIGSQYLLNWCVQIAKGMN YLEDRRLVHRDLAARNVLVKTPQHVKITDFGLAKLLGAEEKEY HAEGGKVPIKWMALESILHRIYTHQSDVWSYGVTVWELMTFG SKPYDGIPASEISSILEKGERLPQPPICTIDVYMIMVKCWMIDAD SRPKFRELIIEFSKMARDPQRYLVIQGDERMHLPSPTDSNFYRAL MDEEDMDDVVDADEYLIPQQGFFSSPSTSRTPLLSSLSATSNNS TVACIDRNGLQSCPIKEDSFLQRYSSDPTGALTEDSIDDTFLPVP EYINQSVPKRPAGSVQNPVYHNQPLNPAPSRDPHYQDPHSTAV GNPEYLNTVQPTCVNSTFDSPAHWAQKGSHQISLDNPDYQQDF FPKEAKPNGIFKGSTAENAEYLRVAPQSSEFIGA (SEQ ID NO:160) |
| 2 (Uniprot ID No.: P00533-2) | MRPSGTAGAALLALLAALCPASRALEEKKVCQGTSNKLTQLGT FEDHFLSLQRMFNNCEVVLGNLEITYVQRNYDLSFLKTIQEVAG YVLIALNTVERIPLENLQIIRGNMYYENSYALAVLSNYDANKTG LKELPMRNLQEILHGAVRFSNNPALCNVESIQWRDIVSSDFLSN MSMDFQNHLGSCQKCDPSCPNGSCWGAGEENCQKLTKIICAQ QCSGRCRGKSPSDCCHNQCAAGCTGPRESDCLVCRKFRDEATC KDTCPPLMLYNPTTYQMDVNPEGKYSFGATCVKKCPRNYVVT DHGSCVRACGADSYEMEEDGVRKCKKCEGPCRKVCNGIGIGE FKDSLSINATNIKHFKNCTSISGDLHILPVAFRGDSFTHTPPLDPQ ELDILKTVKEITGLS (SEQ ID NO:161) |

(continued)

| EGFR Isoform | Amino Acid Sequence |
|---|---|
| 3 (Uniprot ID No.: P00533-3) | MRPSGTAGAALLALLAALCPASRALEEKKVCQGTSNKLTQLGT FEDHFLSLQRMFNNCEVVLGNLEITYVQRNYDLSFLKTIQEVAG YVLIALNTVERIPLENLQIIRGNMYYENSYALAVLSNYDANKTG LKELPMRNLQEILHGAVRFSNNPALCNVESIQWRDIVSSDFLSN MSMDFQNHLGSCQKCDPSCPNGSCWGAGEENCQKLTKIICAQ QCSGRCRGKSPSDCCHNQCAAGCTGPRESDCLVCRKFRDEATC KDTCPPLMLYNPTTYQMDVNPEGKYSFGATCVKKCPRNYVVT DHGSCVRACGADSYEMEEDGVRKCKKCEGPCRKVCNGIGIGE FKDSLSINATNIKHFKNCTSISGDLHILPVAFRGDSFTHTPPLDPQ ELDILKTVKEITGFLLIQAWPENRTDLHAFENLEIIRGRTKQHGQ FSLAVVSLNITSLGLRSLKEISDGDVIISGNKNLCYANTINWKKL FGTSGQKTKIISNRGENSCKATGQVCHALCSPEGCWGPEPRDC VSCRNVSRGRECVDKCNLLEGEPREFVENSECIQCHPECLPQA MNITCTGRGPDNCIQCAHYIDGPHCVKTCPAGVMGENNTLVW KYADAGHVCHLCHPNCTYPGNESLKAMLFCLFKLSSCNQSN DGSVSHQSGSPAAQESCLGWIPSLLPSEFQLGWGGCSHLHAWP SASVIITASSCH<br>(SEQ ID NO:162) |
| 4 (Uniprot ID No.: P00533-4) | MRPSGTAGAALLALLAALCPASRALEEKKVCQGTSNKLTQLGT FEDHFLSLQRMFNNCEVVLGNLEITYVQRNYDLSFLKTIQEVAG YVLIALNTVERIPLENLQIIRGNMYYENSYALAVLSNYDANKTG LKELPMRNLQEILHGAVRFSNNPALCNVESIQWRDIVSSDFLSN MSMDFQNHLGSCQKCDPSCPNGSCWGAGEENCQKLTKIICAQ QCSGRCRGKSPSDCCHNQCAAGCTGPRESDCLVCRKFRDEATC KDTCPPLMLYNPTTYQMDVNPEGKYSFGATCVKKCPRNYVVT DHGSCVRACGADSYEMEEDGVRKCKKCEGPCRKVCNGIGIGE FKDSLSINATNIKHFKNCTSISGDLHILPVAFRGDSFTHTPPLDPQ ELDILKTVKEITGFLLIQAWPENRTDLHAFENLEIIRGRTKQHGQ FSLAVVSLNITSLGLRSLKEISDGDVIISGNKNLCYANTINWKKL FGTSGQKTKIISNRGENSCKATGQVCHALCSPEGCWGPEPRDC VSCRNVSRGRECVDKCNLLEGEPREFVENSECIQCHPECLPQA MNITCTGRGPDNCIQCAHYIDGPHCVKTCPAGVMGENNTLVW KYADAGHVCHLCHPNCTYGS<br>(SEQ ID NO:163) |

In some embodiments, novel antigen-binding sites that can bind to EGFR can be identified by screening for binding to the amino acid sequence defined by SEQ ID NOs: 160-163, a variant thereof, a mature extracellular fragment thereof or a fragment containing a domain of EGFR.

In each of the foregoing embodiments, it is contemplated herein that the scFv, VH and/or VL sequences that bind EGFR may contain amino acid alterations (*e.g.*, at least 1, 2, 3, 4, 5, or 10 amino acid substitutions, deletions, or additions) in the framework regions of the VH and/or VL without affecting their ability to bind EGFR. For example, it is contemplated herein that scFv, VH and/or VL sequences that bind EGFR may contain cysteine heterodimerization mutations, facilitating formation of a disulfide bridge between the VH and VL of the scFv.

In certain embodiments, the second antigen-binding site of the multispecific binding protein disclosed herein binds human EGFR or the extracellular region thereof at a $K_D$ value less than or equal to (affinity greater than or equal to) 10 nM, 9 nM, 8 nM, 7 nM, 6 nM, 5 nM, or 4 nM. In certain embodiments, the antigen-binding site of the present application binds human EGFR or the extracellular region thereof at a $K_D$ value less than or equal to (affinity greater than or equal to) 4 nM. In certain embodiments, an antigen-binding site of the present application binds human EGFR or the extracellular region thereof at a $K_D$ value less than or equal to (affinity greater than or equal to) about 2.0 nM, 2.1 nM, 2.2 nM, 2.3 nM, 2.4 nM, 2.5 nM, 2.6 nM, 2.7 nM, 2.8 nM, 2.9 nM, 3.0 nM, 3.1 nM, 3.2 nM, 3.3 nM, 3.4 nM, 3.5 nM, 3.6 nM, 3.7 nM, 3.8 nM, 3.9 nM, 4.0 nM, 4.1 nM, 4.2 nM, 4.3 nM, 4.4 nM, 4.5 nM, 4.6 nM, 4.7 nM, 4.8 nM, 4.9 nM or 5.0 nM. In certain embodiments, an antigen-binding site of the present application binds human EGFR or the extracellular region thereof at a $K_D$ value in the range of about 1.0-3.5 nM, 1.0-4.0 nM, 1.0-4.5 nM, 1.0-5.0 nM, 1.5-3.5 nM, 1.5-4.0 nM, 1.5-4.5 nM, 1.5-5.0 nM, 2.0-3.5 nM, 2.0-4.0 nM, 2.0-4.5 nM, 2.0-5.0 nM, 2.5-3.5 nM, 2.5-4.0 nM, 2.5-4.5 nM, 2.5-5.0 nM, 3.0-3.5 nM, 3.0-4.0 nM, 3.0-4.5 nM, or 3.0-5.0 nM.

In certain embodiments, the second antigen-binding site of the multispecific binding protein disclosed herein binds rhesus macaque EGFR or the extracellular region thereof at a $K_D$ value less than or equal to (affinity greater than or equal to) 10 nM, 9 nM, 8 nM, 7 nM, 6 nM, 5 nM, or 4 nM. In certain embodiments, an antigen-binding site of the present application binds rhesus macaque EGFR or the extracellular region thereof at a $K_D$ value in the range of about 1-10 nM, 1-6 nM, 2-10 nM, 2-6 nM, 3-10 nM, 3-6 nM, 4-10 nM, 4-6 nM, 5-10 nM, or 5-6 nM.

In certain embodiments, the second antigen-binding site of the multispecific binding protein disclosed herein has greater thermostability than a corresponding antigen-binding site having the VH and VL sequences of SEQ ID NOs: 135 and 139, wherein the second antigen-binding site does not comprise a G44C mutation in the VH and a G100C mutation in the VL. In certain embodiments, the second antigen-binding site of the multispecific binding protein disclosed herein has greater thermostability than a corresponding antigen-binding site having an amino acid sequence of SEQ ID NO: 143 or 144, wherein the second antigen-binding site takes an scFv format in the VL-VH or VH-VL orientation, respectively. Methods of measuring thermostability include but are not limited to differential scanning calorimetry (DSC), for example, as disclosed in Example 3 below. In certain embodiments, where the second antigen-binding site takes an scFv format in the VL-VH orientation, a melting temperature of the second antigen-binding site (*e.g.*, Tonset or Tm1 of a TriNKET in the F3' format, as measured by DSC) is higher than the corresponding melting temperature of an scFv having the amino acid sequence of SEQ ID NO: 143 by at least 1 °C, 2 °C, 3 °C, 4 °C, 5 °C, or 6 °C. In certain embodiments, where the second antigen-binding site takes an scFv format in the VH-VL orientation, a melting temperature of the second antigen-binding site (e.g., Tonset or Tm1 of a TriNKET in the F3' format, as measured by DSC) is higher than the corresponding melting temperature of an scFv having the amino acid sequence of SEQ ID NO: 144 by at least 1 °C, 2 °C, 3 °C, 4 °C, 5 °C, or 6 °C.

### Fc domain

Within the Fc domain, CD16 binding is mediated by the hinge region and the CH2 domain. For example, within human IgG1, the interaction with CD16 is primarily focused on amino acid residues Asp 265 - Glu 269, Asn 297 - Thr 299, Ala 327 - Ile 332, Leu 234 - Ser 239, and carbohydrate residue N-acetyl-D-glucosamine in the CH2 domain (see, Sondermann et al., Nature, 406 (6793):267-273). Based on the known domains, mutations can be selected to enhance or reduce the binding affinity to CD16, such as by using phagedisplayed libraries or yeast surface-displayed cDNA libraries, or can be designed based on the known three-dimensional structure of the interaction. Accordingly, in certain embodiments, the antibody Fc domain or the portion thereof comprises a hinge and a CH2 domain.

The assembly of heterodimeric antibody heavy chains can be accomplished by expressing two different antibody heavy chain sequences in the same cell, which may lead to the assembly of homodimers of each antibody heavy chain as well as assembly of heterodimers. Promoting the preferential assembly of heterodimers can be accomplished by incorporating different mutations in the CH3 domain of each antibody heavy chain constant region as shown in US13/494870, US16/028850, US11/533709, US12/875015, US13/289934, US14/773418, US12/811207, US13/866756, US14/647480, and US14/830336. For example, mutations can be made in the CH3 domain based on human IgG1 and incorporating distinct pairs of amino acid substitutions within a first polypeptide and a second polypeptide that allow these two chains to selectively heterodimerize with each other. The positions of amino acid substitutions illustrated below are all numbered according to the EU index as in Kabat.

In one scenario, an amino acid substitution in the first polypeptide replaces the original amino acid with a larger amino acid, selected from arginine (R), phenylalanine (F), tyrosine (Y) or tryptophan (W), and at least one amino acid substitution in the second polypeptide replaces the original amino acid(s) with a smaller amino acid(s), chosen from alanine (A), serine (S), threonine (T), or valine (V), such that the larger amino acid substitution (a protuberance) fits into the surface of the smaller amino acid substitutions (a cavity). For example, one polypeptide can incorporate a T366W substitution, and the other can incorporate three substitutions including T366S, L368A, and Y407V.

An antibody heavy chain variable domain of the present application can optionally be coupled to an amino acid sequence at least 90% identical to an antibody constant region, such as an IgG constant region including hinge, CH2 and CH3 domains with or without CH1 domain. In some embodiments, the amino acid sequence of the constant region is at least 90% identical to a human antibody constant region, such as a human IgG1 constant region, an IgG2 constant region, IgG3 constant region, or IgG4 constant region. In one embodiment, the antibody Fc domain or a portion thereof sufficient to bind CD16 comprises an amino acid sequence at least 90% *(e.g.,* at least 91%, at least 92%, at least 93%, at least 94%, at least 95%, at least 96%, at least 97%, at least 98%, at least 99%, or 100%) identical to wild-type human IgG1 Fc sequence: DKTHTCPPCPAPELLGGPSVFLFPPKPKDTLMISRTPEVTCVVVDVSHEDPEVKFNWY VDGVEVHNAKTKPREEQY NSTYRVVSVLTVLHQDWLNGKEYKCKVSNKALPAPIE KTISKAKGQPREPQVYTLPPSRDELTKNQVSLTCLVKGFYP SDIAVEWESNGQPENN YKTTPPVLDSDGSFFLYSKLTVDKSRWQQGNVFSCSVMHEALHNHYTQKSLSLSPG (SEQ ID NO: 118). In some other embodiments, the amino acid sequence of the constant region is at least 90% identical to an antibody constant region from another mammal, such as rabbit, dog, cat, mouse, or horse.

In some embodiments, the antibody constant domain linked to the scFv or the Fab fragment is able to bind to CD16. In some embodiments, the protein incorporates a portion of an antibody Fc domain (for example, a portion of an antibody Fc domain sufficient to bind CD16), wherein the antibody Fc domain comprises a hinge and a CH2 domain (for example, a hinge and a CH2 domain of a human IgG1 antibody), and/or amino acid sequences at least 90% identical to amino acid sequence 234-332 of a human IgG antibody.

One or more mutations can be incorporated into the constant region as compared to a human IgG1 constant region, for example at Q347, Y349, L351, S354, E356, E357, K360, Q362, S364, T366, L368, K370, N390, K392, T394, D399, S400, D401, F405, Y407, K409, T411 and/or K439. Exemplary substitutions include, for example, Q347E, Q347R, Y349S, Y349K, Y349T, Y349D, Y349E, Y349C, T350V, L351K, L351D, L351Y, S354C, E356K, E357Q, E357L, E357W, K360E, K360W, Q362E, S364K, S364E, S364H, S364D, T366V, T366I, T366L, T366M, T366K, T366W, T366S, L368E, L368A, L368D, K370S, N390D, N390E, K392L, K392M, K392V, K392F, K392D, K392E, T394F, T394W, D399R, D399K, D399V, S400K, S400R, D401K, F405A, F405T, Y407A, Y407I , Y407V, K409F, K409W, K409D, K409R, T411D, T411E, K439D, and K439E.

In certain embodiments, mutations that can be incorporated into the CH1 of a human IgG1 constant region may be at amino acid V125, F126, P127, T135, T139, A140, F170, P171, and/or V173. In certain embodiments, mutations that can be incorporated into the Cκ of a human IgG1 constant region may be at amino acid E123, F116, S176, V163, S174, and/or T164.

Alternatively, amino acid substitutions could be selected from the following sets of substitutions shown in **Table 4.**

| Table 4 | | |
|---------|------------------|-------------------|
| | First Polypeptide | Second Polypeptide |
| Set 1 | S364E/F405A | Y349K/T394F |
| Set 2 | S364H/D401K | Y349T/T411E |
| Set 3 | S364H/T394F | Y349T/F405A |
| Set 4 | S364E/T394F | Y349K/F405A |
| Set 5 | S364E/T411E | Y349K/D401K |
| Set 6 | S364D/T394F | Y349K/F405A |
| Set 7 | S364H/F405A | Y349T/T394F |
| Set 8 | S364K/E357Q | L368D/K370S |
| Set 9 | L368D/K370S | S364K |
| Set 10 | L368E/K370S | S364K |
| Set 11 | K360E/Q362E | D401K |
| Set 12 | L368D/K370S | S364K/E357L |
| Set 13 | K370S | S364K/E357Q |
| Set 14 | F405L | K409R |
| Set 15 | K409R | F405L |

Alternatively, amino acid substitutions could be selected from the following sets of substitutions shown in **Table 5.**

| Table 5 | | |
|---------|------------------|-------------------|
| | First Polypeptide | Second Polypeptide |
| Set 1 | K409W | D399V/F405T |
| Set 2 | Y349S | E357W |

(continued)

| Table 5 | | First Polypeptide | Second Polypeptide |
|---|---|---|---|
| | Set 3 | K360E | Q347R |
| | Set 4 | K360E/K409W | Q347R/D399V/F405T |
| | Set 5 | Q347E/K360E/K409W | Q347R/D399V/F405T |
| | Set 6 | Y349S/K409W | E357W/D399V/F405T |

Alternatively, amino acid substitutions could be selected from the following sets of substitutions shown in **Table 6.**

| Table 6 | | First Polypeptide | Second Polypeptide |
|---|---|---|---|
| | Set 1 | T366K/L351K | L351D/L368E |
| | Set 2 | T366K/L351K | L351D/Y349E |
| | Set 3 | T366K/L351K | L351D/Y349D |
| | Set 4 | T366K/L351K | L351D/Y349E/L368E |
| | Set 5 | T366K/L351K | L351D/Y349D/L368E |
| | Set 6 | E356K/D399K | K392D/K409D |

Alternatively, at least one amino acid substitution in each polypeptide chain could be selected from **Table 7.**

| Table 7 | |
|---|---|
| First Polypeptide | Second Polypeptide |
| L351Y, D399R, D399K, S400K, S400R, Y407A, Y407I, Y407V | T366V, T366I, T366L, T366M, N390D, N390E, K392L, K392M, K392V, K392F K392D, K392E, K409F, K409W, T411D and T411E |

Alternatively, at least one amino acid substitution could be selected from the following sets of substitutions in **Table 8,** where the position(s) indicated in the First Polypeptide column is replaced by any known negatively-charged amino acid, and the position(s) indicated in the Second Polypeptide Column is replaced by any known positively-charged amino acid.

| Table 8 | |
|---|---|
| First Polypeptide | Second Polypeptide |
| K392, K370, K409, or K439 | D399, E356, or E357 |

Alternatively, at least one amino acid substitution could be selected from the following set in **Table 9,** where the position(s) indicated in the First Polypeptide column is replaced by any known positively-charged amino acid, and the position(s) indicated in the Second Polypeptide Column is replaced by any known negatively-charged amino acid.

| Table 9 | |
|---|---|
| First Polypeptide | Second Polypeptide |
| D399, E356, or E357 | K409, K439, K370, or K392 |

Alternatively, amino acid substitutions could be selected from the following sets in **Table 10.**

| First Polypeptide | Second Polypeptide |
|---|---|
| T350V, L351Y, F405A, and Y407V | T350V, T366L, K392L, and T394W |

Alternatively, or in addition, the structural stability of a hetero-multimeric protein may be increased by introducing S354C on either of the first or second polypeptide chain, and Y349C on the opposing polypeptide chain, which forms an artificial disulfide bridge within the interface of the two polypeptides.

In some embodiments, the amino acid sequence of one polypeptide chain of the antibody constant region differs from the amino acid sequence of an IgG1 constant region at position T366, and wherein the amino acid sequence of the other polypeptide chain of the antibody constant region differs from the amino acid sequence of an IgG1 constant region at one or more positions selected from the group consisting of T366, L368 and Y407.

In some embodiments, the amino acid sequence of one polypeptide chain of the antibody constant region differs from the amino acid sequence of an IgG1 constant region at one or more positions selected from the group consisting of T366, L368 and Y407, and wherein the amino acid sequence of the other polypeptide chain of the antibody constant region differs from the amino acid sequence of an IgG1 constant region at position T366.

In some embodiments, the amino acid sequence of one polypeptide chain of the antibody constant region differs from the amino acid sequence of an IgG1 constant region at one or more positions selected from the group consisting of E357, K360, Q362, S364, L368, K370, T394, D401, F405, and T411 and wherein the amino acid sequence of the other polypeptide chain of the antibody constant region differs from the amino acid sequence of an IgG1 constant region at one or more positions selected from the group consisting of Y349, E357, S364, L368, K370, T394, D401, F405 and T411.

In some embodiments, the amino acid sequence of one polypeptide chain of the antibody constant region differs from the amino acid sequence of an IgG1 constant region at one or more positions selected from the group consisting of Y349, E357, S364, L368, K370, T394, D401, F405 and T411 and wherein the amino acid sequence of the other polypeptide chain of the antibody constant region differs from the amino acid sequence of an IgG1 constant region at one or more positions selected from the group consisting of E357, K360, Q362, S364, L368, K370, T394, D401, F405, and T411.

In some embodiments, the amino acid sequence of one polypeptide chain of the antibody constant region differs from the amino acid sequence of an IgG1 constant region at one or more positions selected from the group consisting of L351, D399, S400 and Y407 and wherein the amino acid sequence of the other polypeptide chain of the antibody constant region differs from the amino acid sequence of an IgG1 constant region at one or more positions selected from the group consisting of T366, N390, K392, K409 and T411.

In some embodiments, the amino acid sequence of one polypeptide chain of the antibody constant region differs from the amino acid sequence of an IgG1 constant region at one or more positions selected from the group consisting of T366, N390, K392, K409 and T411 and wherein the amino acid sequence of the other polypeptide chain of the antibody constant region differs from the amino acid sequence of an IgG1 constant region at one or more positions selected from the group consisting of L351, D399, S400 and Y407.

In some embodiments, the amino acid sequence of one polypeptide chain of the antibody constant region differs from the amino acid sequence of an IgG1 constant region at one or more positions selected from the group consisting of Q347, Y349, K360, and K409, and wherein the amino acid sequence of the other polypeptide chain of the antibody constant region differs from the amino acid sequence of an IgG1 constant region at one or more positions selected from the group consisting of Q347, E357, D399 and F405.

In some embodiments, the amino acid sequence of one polypeptide chain of the antibody constant region differs from the amino acid sequence of an IgG1 constant region at one or more positions selected from the group consisting of Q347, E357, D399 and F405, and wherein the amino acid sequence of the other polypeptide chain of the antibody constant region differs from the amino acid sequence of an IgG1 constant region at one or more positions selected from the group consisting of Y349, K360, Q347 and K409.

In some embodiments, the amino acid sequence of one polypeptide chain of the antibody constant region differs from the amino acid sequence of an IgG1 constant region at one or more positions selected from the group consisting of K370, K392, K409 and K439, and wherein the amino acid sequence of the other polypeptide chain of the antibody constant region differs from the amino acid sequence of an IgG1 constant region at one or more positions selected from the group consisting of D356, E357 and D399.

In some embodiments, the amino acid sequence of one polypeptide chain of the antibody constant region differs from the amino acid sequence of an IgG1 constant region at one or more positions selected from the group consisting of D356, E357 and D399, and wherein the amino acid sequence of the other polypeptide chain of the antibody constant region differs from the amino acid sequence of an IgG1 constant region at one or more positions selected from the group consisting of K370, K392, K409 and K439.

In some embodiments, the amino acid sequence of one polypeptide chain of the antibody constant region differs from the amino acid sequence of an IgG1 constant region at one or more positions selected from the group consisting of L351, E356, T366 and D399, and wherein the amino acid sequence of the other polypeptide chain of the antibody constant region differs from the amino acid sequence of an IgG1 constant region at one or more positions selected from the group consisting of Y349, L351, L368, K392 and K409.

In some embodiments, the amino acid sequence of one polypeptide chain of the antibody constant region differs from the amino acid sequence of an IgG1 constant region at one or more positions selected from the group consisting of Y349, L351, L368, K392 and K409, and wherein the amino acid sequence of the other polypeptide chain of the antibody constant region differs from the amino acid sequence of an IgG1 constant region at one or more positions selected from the group consisting of L351, E356, T366 and D399.

In some embodiments, the amino acid sequence of one polypeptide chain of the antibody constant region differs from the amino acid sequence of an IgG1 constant region by an S354C substitution and wherein the amino acid sequence of the other polypeptide chain of the antibody constant region differs from the amino acid sequence of an IgG1 constant region by a Y349C substitution.

In some embodiments, the amino acid sequence of one polypeptide chain of the antibody constant region differs from the amino acid sequence of an IgG1 constant region by a Y349C substitution and wherein the amino acid sequence of the other polypeptide chain of the antibody constant region differs from the amino acid sequence of an IgG1 constant region by an S354C substitution.

In some embodiments, the amino acid sequence of one polypeptide chain of the antibody constant region differs from the amino acid sequence of an IgG1 constant region by K360E and K409W substitutions and wherein the amino acid sequence of the other polypeptide chain of the antibody constant region differs from the amino acid sequence of an IgG1 constant region by Q347R, D399V and F405T substitutions.

In some embodiments, the amino acid sequence of one polypeptide chain of the antibody constant region differs from the amino acid sequence of an IgG1 constant region by Q347R, D399V and F405T substitutions and wherein the amino acid sequence of the other polypeptide chain of the antibody constant region differs from the amino acid sequence of an IgG1 constant region by K360E and K409W substitutions.

In some embodiments, the amino acid sequence of one polypeptide chain of the antibody constant region differs from the amino acid sequence of an IgG1 constant region by a T366W substitutions and wherein the amino acid sequence of the other polypeptide chain of the antibody constant region differs from the amino acid sequence of an IgG1 constant region by T366S, T368A, and Y407V substitutions.

In some embodiments, the amino acid sequence of one polypeptide chain of the antibody constant region differs from the amino acid sequence of an IgG1 constant region by T366S, T368A, and Y407V substitutions and wherein the amino acid sequence of the other polypeptide chain of the antibody constant region differs from the amino acid sequence of an IgG1 constant region by a T366W substitution.

In some embodiments, the amino acid sequence of one polypeptide chain of the antibody constant region differs from the amino acid sequence of an IgG1 constant region by T350V, L351Y, F405A, and Y407V substitutions and wherein the amino acid sequence of the other polypeptide chain of the antibody constant region differs from the amino acid sequence of an IgG1 constant region by T350V, T366L, K392L, and T394W substitutions.

In some embodiments, the amino acid sequence of one polypeptide chain of the antibody constant region differs from the amino acid sequence of an IgG1 constant region by T350V, T366L, K392L, and T394W substitutions and wherein the amino acid sequence of the other polypeptide chain of the antibody constant region differs from the amino acid sequence of an IgG1 constant region by T350V, L351Y, F405A, and Y407V substitutions.

### *Exemplary multispecific binding proteins*

Listed below are examples of TriNKETs comprising an antigen-binding site that binds EGFR and an antigen-binding site that binds NKG2D each linked to an antibody constant region, wherein the antibody constant regions include mutations that enable heterodimerization of two Fc chains.

TriNKETs are contemplated in the F3 format, *i.e.,* the antigen-binding site that binds EGFR is a Fab, and the antigen-binding site that binds NKG2D is an scFv. All the TriNKETs shown *infra* are in the F3' format, *i.e.,* the antigen-binding site that binds EGFR is an scFv and the antigen-binding site that binds NKG2D is a Fab. In each TriNKET, the scFv may comprise substitution of Cys in the VH and VL regions, facilitating formation of a disulfide bridge between the VH and VL of the scFv.

The VH and VL of the scFv can be connected via a linker, *e.g.,* a peptide linker. In certain embodiments, the peptide linker is a flexible linker. Regarding the amino acid composition of the linker, peptides are selected with properties that confer flexibility, do not interfere with the structure and function of the other domains of the proteins of the present application, and resist cleavage from proteases. For example, glycine and serine residues generally provide protease resistance. In certain embodiments, the VL is linked N-terminal or C-terminal to the VH via a (GlyGlyGlyGlySer)$_4$ (($G_4S$)$_4$) linker (SEQ ID NO:119).

The length of the linker (*e.g.,* flexible linker) can be "short," *e.g.,* 0, 1, 2, 3, 4, 5, 6, 7, 8, 9, 10, 11 or 12 amino acid residues, or "long," *e.g.,* at least 13 amino acid residues. In certain embodiments, the linker is 10-50, 10-40, 10-30, 10-25, 10-20, 15-50, 15-40, 15-30, 15-25, 15-20, 20-50, 20-40, 20-30, or 20-25 amino acid residues in length.

In certain embodiments, the linker comprises or consists of a $(GS)_n$ (SEQ ID NO:120), $(GGS)_n$ (SEQ ID NO:121), $(GGGS)_n$ (SEQ ID NO:122), $(GGSG)_n$ (SEQ ID NO:123), $(GGSGG)_n$ (SEQ ID NO:124), and $(GGGGS)_n$ (SEQ ID NO:125) sequence, wherein n is 1, 2, 3, 4, 5, 6, 7, 8, 9, 10, 11, 12, 13, 14, 15, 16, 17, 18, 19, or 20. In certain embodiments, the linker comprises or consists of an amino acid sequence selected from SEQ ID NO:119, and 126-134, as listed in **Table 11.**

| Table 11 | |
|---|---|
| SEQ ID | Amino Acid Sequence |
| SEQ ID NO: 126 | GSGSGSGSGSGSGSGSGSGS |
| SEQ ID NO: 127 | GGSGGSGGSGGSGGSGGSGGSGGSGGSGGS |
| SEQ ID NO: 128 | GGGSGGGSGGGSGGGSGGGSGGGSGGGSGGGSGGSGGGSGGGS |
| SEQ ID NO: 129 | GGSGGGSGGGSGGGSGGGSGGGSGGGSGGGSGGGSGGGSGGGSG |
| SEQ ID NO:130 | GGSGGGGSGGGGSGGGGSGGGGSGGGGGSGGGGSGGGGSGGGGSGGGGSGG |
| SEQ ID NO:131 | GGGGSGGGGSGGGGSGGGGSGGGGSGGGGSGGGGSGGGGSGGGGSGGGGS |
| SEQ ID NO:119 | GGGGSGGGGSGGGGSGGGGS |
| SEQ ID NO:132 | GGGGSGGGGSGGGGS |
| SEQ ID NO:133 | GGGGSGGGGSGGGGSGGGGSGGGGSGGGGSGGGGSGGGGSGGGGSGGGGSGGGGSGGGGSGGGGSGGGGSGGGGSGGGGSGGGGSGGGGSGGGGSGGGGS |
| SEQ ID NO:134 | GGSGGGGSGGGGSGGGGSGGGGSGGGGSGGGGSGGGGSGGGGSGGGGSGGGGSGGGGSGGGGSGGGGSGGGGSGGGGSGGGGSGGGGSGGGGSGGGGSGGGGSGG |

In the F3'-TriNKETs, an EGFR-binding scFv is linked to the N-terminus of an Fc via a Gly-Ser linker. The Ala-Ser or Gly-Ser linker is included at the elbow hinge region sequence to balance between flexibility and optimal geometry. In certain embodiments, an additional sequence Thr-Lys-Gly can be added N-terminal or C-terminal to the Ala-Ser or Gly-Ser sequence at the hinge.

As used herein to describe these exemplary TriNKETs, an Fc includes an antibody hinge, CH2, and CH3. In each exemplary TriNKET, the Fc domain polypeptide linked to an scFv comprises the mutations of Q347R, D399V, and F405T, and the Fc domain polypeptide linked to a Fab comprises matching mutations K360E and K409W for forming a heterodimer. The Fc domain polypeptide linked to the scFv further includes an S354C substitution in the CH3 domain, which forms a disulfide bond with a Y349C substitution on the Fc linked to the Fab. These substitutions are bold in the sequences described in this subsection.

For example, a TriNKET described in the present disclosure is EGFR-TriNKET-2. EGFR-TriNKET-2 includes (a) an EGFR-scFv-2 (VL-VH) sequence provided in **Table 2,** in the orientation of VH positioned C-terminal to VL, linked to an Fc domain polypeptide and (b) an NKG2D-binding Fab fragment derived from A49MI including a heavy chain portion comprising a heavy chain variable domain and a CH1 domain, and a light chain portion comprising a light chain variable domain and a light chain constant domain, wherein the CH1 domain is connected to the Fc domain polypeptide. EGFR-TriNKET-2 includes three polypeptides: EGFR-scFv-2 (VL-VH)-Fc (SEQ ID NO:166), A49MI-VH-CH1-Fc (SEQ ID NO: 164), and A49MI-VL-CL (SEQ ID NO: 165).

<u>EGFR-scFv-2 (VL-VH)-Fc</u> (SEQ ID NO:166). Residues in **bold** indicate mutated residues in the Fc domain.

DIQMTQSPSSLSASVGDRVTITCQASQDISNYLNWYQQKPGKAPKLLIYDASNLETG
VPSRFSGSGSGTDFTFTISSLQPEDIATYYCQHFDHLPLAFGCGTKVEIK
GGGGSGGGGSGGGGSGGGGS
QVQLQESGPGLVKPSETLSLTCTVSGGSVSSGDYYWTWIRQSPGKCLEWIGHIYYSG
NTNYNPRLKSRLTISIDTSKTQFSLKLSSVTAADTAIYYCVRDRVTGAFDIWGQGTM
VTVSS
GS

DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREP**R**VYTLPP**C**RDELTKNQVSLTCLVKGFYPSDIAVEWESNGQPENN
YKTTPPVL**V**SDGSF**T**LYSKLTVDKSRWQQGNVFSCSVMHEALHNHYTQKSLSLSPG

*A49MI-VH-CH1-Fc* (SEQ ID NO: 164). Residues in **bold** indicate mutated residues in the Fc domain and <u>underlined</u> sequences indicate CDR sequences.

EVQLVESGGGLVKPGGSLRLSCAAS<u>GFTFSSY</u>SMNWVRQAPGKGLEWVSS<u>ISSSSSY</u>I
YYADSVKGRFTISRDNAKNSLYLQMNSLRAEDTAVYYCAR<u>GAPIGAAAGWFDP</u>WG
QGTLVTVSS
ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAP
ELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPR
EPQV**C**TLPPSRDELT**E**NQVSLTCLVKGFYPSDIAVEWESNGQPENNYKTTPPVLDSD
GSFFLYS**W**LTVDKSRWQQGNVFSCSVMHEALHNHYTQKSLSLSPG

*A49MI-VL-CL* (SEQ ID NO:165). <u>Underlined</u> sequences indicate CDR sequences.

DIQMTQSPSSVSASVGDRVTITC<u>RASQGISSWLA</u>WYQQKPGKAPKLLIY<u>AASSLQS</u>G
VPSRFSGSGSGTDFTLTISSLQPEDFATYYC<u>QQGVSFPRT</u>FGGGTKVEIK
RTVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

EGFR-scFv-2 (VL-VH)-Fc (SEQ ID NO:166) represents the full sequence of an EGFR-binding scFv linked to an Fc domain polypeptide via a hinge comprising Gly-Ser. The Fc domain polypeptide linked to the scFv includes Q347R, D399V, and F405T substitutions for heterodimerization and an S354C substitution for forming a disulfide bond with a Y349C substitution in A49MI-VH-CH1-Fc as described below. The scFv includes a heavy chain variable domain of EGFR-binder-2 in **Table 2** connected to the C-terminus of a light chain variable domain of EGFR-binder-2 in **Table 2** via a $(G_4S)_4$ linker (SEQ ID NO: 119), wherein the scFv further comprises substitution of Cys in the VH and VL regions at G44 and S100, respectively, thereby facilitating formation of a disulfide bridge between the VH and VL of the scFv.
A49MI-VH-CH1-Fc (SEQ ID NO:164) represents the heavy chain portion of the Fab fragment, which comprises a heavy chain variable domain of NKG2D-binding A49MI (SEQ ID NO:95) and a CH1 domain, connected to an Fc domain. The Fc domain polypeptide in A49MI-VH-CH1-Fc includes a Y349C substitution in the CH3 domain, which forms a disulfide bond with an S354C substitution on the Fc polypeptide in EGFR-scFv-2 (VL-VH)-Fc. In A49MI-VH-CH1-Fc, the Fc domain also includes K360E and K409W substitutions for heterodimerization with the Fc in EGFR-scFv-2 (VL-VH)-Fc.
A49MI-VL-CL (SEQ ID NO: 165) represents the light chain portion of the Fab fragment comprising a light chain variable domain of NKG2D-binding A49MI (SEQ ID NO:85) and a light chain constant domain.

In another example, a TriNKET described in the present disclosure is EGFR-TriNKET-3. EGFR-TriNKET-3 includes (a) an EGFR-scFv-3 (VL-VH) sequence provided in **Table 2,** in the orientation of VH positioned C-terminal to VL, linked to an Fc domain polypeptide and (b) an NKG2D-binding Fab fragment derived from A49MI including a heavy chain portion comprising a heavy chain variable domain and a CH1 domain, and a light chain portion comprising a light chain variable domain and a light chain constant domain, wherein the CH1 domain is connected to the Fc domain polypeptide. EGFR-TriNKET-3 includes three polypeptides: EGFR-scFv-3 (VL-VH)-Fc (SEQ ID NO:167), A49MI-VH-CH1-Fc (SEQ ID NO: 164), and A49MI-VL-CL (SEQ ID NO:165).

<u>EGFR-scFv-3 (VL-VH)-Fc</u> (SEQ ID NO:167). Residues in **bold** indicate mutated residues in the Fc domain.

DIQMTQSPSSLSASVGDRVTITCQASQDISNYLNWYQQKPGKAPKLLIYDASNLETG
VPSRFSGSGSGTDFTFTISSLQPEDIATYYCQHF**R**HLPLAFG**C**GTKVEIK
GGGGSGGGGSGGGGSGGGGS
QVQLQESGPGLVKPSETLSLTCTVSGGSVSSGDYYWTWIRQSPGK**C**LEWIGHIYYSG
NTNYNP**R**LKSRLTISIDTSKTQFSLKLSSVTAADTAIYYCVRDRVTGAFDIWGQGTM
VTVSS
GS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREP**R**VYTLPP**C**RDELTKNQVSLTCLVKGFYPSDIAVEWESNGQPENN
YKTTPPVL**V**SDGSF**T**LYSKLTVDKSRWQQGNVFSCSVMHEALHNHYTQKSLSLSPG

EGFR-scFv-3 (VL-VH)-Fc (SEQ ID NO:167) represents the full sequence of an EGFR-binding scFv linked to an Fc domain via a hinge comprising Gly-Ser. The Fc domain polypeptide linked to the scFv includes Q347R, D399V, and F405T substitutions for heterodimerization and an S354C substitution for forming a disulfide bond with a Y349C substitution in A49MI-VH-CH1-Fc as described below. The scFv includes a heavy chain variable domain of EGFR-binder-3 in **Table 2** connected to the C-terminus of a light chain variable domain of EGFR-binder-3 clone in **Table 2** via a $(G_4S)_4$ linker (SEQ ID NO: 119), wherein the scFv further comprises substitution of Cys in the VH and VL regions at G44 and S100, respectively, thereby facilitating formation of a disulfide bridge between the VH and VL of the scFv.

In another example, a TriNKET described in the present disclosure is EGFR-TriNKET-4. EGFR-TriNKET-4 includes (a) an EGFR-scFv-4 (VL-VH) sequence provided in **Table 2,** in the orientation of VH positioned C-terminal to VL, linked to an Fc domain polypeptide and (b) an NKG2D-binding Fab fragment derived from A49MI including a heavy chain portion comprising a heavy chain variable domain and a CH1 domain, and a light chain portion comprising a light chain variable domain and a light chain constant domain, wherein the CH1 domain is connected to the Fc domain polypeptide. EGFR-TriNKET-4 includes three polypeptides: EGFR-scFv-4 (VL-VH)-Fc (SEQ ID NO:168), A49MI-VH-CH1-Fc (SEQ ID NO: 164), and A49MI-VL-CL (SEQ ID NO:165).

<u>EGFR-scFv-4 (VL-VH)-Fc</u> (SEQ ID NO:168). Residues in **bold** indicate mutated residues in the Fc domain.

DIQMTQSPSSLSASVGDRVTITCQASQDISNYLNWYQQKPGKAPKLLIYDASNLETG
VPSRFSGSGSGTDFTFTISSLQPEDIATYYCQHFRHLPLAFGCGTKVEIK
GGGGSGGGGSGGGGSGGGGS
QVQLQESGPGLVKPSETLSLTCTVSGGSVSSGDYYWTWIRQSPGKCLEWIGHIYYSG
NTNYNPSLKSRLTISIDTSKTQFSLKLSSVTAADTAIYYCVRDRVTGAFDIWGQGTMV
TVSS
GS
DKTHTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREP**R**VYTLPP**C**RDELTKNQVSLTCLVKGFYPSDIAVEWESNGQPENN
YKTTPPVL**V**SDGSF**T**LYSKLTVDKSRWQQGNVFSCSVMHEALHNHYTQKSLSLSPG

EGFR-scFv-4 (VL-VH)-Fc (SEQ ID NO:168) represents the full sequence of an EGFR-binding scFv linked to an Fc domain polypeptide via a hinge comprising Gly-Ser. The Fc domain polypeptide linked to the scFv includes Q347R, D399V, and F405T substitutions for heterodimerization and an S354C substitution for forming a disulfide bond with a Y349C substitution in A49MI-VH-CH1-Fc as described below. The scFv includes a heavy chain variable domain of EGFR-binder-4 clone in **Table 2** connected to the C-terminus of a light chain variable domain of EGFR-binder-4 clone in **Table 2** via a $(G_4S)_4$ linker (SEQ ID NO: 119), wherein the scFv further comprises substitution of Cys in the VH and VL regions at G44 and S100, respectively, thereby facilitating formation of a disulfide bridge between the VH and VL of the scFv.

In certain embodiments, a TriNKET described in the present disclosure is identical to one of the exemplary TriNKETs described above that includes the EW-RVT Fc mutations, except that the Fc domain polypeptide linked to the NKG2D-binding Fab fragment comprises the substitutions of Q347R, D399V, and F405T, and the Fc domain polypeptide linked to EGFR binding scFv comprises matching substitutions K360E and K409W for forming a heterodimer. In certain embodiments, a TriNKET described in the present disclosure is identical to one of the exemplary TriNKETs described above that includes the KiH Fc mutations, except that the Fc domain polypeptide linked to the NKG2D-binding Fab fragment comprises the "hole" substitutions of T366S, L368A, and Y407V, and the Fc domain polypeptide linked to EGFR-binding scFv comprises the "knob" substitution of T366W for forming a heterodimer.

A skilled person in the art would appreciate that during production and/or storage of proteins, N-terminal glutamate (E) or glutamine (Q) can be cyclized to form a lactam (*e.g.,* spontaneously or catalyzed by an enzyme present during production and/or storage). Accordingly, in some embodiments where the N-terminal residue of an amino acid sequence of a polypeptide is E or Q, a corresponding amino acid sequence with the E or Q replaced with pyroglutamate is also contemplated herein.

A skilled person in the art would also appreciate that during protein production and/or storage, the C-terminal lysine (K) of a protein can be removed (*e.g.*, spontaneously or catalyzed by an enzyme present during production and/or storage). Such removal of K is often observed with proteins that comprise an Fc domain at its C-terminus. Accordingly, in some embodiments where the C-terminal residue of an amino acid sequence of a polypeptide (*e.g.,* an Fc domain sequence) is K, a corresponding amino acid sequence with the K removed is also contemplated herein.

The multispecific binding proteins described above can be made using recombinant DNA technology well known to a skilled person in the art. For example, a first nucleic acid sequence encoding the first immunoglobulin heavy chain can be cloned into a first expression vector; a second nucleic acid sequence encoding the second immunoglobulin heavy chain can be cloned into a second expression vector; a third nucleic acid sequence encoding the immunoglobulin light chain can be cloned into a third expression vector; and the first, second, and third expression vectors can be stably transfected together into host cells to produce the multimeric proteins.

To achieve the highest yield of the multispecific binding protein , different ratios of the first, second, and third expression vector can be explored to determine the optimal ratio for transfection into the host cells. After transfection, single clones can be isolated for cell bank generation using methods known in the art, such as limited dilution, ELISA, FACS, microscopy, or Clonepix.

Clones can be cultured under conditions suitable for bio-reactor scale-up and maintained expression of the multispecific binding protein . The multispecific binding proteins can be isolated and purified using methods known in the art including centrifugation, depth filtration, cell lysis, homogenization, freeze-thawing, affinity purification, gel filtration, ion exchange chromatography, hydrophobic interaction exchange chromatography, and mixed-mode chromatography.

## II. CHARACTERISTICS OF THE MULTISPECIFIC BINDING PROTEINS

The multispecific binding proteins described herein include an NKG2D-binding site, a tumor-associated antigen-binding site that binds EGFR, and an antibody Fc domain or a portion thereof sufficient to bind CD16, or an antigen-binding site that binds CD16. In some embodiments, the multispecific binding proteins contains an additional antigen-binding site that binds to the same tumor-associated antigen (EGFR), as exemplified in the F4-TriNKET format (*e.g.,* **FIGs. 2C and 2D**).

In some embodiments, the multispecific binding proteins display similar thermal stability to the corresponding monoclonal antibody, *i.e.,* a monoclonal antibody containing the same EGFR antigen-binding site as the one incorporated in the multispecific binding proteins.

In some embodiments, the multispecific binding proteins simultaneously bind to cells expressing NKG2D and/or CD16, such as NK cells, and cells expressing EGFR, such as certain tumor cells. Binding of the multispecific binding proteins to NK cells can enhance the activity of the NK cells toward destruction of EGFR expressing tumor cells. It has been reported that NK cells exhibit more potent cytotoxicity against target cells that are stressed (*see* Chan et al., (2014) Cell Death Differ. 21(1):5-14). Without wishing to be bound by theory, it is hypothesized that when NK cells are engaged to a population of cells by a TriNKET, the NK cells may selectively kill the target cells that are stressed (*e.g.*, malignant cells and cells in a tumor microenvironment). This mechanism could contribute to increased specificity and reduced toxicity of TriNKETs, making it possible to selectively clear the stressed cells even if expression of EGFR is not limited to the desired target cells.

In some embodiments, the multispecific binding proteins bind to EGFR with a similar affinity to the corresponding anti-EGFR monoclonal antibody (*i.e.,* a monoclonal antibody containing the same EGFR binding site as the one incorporated in the multispecific binding protein). In some embodiments, the multispecific binding proteins are more effective in mediating killing of the tumor cells expressing EGFR by immune effector cells (e.g., NK cells or CD8$^+$ T cells) than the corresponding monoclonal antibodies.

In certain embodiments, the multispecific binding proteins described herein, which include a binding site for EGFR, activate primary human NK cells when co-culturing with cells expressing EGFR. NK cell activation is marked by the increase in CD107a degranulation and IFN-γ cytokine production. Furthermore, compared to a corresponding anti-EGFR monoclonal antibody, the multispecific binding proteins show superior activation of human NK cells in the presence of cells expressing EGFR.

In some embodiments, the multispecific binding proteins described herein, which include a binding site for EGFR, enhance the activity of rested and/or IL-2-activated human NK cells when co-culturing with cells expressing EGFR.

In some embodiments, compared to the corresponding monoclonal antibody that binds to EGFR, the multispecific binding proteins offer an advantage in targeting tumor cells that express medium and low levels of EGFR.

In some embodiments, a multispecific binding protein disclosed herein that has monovalence to EGFR, for example, one in F3 or F3' format, has reduced or delayed skin-related toxicity compared to a corresponding anti-EGFR mAb that is bivalent.

In some embodiments, the bivalent F4 format of the TriNKETs (i.e., TriNKETs include an additional antigen-binding site that binds to EGFR) improves the avidity with which the TriNKETs bind to EGFR, which in effect stabilizes expression and maintenance of high levels of TriNKETs on the surface of the tumor cells. In some embodiments, the F4-TriNKETs mediate more potent killing of tumor cells than the corresponding F3-TriNKETs or F3'-TriNKETs.

## III. THERAPEUTIC APPLICATIONS

The present application provides methods for treating cancer using a multispecific binding protein described herein and/or a pharmaceutical composition described herein. The methods may be used to treat a variety of cancers expressing EGFR.

The therapeutic method can be characterized according to the cancer to be treated. The cancer to be treated can be characterized according to the presence of a particular antigen expressed on the surface of the cancer cell.

Cancers characterized by the expression of EGFR, include, without limitation, solid tumor cancers. For example, in certain embodiments, the cancer is head and neck cancer, colorectal cancer, non-small cell lung cancer, glioma, renal cell carcinoma, bladder cancer, cervical cancer, ovarian cancer, pancreatic cancer, or liver cancer. In certain embodiments, the cancer is lung cancer (including, but not limited to, small cell lung carcinoma or lung adenocarcinoma), breast cancer, breast invasive ductal carcinoma, kidney cancer, conventional glioblastoma multiforme, colon cancer, colon adenocarcinoma, gastric cancer, brain cancer, glioblastoma, bladder, head and neck cancers, ovarian cancer or prostate cancer.

It is contemplated that the protein, conjugate, cells, and/or the pharmaceutical compositions described herein can be used to treat a variety of cancers, not limited to cancers in which the cancer cells or the cells in the cancer microenvironment express EGFR.

In certain embodiments, the cancer is a solid tumor. In certain other embodiments, the cancer is brain cancer, bladder cancer, breast cancer, cervical cancer, colon cancer, colorectal cancer, endometrial cancer, esophageal cancer, leukemia, lung cancer, liver cancer, melanoma, ovarian cancer, pancreatic cancer, prostate cancer, rectal cancer, renal cancer, stomach cancer, testicular cancer, or uterine cancer. In yet other embodiments, the cancer is a vascularized tumor, squamous cell carcinoma, adenocarcinoma, small cell carcinoma, melanoma, glioma, neuroblastoma, sarcoma (e.g., an angiosarcoma or chondrosarcoma), larynx cancer, parotid cancer, biliary tract cancer, thyroid cancer, acral lentiginous melanoma, actinic keratoses, acute lymphocytic leukemia, acute myeloid leukemia, adenoid cystic carcinoma, adenomas, adenosarcoma, adenosquamous carcinoma, anal canal cancer, anal cancer, anorectum cancer, astrocytic tumor, Bartholin gland carcinoma, basal cell carcinoma, biliary cancer, bone cancer, bone marrow cancer, bronchial cancer, bronchial gland carcinoma, carcinoid, cholangiocarcinoma, chondrosarcoma, choroid plexus papilloma/carcinoma, chronic lymphocytic leukemia, chronic myeloid leukemia, clear cell carcinoma, connective tissue cancer, cystadenoma, digestive system cancer, duodenum cancer, endocrine system cancer, endodermal sinus tumor, endometrial hyperplasia, endometrial stromal sarcoma, endometrioid adenocarcinoma, endothelial cell cancer, ependymal cancer, epithelial cell cancer, Ewing's sarcoma, eye and orbit cancer, female genital cancer, focal nodular hyperplasia, gallbladder cancer, gastric antrum cancer, gastric fundus cancer, gastrinoma, glioblastoma, glucagonoma, heart cancer, hemangioblastomas, hemangioendothelioma, hemangiomas, hepatic adenoma, hepatic adenomatosis, hepatobiliary cancer, hepatocellular carcinoma, Hodgkin's disease, ileum cancer, insulinoma, intraepithelial neoplasia, intraepithelial squamous cell neoplasia, intrahepatic bile duct cancer, invasive squamous cell carcinoma, jejunum cancer, joint cancer, Kaposi's sarcoma, pelvic cancer, large cell carcinoma, large intestine cancer, leiomyosarcoma, lentigo maligna melanomas, lymphoma, male genital cancer, malignant melanoma, malignant mesothelial tumors, medulloblastoma, medulloepithelioma, meningeal cancer, mesothelial cancer, metastatic carcinoma, mouth cancer, mucoepidermoid carcinoma, multiple myeloma, muscle cancer, nasal tract cancer, nervous system cancer, neuroepithelial adenocarcinoma nodular melanoma, non-epithelial skin cancer, non-Hodgkin's lymphoma, oat cell carcinoma, oligodendroglial cancer, oral cavity cancer, osteosarcoma, papillary serous adenocarcinoma, penile cancer, pharynx cancer, pituitary tumors, plasmacytoma, pseudosarcoma, pulmonary blastoma, rectal cancer, renal cell carcinoma, respiratory system cancer, retinoblastoma, rhabdomyosarcoma, sarcoma, serous carcinoma, sinus cancer, skin cancer, small cell carcinoma, small intestine cancer, smooth muscle cancer, soft tissue cancer, somatostatinsecreting tumor, spine cancer, squamous cell carcinoma, striated muscle cancer, submesothelial cancer, superficial spreading melanoma, T cell leukemia, tongue cancer, undifferentiated carcinoma, ureter cancer, urethra cancer, urinary bladder cancer, urinary system cancer, uterine cervix cancer, uterine corpus cancer, uveal melanoma, vaginal cancer, verrucous carcinoma, VIPoma, vulva cancer, well differentiated carcinoma, or Wilms tumor.

In certain other embodiments, the cancer is non-Hodgkin's lymphoma, such as a B-cell lymphoma or a T-cell lymphoma. In certain embodiments, the non-Hodgkin's lymphoma is a B-cell lymphoma, such as a diffuse large B-cell lymphoma, primary mediastinal B-cell lymphoma, follicular lymphoma, small lymphocytic lymphoma, mantle cell lymphoma, marginal zone B-cell lymphoma, extranodal marginal zone B-cell lymphoma, nodal marginal zone B-cell lymphoma, splenic marginal zone B-cell lymphoma, Burkitt lymphoma, lymphoplasmacytic lymphoma, hairy cell leukemia, or primary central nervous system (CNS) lymphoma. In certain other embodiments, the non-Hodgkin's lymphoma is a T-cell lymphoma, such as a precursor T-lymphoblastic lymphoma, peripheral T-cell lymphoma, cutaneous T-cell lymphoma, angioimmunoblastic T-cell lymphoma, extranodal natural killer/T-cell lymphoma, enteropathy type T-cell lymphoma, subcutaneous panniculitis-like T-cell lymphoma, anaplastic large cell lymphoma, or peripheral T-cell lymphoma.

The cancer to be treated can be characterized according to the presence of a particular antigen expressed on the surface of the cancer cell. In certain embodiments, the cancer cell can express one or more of the following in addition to EGFR: CD2, CD19, CD38, CD40, CD52, CD30, CD70, IGF1R, HER3/ERBB3, HER4/ERBB4, MUC1, TROP2, cMET, SLAMF7, PSCA, MICA, MICB, TRAILR1, TRAILR2, MAGE-A3, B7.1, B7.2, CTLA4, and PD1.

## IV. COMBINATION THERAPY

Another aspect of the present application provides for combination therapy. A multispecific binding protein described herein can be used in combination with additional therapeutic agents to treat cancer.

Exemplary therapeutic agents that may be used as part of a combination therapy in treating cancer include, for example, radiation, mitomycin, tretinoin, ribomustin, gemcitabine, vincristine, etoposide, cladribine, mitobronitol, methotrexate, doxorubicin, carboquone, pentostatin, nitracrine, zinostatin, cetrorelix, letrozole, raltitrexed, daunorubicin, fadrozole, fotemustine, thymalfasin, sobuzoxane, nedaplatin, cytarabine, bicalutamide, vinorelbine, vesnarinone, aminoglutethimide, amsacrine, proglumide, elliptinium acetate, ketanserin, doxifluridine, etretinate, isotretinoin, streptozocin, nimustine, vindesine, flutamide, drogenil, butocin, carmofur, razoxane, sizofilan, carboplatin, mitolactol, tegafur, ifosfamide, prednimustine, picibanil, levamisole, teniposide, improsulfan, enocitabine, lisuride, oxymetholone, tamoxifen, progesterone, mepitiostane, epitiostanol, formestane, interferon-alpha, interferon-2 alpha, interferon-beta, interferon-gamma (IFN-γ), colony stimulating factor-1, colony stimulating factor-2, denileukin diftitox, interleukin-2, luteinizing hormone releasing factor and variations of the aforementioned agents that may exhibit differential binding to its cognate receptor, or increased or decreased serum half-life.

An additional class of agents that may be used as part of a combination therapy in treating cancer is immune checkpoint inhibitors. Exemplary immune checkpoint inhibitors include agents that inhibit one or more of (i) cytotoxic T lymphocyte-associated antigen 4 (CTLA4), (ii) programmed cell death protein 1 (PD1), (iii) PDL1, (iv) LAG3, (v) B7-H3, (vi) B7-H4, and (vii) TIM3. The CTLA4 inhibitor ipilimumab has been approved by the United States Food and Drug Administration for treating melanoma.

Yet other agents that may be used as part of a combination therapy in treating cancer are monoclonal antibody agents that target non-checkpoint targets (e.g., herceptin) and non-cytotoxic agents (e.g., tyrosine-kinase inhibitors).

Yet other categories of anti-cancer agents include, for example: (i) an inhibitor selected from an ALK Inhibitor, an ATR Inhibitor, an A2A Antagonist, a Base Excision Repair Inhibitor, a Bcr-Abl Tyrosine Kinase Inhibitor, a Bruton's Tyrosine Kinase Inhibitor, a CDC7 Inhibitor, a CHK1 Inhibitor, a Cyclin-Dependent Kinase Inhibitor, a DNA-PK Inhibitor, an Inhibitor of both DNA-PK and mTOR, a DNMT1 Inhibitor, a DNMT1 Inhibitor plus 2-chloro-deoxyadenosine, an HDAC Inhibitor, a Hedgehog Signaling Pathway Inhibitor, an IDO Inhibitor, a JAK Inhibitor, a mTOR Inhibitor, a MEK Inhibitor, a MELK Inhibitor, a MTH1 Inhibitor, a PARP Inhibitor, a Phosphoinositide 3-Kinase Inhibitor, an Inhibitor of both PARP1 and DHODH, a Proteasome Inhibitor, a Topoisomerase-II Inhibitor, a Tyrosine Kinase Inhibitor, a VEGFR Inhibitor, and a WEE1 Inhibitor; (ii) an agonist of OX40, CD137, CD40, GITR, CD27, HVEM, TNFRSF25, or ICOS; and (iii) a cytokine selected from IL-12, IL-15, GM-CSF, and G-CSF.

Proteins of the present application can also be used as an adjunct to surgical removal of the primary lesion.

The amount of multispecific binding protein and additional therapeutic agent and the relative timing of administration may be selected in order to achieve a desired combined therapeutic effect. For example, when administering a combination therapy to a patient in need of such administration, the therapeutic agents in the combination, or a pharmaceutical composition or compositions comprising the therapeutic agents, may be administered in any order such as, for example, sequentially, concurrently, together, simultaneously and the like. Further, for example, a multispecific binding protein may be administered during a time when the additional therapeutic agent(s) exerts its prophylactic or therapeutic effect, or *vice versa.*

## V. PHARMACEUTICAL COMPOSITIONS

The present disclosure also features pharmaceutical compositions that contain a therapeutically effective amount of a protein described herein. The composition can be formulated for use in a variety of drug delivery systems. One or more physiologically acceptable excipients or carriers can also be included in the composition for proper formulation. Suitable formulations for use in compositions disclosed herein are found in Remington's Pharmaceutical Sciences, Mack Publishing Company, Philadelphia, Pa., 17th ed., 1985. For a brief review of methods for drug delivery, *see, e.g.,* Langer (Science 249:1527-1533, 1990).

In certain embodiments, the composition may be a drug delivery formulation. The intravenous drug delivery formulation described herein may be contained in a bag, a pen, or a syringe. In certain embodiments, the bag may be connected to a channel comprising a tube and/or a needle. In certain embodiments, the formulation may be a lyophilized formulation or a liquid formulation. In certain embodiments, the formulation may be freeze-dried (lyophilized) and contained in about 12-60 vials. In certain embodiments, the formulation may be freeze-dried and 45 mg of the freeze-dried formulation may be contained in one vial. In certain embodiments, the about 40 mg to about 100 mg of freeze-dried formulation may be contained in one vial. In certain embodiments, freeze-dried formulation from 12, 27, or 45 vials are combined to obtain a therapeutic dose of the protein in the intravenous drug formulation. In certain embodiments, the formulation may be a liquid formulation and stored as about 250 mg/vial to about 1000 mg/vial. In certain embodiments, the formulation may be a liquid formulation and stored as about 600 mg/vial. In certain embodiments, the formulation may be a liquid formulation and stored as about 250 mg/vial.

These compositions may be sterilized by conventional sterilization techniques, or may be sterile filtered. The resulting aqueous solutions may be packaged for use as-is, or lyophilized, the lyophilized preparation being combined with a sterile aqueous carrier prior to administration. The pH of the preparations typically will be between 3 and 11, for example, between 5 and 9 or between 6 and 8, and in certain embodiments between 7 and 8, such as 7 to 7.5. The resulting compositions in solid form may be packaged in multiple single dose units, each containing a fixed amount of the above-mentioned agent or agents. The composition in solid form can also be packaged in a container for a flexible quantity.

In certain embodiments, the present disclosure provides a formulation with an extended shelf life including the protein described in the present disclosure, in combination with mannitol, citric acid monohydrate, sodium citrate, disodium phosphate dihydrate, sodium dihydrogen phosphate dihydrate, sodium chloride, polysorbate 80, water, and sodium hydroxide.

In certain embodiments, an aqueous formulation is prepared including a protein described in the present disclosure in a pH-buffered solution. The buffer of the present application may have a pH ranging from about 4 to about 8, *e.g.,* from about 4.5 to about 6.0, or from about 4.8 to about 5.5, or may have a pH of about 5.0 to about 5.2. Ranges intermediate to the above recited pHs are also intended to be part of this disclosure. For example, ranges of values using a combination of any of the above recited values as upper and/or lower limits are intended to be included. Examples of buffers that will control the pH within this range include acetate (e.g., sodium acetate), succinate (such as sodium succinate), gluconate, histidine, citrate and other organic acid buffers.

In certain embodiments, the formulation includes a buffer system which contains citrate and phosphate to maintain the pH in a range of about 4 to about 8. In certain embodiments the pH range may be from about 4.5 to about 6.0, or from about pH 4.8 to about 5.5, or in a pH range of about 5.0 to about 5.2. In certain embodiments, the buffer system includes citric acid monohydrate, sodium citrate, disodium phosphate dihydrate, and/or sodium dihydrogen phosphate dihydrate. In certain embodiments, the buffer system includes about 1.3 mg/mL of citric acid (*e.g.,* 1.305 mg/mL), about 0.3 mg/mL of sodium citrate (*e.g.,* 0.305 mg/mL), about 1.5 mg/mL of disodium phosphate dihydrate (*e.g.,* 1.53 mg/mL), about 0.9 mg/mL of sodium dihydrogen phosphate dihydrate (*e.g.,* 0.86 mg/mL), and about 6.2 mg/mL of sodium chloride (*e.g.,* 6.165 mg/mL). In certain embodiments, the buffer system includes about 1 to about 1.5 mg/mL of citric acid, about 0.25 to about 0.5 mg/mL of sodium citrate, about 1.25 to about 1.75 mg/mL of disodium phosphate dihydrate, about 0.7 to about 1.1 mg/mL of sodium dihydrogen phosphate dihydrate, and about 6.0 to about 6.4 mg/mL of sodium chloride. In certain embodiments, the pH of the formulation is adjusted with sodium hydroxide.

A polyol, which acts as a tonicifier and may stabilize the antibody, may also be included in the formulation. The polyol is added to the formulation in an amount which may vary with respect to the desired isotonicity of the formulation. In certain embodiments, the aqueous formulation may be isotonic. The amount of polyol added may also be altered with respect to the molecular weight of the polyol. For example, a lower amount of a monosaccharide (*e.g.*, mannitol) may be added, compared to a disaccharide (such as trehalose). In certain embodiments, the polyol which may be used in the formulation as a tonicity agent is mannitol. In certain embodiments, the mannitol concentration may be about 5 to about 20 mg/mL. In certain embodiments, the concentration of mannitol may be about 7.5 to about 15 mg/mL. In certain embodiments, the concentration of mannitol may be about 10 to about 14 mg/mL. In certain embodiments, the concentration of mannitol may be about 12 mg/mL. In certain embodiments, the polyol sorbitol may be included in the formulation.

A detergent or surfactant may also be added to the formulation. Exemplary detergents include nonionic detergents such as polysorbates (*e.g.*, polysorbates 20, 80 etc.) or poloxamers (*e.g.*, poloxamer 188). The amount of detergent added is such that it reduces aggregation of the formulated antibody and/or minimizes the formation of particulates in the formulation and/or reduces adsorption. In certain embodiments, the formulation may include a surfactant which is a polysorbate. In certain embodiments, the formulation may contain the detergent polysorbate 80 or Tween 80. Tween 80 is a term used to describe polyoxyethylene (20) sorbitanmonooleate (*see* Fiedler, Lexikon der Hifsstoffe, Editio Cantor Verlag Aulendorf, 4th ed., 1996). In certain embodiments, the formulation may contain between about 0.1 mg/mL and about 10 mg/mL of polysorbate 80, or between about 0.5 mg/mL and about 5 mg/mL. In certain embodiments, about 0.1% polysorbate 80 may be added in the formulation.

In embodiments, the protein product described herein is formulated as a liquid formulation. The liquid formulation may be presented at a 10 mg/mL concentration in either a USP / Ph Eur type I 50R vial closed with a rubber stopper and sealed with an aluminum crimp seal closure. The stopper may be made of elastomer complying with USP and Ph Eur. In certain embodiments vials may be filled with 61.2 mL of the protein product solution in order to allow an extractable volume of 60 mL. In certain embodiments, the liquid formulation may be diluted with 0.9% saline solution.

In certain embodiments, the liquid formulation disclosed herein may be prepared as a 10 mg/mL concentration solution in combination with a sugar at stabilizing levels. In certain embodiments the liquid formulation may be prepared in an aqueous carrier. In certain embodiments, a stabilizer may be added in an amount no greater than that which may result in a viscosity undesirable or unsuitable for intravenous administration. In certain embodiments, the sugar may be disaccharides, *e.g.*, sucrose. In certain embodiments, the liquid formulation may also include one or more of a buffering agent, a surfactant, and a preservative.

In certain embodiments, the pH of the liquid formulation may be set by addition of a pharmaceutically acceptable acid and/or base. In certain embodiments, the pharmaceutically acceptable acid may be hydrochloric acid. In certain embodiments, the base may be sodium hydroxide.

In addition to aggregation, deamidation is a common product variant of peptides and proteins that may occur during fermentation, harvest/cell clarification, purification, drug substance/drug product storage and during sample analysis. Deamidation is the loss of $NH_3$ from a protein forming a succinimide intermediate that can undergo hydrolysis. The succinimide intermediate results in a 17 dalton mass decrease of the parent peptide. The subsequent hydrolysis results in an 18 dalton mass increase. Isolation of the succinimide intermediate is difficult due to instability under aqueous conditions. As such, deamidation is typically detectable as 1 dalton mass increase. Deamidation of an asparagine results in either aspartic or isoaspartic acid. The parameters affecting the rate of deamidation include pH, temperature, solvent dielectric constant, ionic strength, primary sequence, local polypeptide conformation and tertiary structure. The amino acid residues adjacent to Asn in the peptide chain affect deamidation rates. Gly and Ser following an Asn in protein sequences results in a higher susceptibility to deamidation.

In certain embodiments, the liquid formulation described herein may be preserved under conditions of pH and humidity to prevent deamination of the protein product.

The aqueous carrier of interest herein is one which is pharmaceutically acceptable (safe and non-toxic for administration to a human) and is useful for the preparation of a liquid formulation. Illustrative carriers include sterile water for injection (SWFI), bacteriostatic water for injection (BWFI), a pH buffered solution (*e.g.*, phosphate-buffered saline), sterile saline solution, Ringer's solution or dextrose solution.

A preservative may be optionally added to the formulations herein to reduce bacterial action. The addition of a preservative may, for example, facilitate the production of a multi-use (multiple-dose) formulation.

Intravenous (IV) delivery may be an administration route in particular instances, such as when a patient is in the hospital after transplantation receiving all drugs via the IV route. In certain embodiments, the liquid formulation is diluted with 0.9% Sodium Chloride solution before administration. In certain embodiments, the diluted drug product for injection is isotonic and suitable for administration by intravenous infusion.

In certain embodiments, a salt or buffer components may be added in an amount of 10 mM - 200 mM. The salts and/or buffers are pharmaceutically acceptable and are derived from various known acids (inorganic and organic) with "base forming" metals or amines. In certain embodiments, the buffer may be phosphate buffer. In certain embodiments, the buffer may be glycinate, carbonate, citrate buffers, in which case, sodium, potassium or ammonium ions can serve as counterion.

A preservative may be optionally added to the formulations herein to reduce bacterial action. The addition of a preservative may, for example, facilitate the production of a multi-use (multiple-dose) formulation.

The aqueous carrier of interest herein is one which is pharmaceutically acceptable (safe and non-toxic for administration to a human) and is useful for the preparation of a liquid formulation. Illustrative carriers include sterile water for injection (SWFI), bacteriostatic water for injection (BWFI), a pH buffered solution (*e.g.*, phosphate-buffered saline), sterile saline solution, Ringer's solution or dextrose solution.

The protein described herein could exist in a lyophilized formulation including the proteins and a lyoprotectant. The lyoprotectant may be sugar, *e.g.*, disaccharides. In certain embodiments, the lyoprotectant may be sucrose or maltose. The lyophilized formulation may also include one or more of a buffering agent, a surfactant, a bulking agent, and/or a preservative.

The amount of sucrose or maltose useful for stabilization of the lyophilized drug product may be in a weight ratio of at least 1:2 protein to sucrose or maltose. In certain embodiments, the protein to sucrose or maltose weight ratio may be of from 1:2 to 1:5.

In certain embodiments, the pH of the formulation, prior to lyophilization, may be set by addition of a pharmaceutically acceptable acid and/or base. In certain embodiments the pharmaceutically acceptable acid may be hydrochloric acid. In certain embodiments, the pharmaceutically acceptable base may be sodium hydroxide.

Before lyophilization, the pH of the solution containing the protein described herein may be adjusted between 6 to 8. In certain embodiments, the pH range for the lyophilized drug product may be from 7 to 8.

In certain embodiments, a salt or buffer component may be added in an amount of 10 mM - 200 mM. The salts and/or buffers are pharmaceutically acceptable and are derived from various known acids (inorganic and organic) with "base forming" metals or amines. In certain embodiments, the buffer may be phosphate buffer. In certain embodiments, the buffer may be glycinate, carbonate, citrate buffers, in which case, sodium, potassium or ammonium ions can serve as counterion.

In certain embodiments, a "bulking agent" may be added. A "bulking agent" is a compound which adds mass to a lyophilized mixture and contributes to the physical structure of the lyophilized cake (*e.g.*, facilitates the production of an essentially uniform lyophilized cake which maintains an open pore structure). Illustrative bulking agents include mannitol, glycine, polyethylene glycol and sorbitol. The lyophilized formulations of the present application may contain such bulking agents.

A preservative may be optionally added to the formulations herein to reduce bacterial action. The addition of a preservative may, for example, facilitate the production of a multi-use (multiple-dose) formulation.

In certain embodiments, the lyophilized drug product may be reconstituted with an aqueous carrier. The aqueous carrier of interest herein is one which is pharmaceutically acceptable (*e.g.*, safe and non-toxic for administration to a human) and is useful for the preparation of a liquid formulation, after lyophilization. Illustrative aqueous carriers include sterile water for injection (SWFI), bacteriostatic water for injection (BWFI), a pH buffered solution (*e.g.*, phosphate-buffered saline), sterile saline solution, Ringer's solution or dextrose solution.

In certain embodiments, the lyophilized drug product disclosed herein is reconstituted with either Sterile Water for Injection, USP (SWFI) or 0.9% Sodium Chloride Injection, USP. During reconstitution, the lyophilized powder dissolves into a solution.

In certain embodiments, the lyophilized protein product disclosed herein is reconstituted to about 4.5 mL water for injection and diluted with 0.9% saline solution (sodium chloride solution).

Actual dosage levels of the active ingredients in the pharmaceutical compositions described herein may be varied so as to obtain an amount of the active ingredient which is effective to achieve the desired therapeutic response for a particular patient, composition, and mode of administration, without being toxic to the patient.

The specific dose can be a uniform dose for each patient, for example, 50-5000 mg of protein. Alternatively, a patient's dose can be tailored to the approximate body weight or surface area of the patient. Other factors in determining the appropriate dosage can include the disease or condition to be treated or prevented, the severity of the disease, the route of administration, and the age, sex and medical condition of the patient. Further refinement of the calculations necessary to determine the appropriate dosage for treatment is routinely made by those skilled in the art, especially in light of the dosage information and assays disclosed herein. The dosage can also be determined through the use of known assays for determining dosages used in conjunction with appropriate dose-response data. An individual patient's dosage can be adjusted as the progress of the disease is monitored. Blood levels of the targetable construct or complex in a patient can be measured to see if the dosage needs to be adjusted to reach or maintain an effective concentration. Pharmacogenomics may be used to determine which targetable constructs and/or complexes, and dosages thereof, are most likely to be effective for a given individual (Schmitz et al., Clinica Chimica Acta 308: 43-53, 2001; Steimer et al., Clinica Chimica Acta 308: 33-41, 2001).

In general, dosages based on body weight are from about 0.01 $\mu$g to about 100 mg per kg of body weight, such as about 0.01 $\mu$g to about 100 mg/kg of body weight, about 0.01 $\mu$g to about 50 mg/kg of body weight, about 0.01 $\mu$g to about 10 mg/kg of body weight, about 0.01 $\mu$g to about 1 mg/kg of body weight, about 0.01 $\mu$g to about 100 $\mu$g/kg of body weight, about 0.01 $\mu$g to about 50 $\mu$g/kg of body weight, about 0.01 $\mu$g to about 10 $\mu$g/kg of body weight, about 0.01 $\mu$g to about 1 $\mu$g/kg of body weight, about 0.01 $\mu$g to about 0.1 $\mu$g/kg of body weight, about 0.1 $\mu$g to about 100 mg/kg of body weight, about 0.1 $\mu$g to about 50 mg/kg of body weight, about 0.1 $\mu$g to about 10 mg/kg of body weight, about 0.1 $\mu$g to about 1

mg/kg of body weight, about 0.1 $\mu$g to about 100 $\mu$g/kg of body weight, about 0.1 $\mu$g to about 10 $\mu$g/kg of body weight, about 0.1 $\mu$g to about 1 $\mu$g/kg of body weight, about 1 $\mu$g to about 100 mg/kg of body weight, about 1 $\mu$g to about 50 mg/kg of body weight, about 1 $\mu$g to about 10 mg/kg of body weight, about 1 $\mu$g to about 1 mg/kg of body weight, about 1 $\mu$g to about 100 $\mu$g/kg of body weight, about 1 $\mu$g to about 50 $\mu$g/kg of body weight, about 1 $\mu$g to about 10 $\mu$g/kg of body weight, about 10 $\mu$g to about 100 mg/kg of body weight, about 10 $\mu$g to about 50 mg/kg of body weight, about 10 $\mu$g to about 10 mg/kg of body weight, about 10 $\mu$g to about 1 mg/kg of body weight, about 10 $\mu$g to about 100 $\mu$g/kg of body weight, about 10 $\mu$g to about 50 $\mu$g/kg of body weight, about 50 $\mu$g to about 100 mg/kg of body weight, about 50 $\mu$g to about 50 mg/kg of body weight, about 50 $\mu$g to about 10 mg/kg of body weight, about 50 $\mu$g to about 1 mg/kg of body weight, about 50 $\mu$g to about 100 $\mu$g/kg of body weight, about 100 $\mu$g to about 100 mg/kg of body weight, about 100 $\mu$g to about 50 mg/kg of body weight, about 100 $\mu$g to about 10 mg/kg of body weight, about 100 $\mu$g to about 1 mg/kg of body weight, about 1 mg to about 100 mg/kg of body weight, about 1 mg to about 50 mg/kg of body weight, about 1 mg to about 10 mg/kg of body weight, about 10 mg to about 100 mg/kg of body weight, about 10 mg to about 50 mg/kg of body weight, about 50 mg to about 100 mg/kg of body weight.

Doses may be given once or more times daily, weekly, monthly or yearly, or even once every 2 to 20 years. Persons of ordinary skill in the art can easily estimate repetition rates for dosing based on measured residence times and concentrations of the targetable construct or complex in bodily fluids or tissues. Administration of the compositions described herein could be intravenous, intraarterial, intraperitoneal, intramuscular, subcutaneous, intrapleural, intrathecal, intracavitary, by perfusion through a catheter or by direct intralesional injection. Administrations may be one or more times daily, one or more times weekly, one or more times monthly, or one or more times annually.

The description above describes multiple aspects and embodiments of the present application. The patent application specifically contemplates all combinations and permutations of the aspects and embodiments.

EXAMPLES

The following examples are merely illustrative and are not intended to limit the scope or content of the multispecific binding proteins described in the present application in any way.

### Example 1. Selection of EGFR-binding interface using SPR and DSC Analysis

This example was designed to develop EGFR-binding sites derived from panitumumab that have improved thermostability than panitumumab. Briefly, 25 constructs, each containing a single point mutation, selected based on the crystal structure of panitumumab Fab in complex with the D3 domain of EGFR (PDB ID: 5SX4), were designed. Other than three constructs that did not express well, the EGFR-binding sites were produced to evaluate the mutation's impact on binding affinity and thermostability. Each scFv construct also contained G44C substitution in the VH and G100C substitution in the VL. Kinetics and affinity for human and Rhesus macaque EGFR was evaluated using surface plasmon resonance (SPR). The results are shown in **Table** 12. Given that the impact for each mutation was minute, those that caused marked reduction in affinity were removed from consideration. Thermostability of eight constructs was further assessed by Differential Scanning Fluorimetry (DSF) using the method described in Example 3. The results are shown in **Table 13.**

**Table 12.** SPR analysis of the 22 anti-EGFR constructs with single point mutations. *-H-denotes mutation in the heavy chain; -L- denotes mutation in the light chain; and PANI denotes the panitumumab framework.

| | | Human EGFR | Rhesus EGFR | Human EGFR | Rhesus EGFR | Human EGFR | Rhesus EGFR |
|---|---|---|---|---|---|---|---|
| Expected Function of Design | Name* | $k_a$ (1/Ms) | $k_a$ (1/Ms) | $k_d$ (1/s) | $k_d$ (1/s) | $K_D$ (M) | $K_D$ (M) |
| 2nd pair of disulfide bond | PANI-G106C-A43C | No binding | | | | | |
| Stability | PANI-H-S62R | 5.12E+0 5 | 4.36E+05 | 1.75E-03 | 1.78E-03 | 3.41E-09 | 4.08E-09 |
| Stability | PANI-H-S62K | 5.11E+0 5 | 4.35E+05 | 1.29E-03 | 1.34E-03 | 2.52E-09 | 3.08E-09 |
| Stability | PANI-H-D101T | 1.14E+0 5 | 1.17E+05 | 4.37E-03 | 5.37E-03 | 3.85E-08 | 4.60E-08 |

(continued)

| Expected Function of Design | Name* | Human EGFR $k_a$ (1/Ms) | Rhesus EGFR $k_a$ (1/Ms) | Human EGFR $k_d$ (1/s) | Rhesus EGFR $k_d$ (1/s) | Human EGFR $K_D$ (M) | Rhesus EGFR $K_D$ (M) |
|---|---|---|---|---|---|---|---|
| Stability | PANI-H-D101S | 2.86E+0 5 | 3.49E+05 | 3.37E-03 | 4.73E-03 | 1.18E-08 | 1.35E-08 |
| Stability | PANI-H-D101N | No binding | | | | | |
| Stability | PANI-H-D101Q | No binding | | | | | |
| Stability | PANI-L-F87Y | 5.22E+0 5 | 3.28E+05 | 8.74E-04 | 9.96E-04 | 1.68E-09 | 3.03E-09 |
| Stability | PANI-L-L96Q | 6.90E+0 5 | 5.05E+05 | 8.32E-03 | 8.98E-03 | 1.21E-08 | 1.78E-08 |
| Stability | PANI-L-L96N | 5.34E+0 5 | 3.48E+05 | 3.24E-03 | 3.75E-03 | 6.07E-09 | 1.08E-08 |
| Stability | PANI-L-L96E | No binding | | | | | |
| Stability | PANI-L-L96D | 3.15E+0 5 | 2.54E+05 | 6.62E-03 | 8.08E-03 | 2.10E-08 | 3.18E-08 |
| Affinity | PANI-H-S54Q | No binding | Not tested | No binding | Not tested | No binding | Not tested |
| Affinity | PANI-H-S54N | 5.75E+0 5 | 4.22E+05 | 4.68E-03 | 5.65E-03 | 8.12E-09 | 1.34E-08 |
| Affinity | PANI-H-S54E | No binding | | | | | |
| Affinity | PANI-H-S54D | No binding | | | | | |
| Affinity | PANI-H-T98Q | 3.90E+0 5 | 2.65E+05 | 5.84E-03 | 6.90E-03 | 1.50E-08 | 2.61E-08 |
| Affinity | PANI-H-T98N | 7.46E+0 5 | 5.00E+05 | 4.21E-02 | 2.00E-02 | 5.64E-08 | 4.00E-08 |
| Affinity | PANI-H-T98E | 4.53E+0 5 | 3.77E+05 | 3.31E-03 | 3.53E-03 | 7.32E-09 | 9.35E-09 |
| Affinity | PANI-H-T98D | 6.07E+0 5 | 5.18E+05 | 8.30E-03 | 9.62E-03 | 1.37E-08 | 1.86E-08 |
| Affinity / Stability | PANI-L-D92R | 2.56E+0 5 | 1.89E+05 | 8.39E-04 | 9.60E-04 | 3.27E-09 | 5.09E-09 |
| Control | Original Pani-scFv | 5.50E+0 5 | 3.67E+05 | 9.59E-04 | 1.08E-03 | 1.74E-09 | 2.95E-09 |

**Table 13.** DSF analysis 8 anti-EGFR constructs with single point mutations. *-H- denotes mutation in the heavy chain; -L- denotes mutation in the light chain; and PANI denotes the panitumumab framework.

| Expected Function of Design | Name* | $T_{m1}$ (°C) | $T_{m2}$ (°C) |
|---|---|---|---|
| Stability | PANI-H-S62R | 66.6 | 84.5 |
| Stability | PANI-L-F87Y | 66.0 | 84.3 |

(continued)

| Expected Function of Design | Name* | $T_{m1}$ (°C) | $T_{m2}$ (°C) |
|---|---|---|---|
| Stability | PANI-L-L96N | 62.4 | 84.1 |
| Affinity | PANI-H-S54Q | 65.4 | 86.3 |
| Affinity | PANI-H-S54N | 67.0 | 84.4 |
| Affinity | PANI-H-T98E | 62.4 | 83.0 |
| Affinity / Stability | PANI-L-D92R | 66.1 | 83.6 |
| Control | Original Pani-scFv | 66.0 | 84.0 |

Based on these results, the following mutations were determined to most effectively improve/retain affinity and thermostability:

- Heavy Chain: S62R (under Chothia numbering scheme)

- Light Chain: F87Y, D92R (under Chothia numbering scheme)

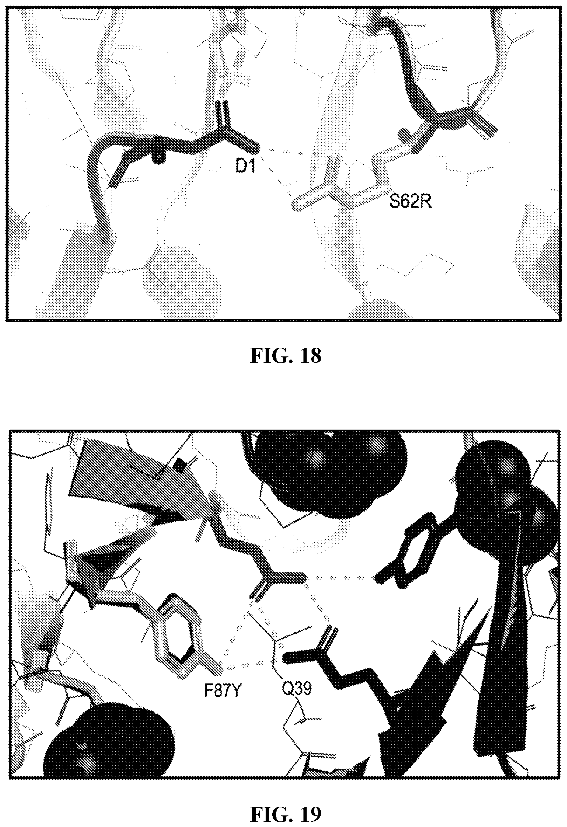

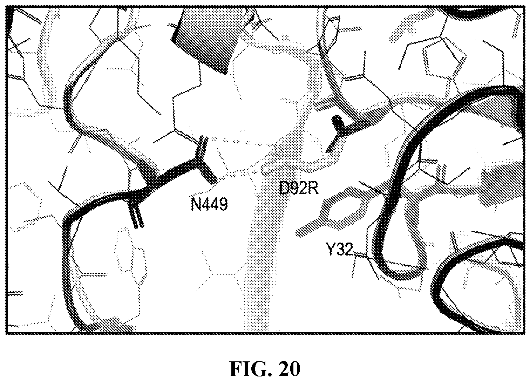

Structural modeling of these mutations are shown in **FIGs. 18-20.** As shown in **FIG. 18,** the S62R mutation of the VH chain of panitumumab was found to introduce additional hydrogen bonds with D1 of VL and contributes to the stability of the VH/VL interface according to the modeling. As shown in **FIG. 19,** the F87Y mutation of the VL chain of panitumumab was found to introduce additional hydrogen bonds with Q39 of VH and contributes to the stability of the VH/VL interface according to the modeling. As shown in **FIG. 20,** the D92R mutation at CDR3 of the VL chain of panitumumab, originally designed to improve affinity (for binding to N449 of EGFR), was found to unexpectedly form additional van der Waals' contacts with Y32 of CDRL1 and stabilizes the paratope, according to the modeling.

Thereafter, the impact of combinations of mutations on affinity and thermostability was evaluated using SPR and Differential Scanning Calorimetry (DSC), respectively. The results from the SPR analysis are shown in in **Table 14** and the results from the DSC analysis are shown in **Table 15.**

Table 14. SPR analysis for combination mutations. *-H denotes mutation in the heavy chain; L- denotes mutation in the light chain; and PANI denotes the panitumumab framework.

| | | Human EGFR | Rhesus EGFR | Human EGFR | Rhesus EGFR | Human EGFR | Rhesus EGFR |
|---|---|---|---|---|---|---|---|
| Expected Function of Design | Name* | $k_a$ (1/Ms) | | $k_d$ (1/s) | | $K_D$ (M) | |
| Stability Combo | PANI-H_S62R-L_F87Y | 4.00E+05 | 2.79E+05 | 1.01E-03 | 1.16E-03 | 2.53E-09 | 4.15E-09 |
| Stability / Affinity Combo | PANI-H_S62R-L_F87Y-L_D92R | 2.93E+05 | 2.09E+05 | 9.90E-04 | 1.12E-03 | 3.38E-09 | 5.36E-09 |
| Stability / Affinity Combo | PANI-H_S62R-L_F87Y-H_T98R | No binding | | | | | |
| Stability / Affinity Combo | PANI-H_S62R-L_F87Y-L_D92R-H_T98R | No binding | | | | | |
| Stability / Affinity Combo | PANI-L_F87Y-L_L96N-L_D92R | 3.52E+05 | 2.43E+05 | 2.97E-03 | 3.30E-03 | 8.46E-09 | 1.35E-08 |
| Expected Function of Design | Name* | $k_a$ (1/Ms) | | $k_d$ (1/s) | | $K_D$ (M) | |

(continued)

|  |  | Human EGFR | Rhesus EGFR | Human EGFR | Rhesus EGFR | Human EGFR | Rhesus EGFR |
|---|---|---|---|---|---|---|---|
| Stability / Affinity Combo | PANI-L_F87Y-L_L96N-H_T98R | Transient binding | | | | | |
| Stability / Affinity Combo | PANI-L_F87Y-L_L96N-L_D92R-H_T98R | No expression | | | | | |
| Stability / Affinity Combo | PANI-H_S62R-L_F87Y-L_L96N-L_D92R | 2.46E+05 | 1.82E+05 | 3.43E-03 | 3.89E-03 | 1.40E-08 | 2.14E-08 |
| Stability / Affinity Combo | PANI-H_S62R-L_F87Y-L_L96N-H_T98R | No binding | | | | | |
| Stability / Affinity Combo | PANI-H_S62R-L_F87Y-L_L96N-L_D92R-H_T98R | No binding | | | | | |
| Stability / Affinity Combo | PANI-L_F87Y-L_D92R | 3.05E+05 | N/A | 7.94E-04 | N/A | 2.45E-09 | N/A |
| Control | Original Pani-scFv | 5.51E+05 | 3.77E+05 | 1.05E-03 | 1.20E-03 | 1.91E-09 | 3.17E-09 |

**Table 15.** DSC analysis of 12 anti-EGFR constructs with single point mutations.

| Expected Function of Design | Name* | DSC | | | | |
|---|---|---|---|---|---|---|
|  |  | $T_{onset}$ (°C) | $T_{m1}$ (°C) | $T_{m2}$ (°C) | $T_{m3}$ (°C) | $T_{m4}$ (°C) |
| Stability Combo | PANI-H_S62R-L_F87Y | 56.06 | 65.53 | 76.66 | 81.59 | 83.32 |
| Stability Combo | PANI-L_F87Y-L_L96N | 51.54 | 59.77 / 66.76** | 76.62 | 81.67 | 83.33 |
| Stability Combo | PANI-H_S62R-L_F87Y-L_L96N | 50.51 | 60.31 / 66.33** | 76.42 | 81.64 | 83.32 |
| Stability / Affinity Combo | PANI-H_S62R-L_F87Y-L_D92R | 59.69 | 67.89 | 76.66 | 81.42 | 83.25 |
| Stability / Affinity Combo | PANI-H_S62R-L_F87Y-H_T98R | 60.62 | 68.82 | 76.84 | 81.51 | 83.27 |
| Stability / Affinity Combo | PANI-H_S62R-L_F87Y-L_D92R-H_T98R | 60.55 | 69.43 | 76.65 | 81.14 | 83.10 |
| Stability / Affinity Combo | PANI-L_F87Y-L_L96N-L_D92R | 56.00 | 64.86 | 76.07 | 81.42 | 83.22 |
| Stability / Affinity Combo | PANI-L_F87Y-L_L96N-H_T98R | 54.69 | 64.33 | 75.83 | 81.19 | 83.12 |
| Stability / Affinity Combo | PANI-H_S62R-L_F87Y-L_L96N-L_D92R | 55.72 | 65.18 | 75.95 | 81.25 | 83.13 |
| Stability / Affinity Combo | PANI-H_S62R-L_F87Y-L_L96N-H_T98R | 55.67 | 64.86 | 75.66 | 81.01 | 83.04 |
| Stability / Affinity Combo | PANI-H_S62R-L_F87Y-L_L96N-L_D92R-H_T98R | 57.17 | 66.55 | 75.97 | 81.18 | 83.15 |

(continued)

| Expected Function of Design | Name* | DSC | | | | |
|---|---|---|---|---|---|---|
| | | $T_{onset}$ (°C) | $T_{m1}$ (°C) | $T_{m2}$ (°C) | $T_{m3}$ (°C) | $T_{m4}$ (°C) |
| Control | Original Pani-scFv | 53.25 | 64.09 | 76.17 | 81.44 | 83.28 |
| *-H denotes mutation in the heavy chain; -L denotes mutation in the light chain; and PANI denotes the panitumumab framework. ** Additional peak was observed between peak 1 and peak 2. | | | | | | |

PANI-H_S62R-L_F87Y-L_D92R (hereinafter "EGFR-scFv-3") and PANI-H_S62R-L_F87Y (hereinafter "EGFR-scFv-2") in **Tables 14** and **15** demonstrated the best affinity and thermostability. The L:G100C and H: G44C mutations facilitate a disulfide bond to enhance the pairing of the VL and VH chains.

**Example 2. Surface Plasmon Resonance Analysis of TriNKETs**

This example was designed to assess the binding affinity of certain panitumumab-derived EGFR-binding sites to EGFR. Four TriNKETs containing scFvs derived from panitumumab and its variants, namely, EGFR-TriNKET-1, EGFR-TriNKET-2, EGFR-TriNKET-3, and EGFR-TriNKET-4 (see the "Exemplary multispecific binding proteins" subsection above), were constructed. Kinetics and affinities of EGFR binding constructs for recombinant human and rhesus EGFR were measured by SPR using a Biacore 8K instrument. Samples were captured on the anti-hFc IgG chip and a range of concentrations of human or rhesus recombinant EGFR-His was injected over captured test articles. Experiments were performed at physiological temperature of 37°C. Data were analyzed using Biacore 8K Insight Evaluation software (GE Healthcare).

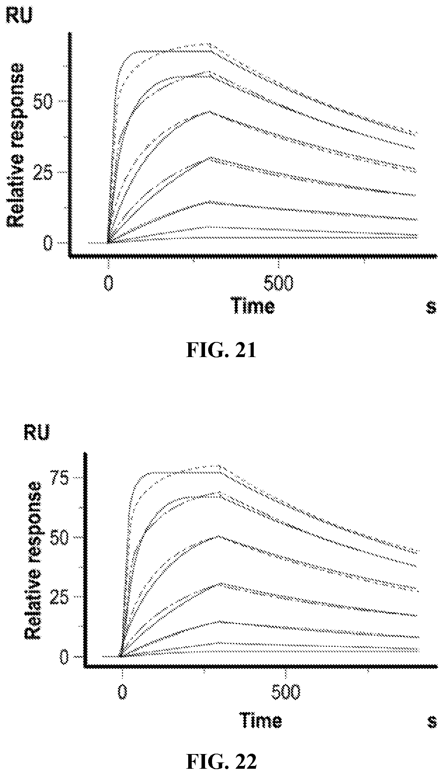

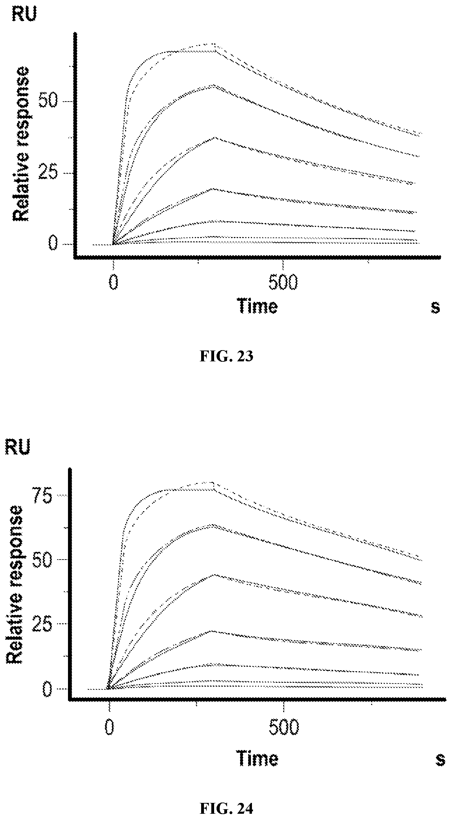

The binding sensorgrams from the SPR analysis are shown in **FIGs. 21-24. FIG. 21** shows SPR analysis for a titration of EGFR-TriNKET-1, **FIG. 22** shows SPR analysis for a titration of EGFR- TriNKET -2, **FIG. 23** shows SPR analysis for a titration of EGFR- TriNKET -3, and **FIG. 24** shows SPR analysis for a titration of EGFR- TriNKET -1. Parameters calculated from these analyses are shown in **Table 16.** The results demonstrated that the mutations of interest did not have a substantial impact on the affinity of the multispecific binding proteins to EGFR.

**Table 16.** SPR Analysis: Human EGFR Binding (N=4)

| | Light Chain Mutations | Heavy Chain Mutations | $k_a$ (M$^{-1}$ s$^{-1}$) | $k_d$ (s$^{-1}$) | $K_D$ (nM) |
|---|---|---|---|---|---|
| EGFR-TriNKET-1 | L: G100C | H: G44C | $(5.92\pm0.2)$ E+05 | $(9.67\pm0.05)$ E-04 | $(1.64\pm0.05)$ E-09 |
| EGFR-TriNKET-2 | L: G100C, F87Y | H: G44C, S62R | $(5.00\pm0.2)$ E+05 | $(9.88\pm0.09)$ E-04 | $(1.98\pm0.07)$ E-09 |
| EGFR-TriNKET-3 | L: G100C, F87Y, D92R | H: G44C, S62R | $(2.95\pm0.1)$ E+05 | $(9.30\pm0.03)$ E-04 | $(3.16\pm0.1)$E-09 |
| EGFR-TriNKET-4 | L: G100C, F87Y, D92R | H: G44C | $(3.16\pm0.2)$ E+05 | $(7.45\pm0.08)$ E-04 | $(2.36\pm0.01)$ E-09 |

**Example 3. Differential Scanning Calorimetry (DSC) analysis of TriNKETs**

This example was designed to assess the thermostability of the TriNKET constructs using DSC analysis. Briefly, test articles were buffer-exchanged into the buffer of choice using Thermo Scientific Zeba Spin Desalting Columns. The eluate was then diluted to 0.5 mg/mL with the same buffer. 325 $\mu$L of the sample was loaded into a 96-well deepwell plate with the corresponding buffer blank and analyzed using a Microcal PEAQ-DSC instrument (Malvern Panalytical). The temperature was ramped from 20-25 °C to 100 °C at a rate of 60°C/hr. Buffer background was run in triplicate before the analytes. The most representative buffer blank was subtracted from each analyte scan prior to analysis. The data was fit using DSC analysis software with a non-two state model and $T_{onset}$ and $T_m$s were reported.

EGFR-TriNKET-1 was used to obtain a melting curve as the baseline and identify the inflection points, especially the T-onset (the temperature under which the molecule starts melting) as well as Tm1, which is related to the stability of the scFv. Thereafter, EGFR-TriNKET-2, EGFR-TriNKET-3 and EGFR-TriNKET-4 were analyzed using the same method.

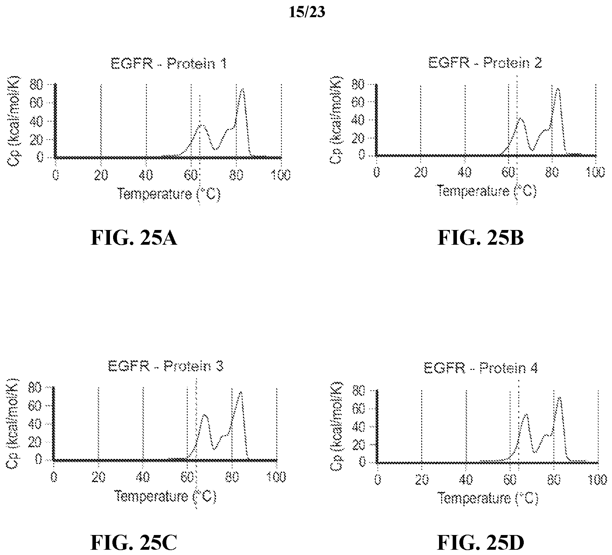

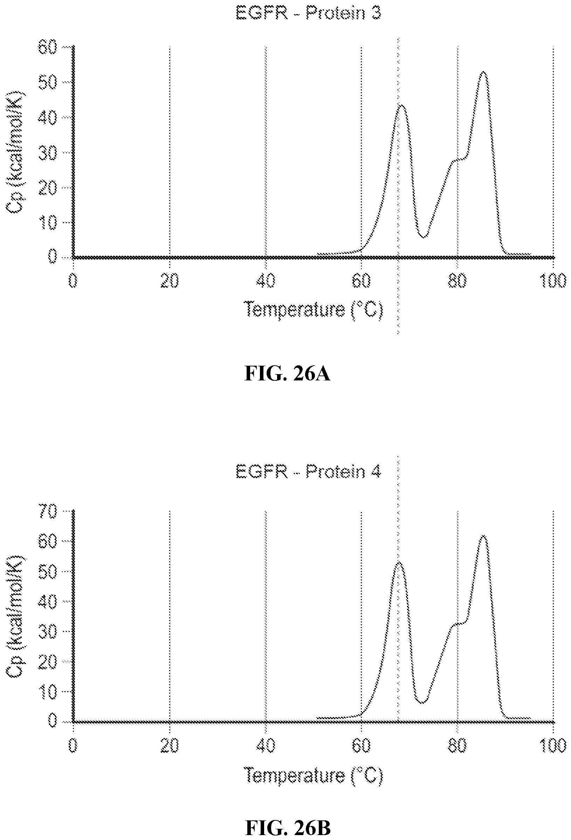

Table 17 and FIGs. 25A-25D show the DSC analysis results from testing in PBS, pH 7.4 buffer. FIG. 25A shows DSC analysis for EGFR-TriNKET-1 in PBS, pH 7.4 buffer. FIG. 25B shows DSC analysis for EGFR- TriNKET -2 in PBS, pH 7.4 buffer. FIG. 25C shows DSC analysis for EGFR- TriNKET -3 in PBS, pH 7.4 buffer. FIG. 25D shows DSC analysis for EGFR- TriNKET -4 in PBS, pH 7.4 buffer. Table 18 and FIGs. 26A and 26B show the DSC analysis results from testing in 20 mM histidine, 250 mM trehalose, 0.01% PS80, pH 6.0. FIG. 26A shows DSC analysis for EGFR-TriNKET-3 in 20 mM histidine, 250 mM trehalose, 0.01% PS80, pH 6.0 buffer. FIG. 26B shows DSC analysis for EGFR-TriNKET-3 in 20 mM histidine, 250 mM trehalose, 0.01% PS80, pH 6.0 buffer. The DSC analysis revealed that EGFR-TriNKET-3 (FIG. 25C) had improved thermostability relative to EGFR-TriNKET-2 (FIG. 25B), which in turn had improved thermostability relative to EGFR-TriNKET-1 (FIG. 25A). The results also showed similar thermostability of EGFR-TriNKET-3 (FIG. 26A) and EGFR-TriNKET-4 (FIG. 26B). However, EGFR-TriNKET-4 was more prone to degradation under accelerated thermostability studies (40 °C for 4 weeks, data not shown), which highlights the benefit of a S62R heavy chain mutation for stability.

Table 17. DSC analysis in PBS buffer, pH7.4

| Test article | Light Chain Mutations | Heavy Chain Mutations | Tonset, (°C) | Tm1, (°C) | Tm2, (°C) | Tm3, (°C) | Tm4, (°C) |
|---|---|---|---|---|---|---|---|
| EGFR-TriNKET-1 | G100C | G44C | 54.9 | 64.5 | 76.6 | 81.5 | 83.3 |
| EGFR-TriNKET-2 | G100C, F87Y | G44C, S62R | 56.2 | 65.3 | 76.4 | 81.5 | 83.2 |
| EGFR-TriNKET-3 | G100C, F87Y, D92R | G44C, S62 | 59.8 | 67.5 | 76.3 | 81.3 | 83.1 |
| EGFR-TriNKET-4 | G100C, F87Y, D92R | G44C | 58.6 | 66.9 | 76.1 | 81.3 | 83.2 |

Table 18. DSC analysis in 20 mM histidine, 250 mM trehalose, 0.01% PS80, pH 6.0

| Test article | Light Chain Mutations | Heavy Chain Mutations | Tonset, (°C) | Tm1, (°C) | Tm2, (°C) | Tm3, (°C) | Tm4, (°C) |
|---|---|---|---|---|---|---|---|
| EGFR-TriNKET-3 | G100C, F87Y, D92R | G44C, S62 | 59.0 | 67.8 | 79.1 | 84.2 | 86.3 |
| EGFR-TriNKET-4 | G100C, F87Y, D92R | G44C | 58.7 | 67.4 | 79.1 | 84.3 | 86.3 |

## Example 4. Assessment of TriNKET binding to EGFR positive cells

This example was designed to assess the binding affinity of the EGFR-targeting TriNKETs to EGFR expressed on cell surface. The H2172 human cancer cell line, derived from non-small cell lung carcinoma, was used. Briefly, H2172 cells were incubated with EGFR-TriNKET-1, EGFR-TriNKET-2, EGFR-TriNKET-3 or panitumumab at 4 °C for 0.5 hours. After incubation, binding patterns of the TriNKETs and panitumumab to EGFR+ cells were detected using a fluorophore conjugated anti-human IgG secondary antibody. Cells were analyzed by flow cytometry and fold MFI over secondary-only controls reported.

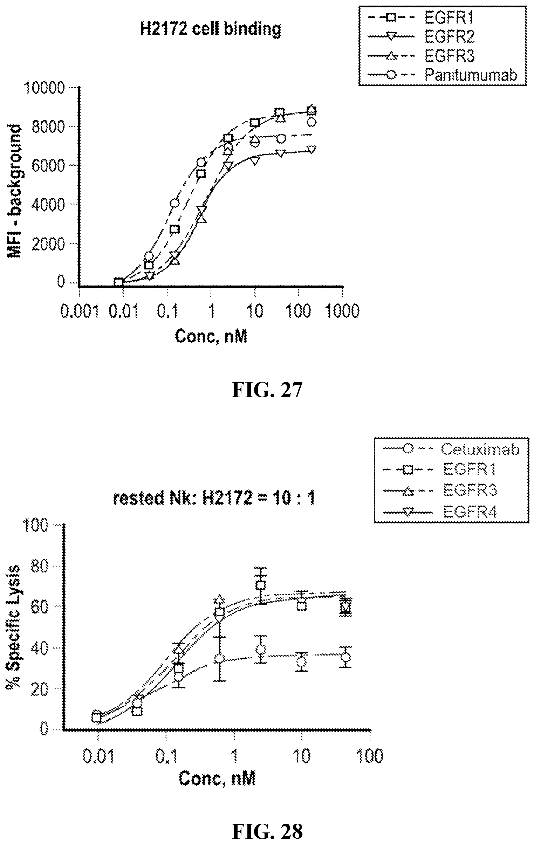

FIG. 27 shows binding to EGFR-positive cells after incubation with EGFR-TriNKET-1, EGFR-TriNKET-2, EGFR-TriNKET-3, or panitumumab. These EGFR-targeting TriNKETs bound the cells with sub-nM concentrations and with similar or higher maximum binding than panitumumab.

## Example 5. Primary human NK or CD8+ cell cytotoxicity assay

This example was designed to assess the ability of the EGFR-targeting TriNKETs to mediate cytotoxicity of immune effector cells against EGFR-expressing cancer cells. Briefly, peripheral blood mononuclear cells (PBMCs) were isolated from human peripheral blood buffy coats using density gradient centrifugation. Isolated PBMCs were washed and prepared for NK or CD8+ cell isolation. NK cells were isolated using a negative selection technique with magnetic beads, and the purity of isolated NK cells was typically >90% CD3-CD56+. Isolated NK cells were rested overnight in culture media without supplemental cytokines and used the following day in cytotoxicity assays. CD8+ were isolated using a negative selection technique with magnetic beads, and the purity of CD8+ cells was typically >90% CD3+ CD8+. Isolated CD8+ T cells were incubated in media with IL-15 for 6-13 days for expansion.

For the cytotoxicity assays, human cancer cell lines expressing EGFR were harvested from culture, cells were washed with HBS, and resuspended in growth media at $10^6$/mL for labeling with BATDA reagent (Perkin Elmer AD0116). Manufacturer instructions were followed for labeling of the target cells. After labeling, cells were washed 3x with HBS, and were resuspended at $0.5-1.0\times10^5$/mL in culture media. To prepare the background wells, an aliquot of the labeled cells was put aside, and the cells were spun out of the media. 100 $\mu$L of the media was carefully added to wells in triplicate to avoid disturbing the pelleted cells. 100 $\mu$L of BATDA labeled cells were added to each well of the 96-well plate. Wells were saved for spontaneous release from target cells, and wells were prepared for maximum lysis of target cells by addition of 1% Triton-X. Monoclonal antibodies (e.g., cetuximab) or TriNKETs against EGFR (e.g., EGFR-TriNKET-1, EGFR-TriNKET-3, and EGFR-TriNKET-4) were diluted in culture media, and 50 $\mu$L of diluted mAb or TriNKET was added to each well. Rested NK cells were harvested from culture, cells were washed, and were resuspended at $10^5-2.0\times10^6$ cells/mL in culture media depending on the desired effectorto-target (E:T) ratio. 50 $\mu$L of NK cell suspension was added to each well of the plate to make a total of 200 $\mu$L culture volume. The plate was incubated at 37 °C with 5% $CO_2$ for 2-3hrs before developing the assay.

After culturing for 2-3 hours, the plate was removed from the incubator and the cells were pelleted by centrifugation at 200 g for 5 minutes. 20 $\mu$L of culture supernatant was transferred to a clean microplate provided by the manufacturer, and 200 $\mu$L of room temperature europium solution was added to each well. The plate was protected from the light and incubated on a plate shaker at 250 rpm for 15 minutes, then read using either Victor 3 or SpectraMax i3X instruments. % Specific lysis was calculated as follows:

% Specific lysis = ((Experimental release - Spontaneous release) / (Maximum release - Spontaneous release)) * 100%

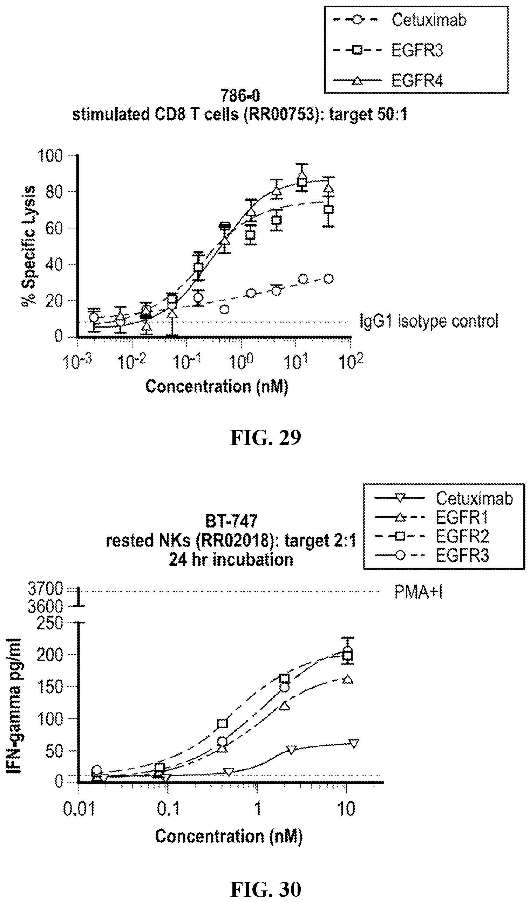

As shown in **FIGs. 28 and 29,** human NK cell- and CD8+ T cell- lysis of H2172 cells **(FIG. 28)** and 786-0 cells **(FIG. 29),** respectively, in the presence of EGFR TriNKETs or anti-EGFR mAb cetuximab, is observed within 2 hours of incubation. In both systems and across multiple donors (data not shown), the EGFR-TriNKETs had sub-nM EC50 values. Compared to the low to absent activity of cetuximab, the TriNKETs provided greater maximum specific lysis and potency.

## Example 6. Assessment of NK IFN-gamma production mediated by TriNKET

This example was designed to assess the ability of the EGFR-targeting TriNKETs to activate NK cells in the presence of EGFR-expressing cancer cells. Briefly, BT-474 EGFR-expressing cancer cells served as the target cells. Isolated NK cells (derived from multiple human donors) were either rested overnight in culture media without supplemental cytokines or in culture media with recombinant human IL-2. The rested NK cells were incubated with the target cells at a 2:1 ratio of effector cells (NK cells) to target cells (E:T ratio) for 24 hours, whereas the IL-2 activated NK cells were incubated with the target cells at a 1:1 E:T ratio for 24 hours. Following incubation, secretion of IFN-$\gamma$ from the NK cells was analyzed using an IFN-$\gamma$ ELISA kit according to the Mesoscale manufacturer's instruction. Plates were read using either Victor 3 or SpectraMax i3X instruments.

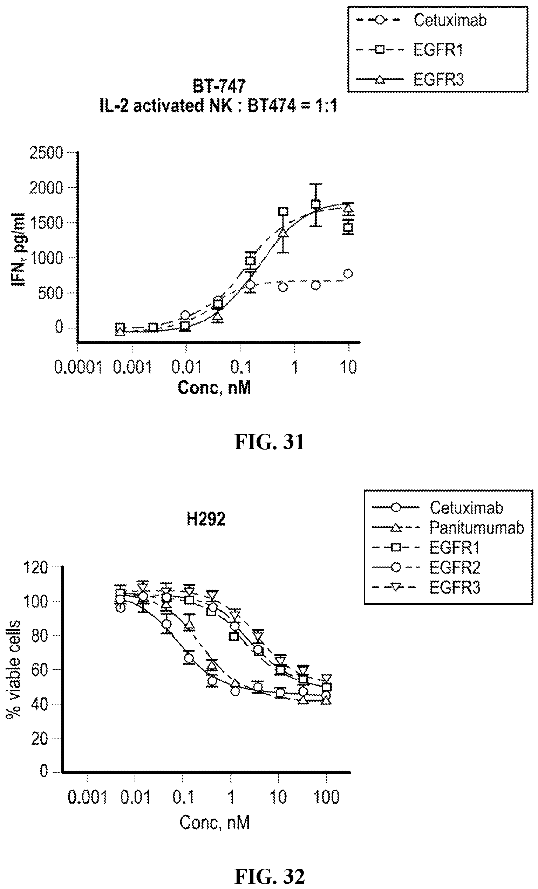

**FIGs. 30** and **31** show the resultant IFN-gamma production from the rested **(FIG. 30)** and IL-2 **(FIG. 31)** activated NK cells, respectively, after incubation with the BT-474 cells in the presence of EGFR-TriNKETs or anti-EGFR mAb cetuximab. In both cases, the EGFR-TriNKETs mediated higher production of IFN-gamma by the NK cells as compared to cells treated with cetuximab.

## Example 7. EGFR Cell Proliferation Assay

This example was designed to assess the impact of EGFR TriNKETs on the proliferation of EGFR-expression human cancer cell line H292 in the absence of effector cells. Briefly, the EGFR-TriNKETs and anti-EGFR-mAbs cetuximab and panitumumab were diluted and incubated with H292 cells for 72 hours. Following incubation, the cell proliferation was measured using Cell-titer Glo according to the manufacturer's instructions.

**FIG. 32** shows H292 cell proliferation in the presence of the EGFR-TriNKETs or the anti-EGFR mAbs. The cells treated with the anti-EGFR mAbs showed less proliferation than those treated with the EGFR-TriNKETs. This was attributed to the difference in the valency of the TriNKETs and mAbs-the TriNKETs are monovalent, while the mAbs are bivalent-and demonstrated the benefit of using a TriNKET that has a lower valency, given the association of EGFR targeting with skin-related toxicity that has been observed in the mAbs.

**Example 8. ADCP Activity Assay**

This example was designed to assess the antibody dependent cellular phagocytosis (ADCP) activity of macrophages when incubated with EGFR-expressing target cells in the presence of EGFR-TriNKETs. Briefly, PBMCs were isolated from human peripheral blood buffy coats using density gradient centrifugation. Isolated PBMCs were washed and prepared for monocyte isolation. Monocyte cells were isolated using a negative selection technique with magnetic beads, and the purity of isolated monocytes cells was typically >90% CD14. Monocytes were differentiated into macrophages by incubating for 6 days in GM-CSF and IL-4.

Macrophages were harvested, labeled with CellTrace Violet and incubated with CFSE labeled target cells (8:1) and molecules for 2 hours. Cells were stained and prepared for FACs analysis. Phagocytosis was calculated as follows:

$$\% \text{ phagocytosis} = \%\text{target+ and effector+ of parent gate "target+"}.$$

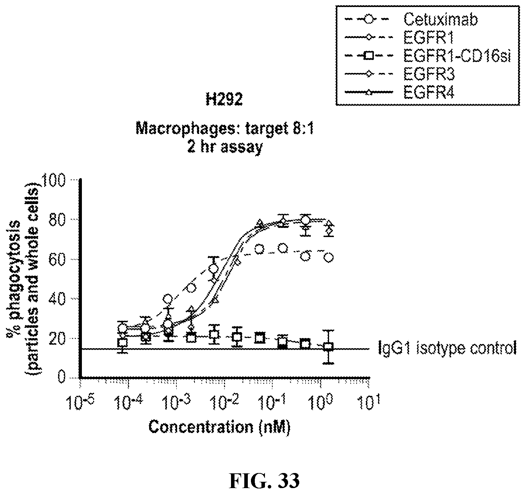

As shown in **FIG. 33,** the macrophages incubated with EGFR-expressing H292 cells in the presence of EGFR-TriNKET-1, EGFR-TriNKET-3 and EGFR-TriNKET-4 showed higher ADCP activity than those incubated in the presence of the anti-EGFR mAb cetuximab. The results also showed that the ADCP activity of EGFR-TriNKET-1 is lost when the ability of the TriNKET to bind CD16 was abrogated by L234A, L235A, and P329G (LALAPG) mutations in the Fc domain.

**Example 9. Anti-Tumor Efficacy of EGFR-TriNKET *in vivo***

This example was designed to test whether EGFR-TriNKET elicits anti-tumor functions *in vivo.* Briefly, nude mice were injected subcutaneously with $4\times10^6$ NCI-H292 tumor cells. Mice were randomized into different treatment groups when tumor size averaged ~90 mm³ volume (Day 7 after inoculation). Mice were treated intraperitoneally (IP) twice weekly with hIgG1 isotype, EGFR-TriNKET, or Cetuximab.

EGFR-TriNKET significantly reduced NCI-H292 tumor growth in mice dosed with 300 μg or 100 μg/dose compared to isotype treated animals ($p<0.01$; **FIG. 34A-C**). **FIG. 34A** shows average tumor volumes of individual mice treated with 300 μg, 100 μg, 30 μg of EGFR-TriNKET, or hIgG1 isotype control. **FIG. 34B** shows tumor volumes of individual mice treated with 300 μg of EGFR-TriNKET, or hIgG1 isotype control. **FIG. 34C** shows tumor volumes of individual mice treated with 100 μg of EGFR-TriNKET, or hIgG1 isotype control. **FIG. 34D** shows tumor volumes of individual mice treated with 30 μg of EGFR-TriNKET, or hIgG1 isotype control. As shown in **FIG. 34A, FIG. 34B,** and **FIG. 34C,** treatment of mice with 300 μg and 100 μg of EGFR-TriNKET, significantly reduced tumor volumes as compared to hIgG1 isotype control. As shown in **FIG. 34D,** treatment of mice with 30 μg/dose of EGFR-TriNKET significantly delayed tumor progression as compared to the hIgG1-treated control group.

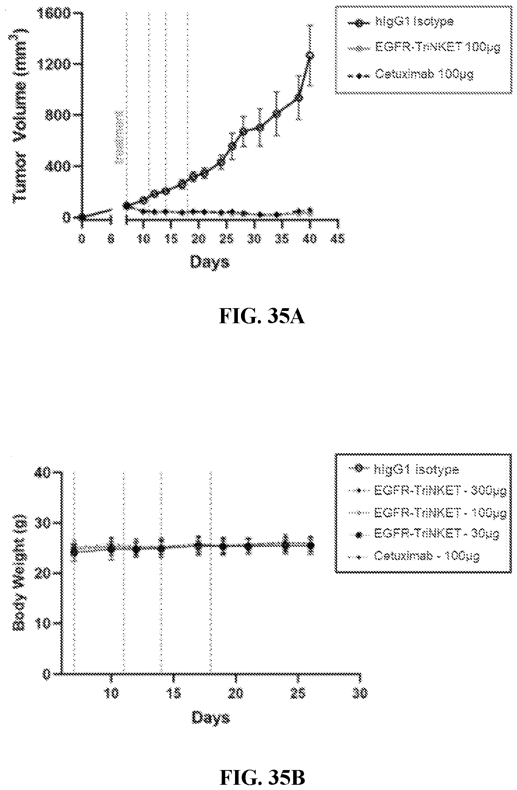

In addition to EGFR-TriNKET dose titration *in vivo,* anti-tumor efficacy mediated by EGFR-TriNKET was compared to Cetuximab at an equimolar dose of 100 μg Cetuximab. As shown in **FIG. 35A,** treatment of individual mice with EGFR-TriNKET was as potent as Cetuximab in inducing anti-tumor responses *in vivo.*

Notably, the regimen of EGFR-TriNKET and Cetuximab appeared to be well tolerated by NCI-H292 tumor-bearing mice. There were neither clinical observations nor effects on body weight **(FIG. 35B).**

**Claims**

**1.** A protein comprising:

(a) an EGFR-binding scFv (VL-VH) sequence in the orientation of heavy chain variable domain (VH) positioned C-terminal to light chain variable domain (VL), linked to an Fc domain polypeptide to form a (VL-VH)-Fc polypeptide, comprising the amino acid sequence of SEQ ID NO: 167;
and
(b) an NKG2D-binding Fab fragment including:

a heavy chain portion comprising a heavy chain variable domain (VH) and a CH1 domain, wherein the CH1 domain is connected to an Fc domain polypeptide to form a VH-CH1-Fc polypeptide comprising the amino acid sequence of SEQ ID NO:164, and
a light chain portion comprising a light chain variable domain (VL) and a light chain constant (CL) domain to form a VL-CL polypeptide comprising the amino acid sequence of SEQ ID NO:165.

**2.** A formulation comprising a protein according to claim 1 and a pharmaceutically acceptable carrier.

**3.** A cell comprising one or more nucleic acids encoding a protein of claim 1.

**4.** The protein of claim 1 for use in a method of treating a cancer, wherein the method comprises administering to a patient in need thereof an effective amount of the protein.

**Patentansprüche**

**1.** Protein, das Folgendes umfasst:

(a) eine EGFR-bindende scFv-(VL-VH)-Sequenz in der Orientierung von C-terminal positionierter variabler Schwerkettendomäne (VH) zu variabler Leichtkettendomäne (VL), die mit einem Fc-Domäne-Polypeptid verknüpft ist, um ein (VL-VH)-Fc-Polypeptid zu bilden, das die Aminosäuresequenz von SEQ ID NO: 167 umfasst; und
(b) ein NKG2D-bindendes Fab-Fragment, das Folgendes beinhaltet:

einen Schwerkettenabschnitt, der eine variable Schwerkettendomäne (VH) und eine CH1-Domäne umfasst, wobei die CH1-Domäne mit einem Fc-Domäne-Polypeptid verbunden ist, um ein VH-CH1-Fc-Polypeptid zu bilden, das die Aminosäuresequenz von SEQ ID NO: 164 umfasst, und
einen Leichtkettenabschnitt, der eine variable Leichtkettendomäne (VL) und eine konstante Leichtkettendomäne (CL) umfasst, um ein VL-CL-Polypeptid zu bilden, das die Aminosäuresequenz von SEQ ID NO: 165 umfasst.

**2.** Formulierung, die ein Protein nach Anspruch 1 und einen pharmazeutisch verträglichen Träger umfasst.

**3.** Zelle, die eine oder mehrere Nukleinsäuren umfasst, die für ein Protein nach Anspruch 1 codieren.

**4.** Protein nach Anspruch 1 zur Verwendung in einem Verfahren zum Behandeln eines Krebses, wobei das Verfahren Verabreichen einer wirksamen Menge des Proteins an einen dieses benötigenden Patienten umfasst.

**Revendications**

**1.** Protéine comprenant :

(a) une séquence de scFv de liaison à l'EGFR (VL-VH) dans l'orientation du domaine variable de chaîne lourde (VH) positionné à l'extrémité C-terminale jusqu'au domaine variable de chaîne légère (VL), liée à un polypeptide de domaine Fc pour former un polypeptide (VL-VH)-Fc, comprenant la séquence d'acides aminés de SEQ ID NO : 167 ;
et
(b) un fragment Fab de liaison à NKG2D comprenant :

une partie de chaîne lourde comprenant un domaine variable de chaîne lourde (VH) et un domaine CH1, dans laquelle le domaine CH1 esnt relié à un polypeptide de domaine Fc pour former un polypeptide VH-CH1-Fc comprenant la séquence d'acides aminés de SEQ ID NO : 164, et
une partie de chaîne légère comprenant un domaine variable de chaîne légère (VL) et un domaine constant de chaîne légère (CL) pour former un polypeptide VL-CL comprenant la séquence d'acides aminés de SEQ ID NO : 165.

**2.** Formulation comprenant la protéine selon la revendication 1 et un support pharmaceutiquement acceptable.

**3.** Cellule comprenant un ou plusieurs acides nucléiques codant pour une protéine selon la revendication 1.

**4.** Protéine selon la revendication 1 pour une utilisation dans un procédé de traitement d'un cancer, dans lequel le procédé comprend l'administration à un patient en ayant besoin d'une quantité efficace de la protéine.

FIG. 1

FIG. 2A

FIG. 2B

EGFR binding arms

NKG2D arm

**FIG. 2C**

EGFR binding arms

NKG2D arm

**FIG. 2D**

NK cell targeting
Fab fragment

EGFR targeting
scFv

**FIG. 2E**

**FIG. 3**

**FIG. 4**

**FIG. 5**

**FIG. 6**

**FIG. 7**

**FIG. 8**

**FIG. 9**

**FIG. 10**

**FIG. 11**

**FIG. 12**

**FIG. 13A**

**FIG. 13B**

**FIG. 14**

**FIG. 15**

**FIG. 16**

**FIG. 17**

**FIG. 18**

**FIG. 19**

**FIG. 20**

**FIG. 21**

**FIG. 22**

**FIG. 23**

**FIG. 24**

**FIG. 25A**

**FIG. 25B**

**FIG. 25C**

**FIG. 25D**

**FIG. 26A**

**FIG. 26B**

**FIG. 27**

**FIG. 28**

**786-0**
**stimulated CD8 T cells (RR00753): target 50:1**

Legend:
- - ○ - - Cetuximab
- - □ - - EGFR3
- △ - EGFR4

**FIG. 29**

**BT-747**
**rested NKs (RR02018): target 2:1**
**24 hr incubation**

Legend:
- ▽ Cetuximab
- △ - - EGFR1
- - □ - - EGFR2
- ○ - - EGFR3

**FIG. 30**

**FIG. 31**

**FIG. 32**

Legend:
- Cetuximab
- EGFR1
- EGFR1-CD16si
- EGFR3
- EGFR4

H292

Macrophages: target 8:1
2 hr assay

% phagocytosis (particles and whole cells) vs Concentration (nM)

IgG1 isotype control

**FIG. 33**

**FIG. 34A**

**FIG. 34B**

**FIG. 34C**

**FIG. 34D**

**FIG. 35A**

**FIG. 35B**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016134371 A
- WO 2015095412 A
- WO 2019035939 A
- WO 2015184203 A
- WO 2018148445 A
- WO 2019157366 A
- US 9273136 B
- US 7879985 B
- US 13494870 B

- US 16028850 B
- US 11533709 B
- US 12875015 B
- US 13289934 B
- US 14773418 B
- US 12811207 B
- US 13866756 B
- US 14647480 B
- US 14830336 B


**Non-patent literature cited in the description**

- **MARTIN**. Remington's Pharmaceutical Sciences. Mack Publ. Co., 1975
- **ATWELL S** ; **RIDGWAY JB** ; **WELLS JA** ; **CARTER P.** Stable heterodimers from remodeling the domain interface of a homodimer using a phage display library. *J. Mol. Biol.*, 1997, vol. 270 (1), 26-35
- **ELLIOTT JM** ; **ULTSCH M** ; **LEE J** ; **TONG R** ; **TAKEDA K** ; **SPIESS C et al.** Antiparallel conformation of knob and hole aglycosylated half-antibody homodimers is mediated by a CH2-CH3 hydrophobic interaction. *J. Mol. Biol.*, 2014, vol. 426 (9), 1947-57
- **MIMOTO F** ; **KADONO S** ; **KATADA H** ; **IGAWA T** ; **KAMIKAWA T** ; **HATTORI K.** Crystal structure of a novel asymmetrically engineered Fc variant with improved affinity for FcγRs.. *Mol. Immunol.*, 2014, vol. 58 (1), 132-8
- **LEWIS SM** ; **WU X** ; **PUSTILNIK A** ; **SERENO A** ; **HUANG F** ; **RICK HL et al.** Generation of bispecific IgG antibodies by structure-based design of an orthogonal Fab interface.. *Nat. Biotechnol*, 2014, vol. 32 (2), 191-8
- **MUDA M. et al.** *Protein Eng. Des. Sel.*, 2011, vol. 24 (5), 447-54

- **WRANIK, BJ. et al.** *J. Biol. Chem.*, 2012, vol. 287, 43331-9
- **DOPPALAPUDI VR et al.** *, PNAS*, 2010, vol. 107 (52), 22611-22616
- **KABAT et al.** Sequences of Proteins of Immunological Interest. *NIH Publication*, 1991
- **CHOTHIA C** ; **LESK A M**. *J. Mol. Biol.*, 1987, vol. 196, 901-917
- **MACCALLUM R M et al.** *J. Mol. Biol.*, 1996, vol. 262, 732-745
- **SONDERMANN et al.** *Nature*, vol. 406 (6793), 267-273
- **CHAN et al.** *Cell Death Differ*, 2014, vol. 21 (1), 5-14
- Remington's Pharmaceutical Sciences. Mack Publishing Company, 1985
- **LANGER**. *Science*, 1990, vol. 249, 1527-1533
- **SCHMITZ et al.** *Clinica Chimica Acta*, 2001, vol. 308, 43-53
- **STEIMER et al.** *, Clinica Chimica Acta*, 2001, vol. 308, 33-41